(19) Europäisches Patentamt / European Patent Office / Office européen des brevets

(11) **EP 2 282 715 B1**

(12) **EUROPÄISCHE PATENTSCHRIFT**

(45) Veröffentlichungstag und Bekanntmachung des Hinweises auf die Patenterteilung:
**07.03.2018 Patentblatt 2018/10**

(21) Anmeldenummer: **09742075.6**

(22) Anmeldetag: **06.05.2009**

(51) Int Cl.:
**A61K 8/87** (2006.01)      **A61Q 19/00** (2006.01)
**C08G 18/10** (2006.01)      **C08G 18/48** (2006.01)
**C08G 18/73** (2006.01)

(86) Internationale Anmeldenummer:
**PCT/EP2009/055440**

(87) Internationale Veröffentlichungsnummer:
**WO 2009/135857 (12.11.2009 Gazette 2009/46)**

(54) **POLYURETHANE ALS RHEOLOGIEMODIFIZIERENDE MITTEL FÜR KOSMETISCHE ZUBEREITUNGEN**

POLYURETHANES AS RHEOLOGICAL MODIFYING MEANS FOR COSMETIC PREPARATIONS

POLYURÉTHANES COMME AGENTS MODIFICATEURS DE RHÉOLOGIE POUR PRÉPARATIONS COSMÉTIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **06.05.2008 EP 08155675**
**25.03.2009 EP 09156217**

(43) Veröffentlichungstag der Anmeldung:
**16.02.2011 Patentblatt 2011/07**

(73) Patentinhaber: **BASF SE**
**67056 Ludwigshafen am Rhein (DE)**

(72) Erfinder:
• WENDEL, Volker
  64342 Seeheim-Jugenheim (DE)
• VÖLLMAR, Helmuth
  67061 Ludwigshafen (DE)
• TÜRK, Holger
  68161 Mannheim (DE)
• BUCHMANN, Markus
  67251 Freinsheim (DE)
• ANDRE, Valerie
  67063 Ludwigshafen (DE)
• LAUBENDER, Matthias
  67105 Schifferstadt (DE)
• WOOD, Claudia
  69469 Weinheim (DE)
• DRAGON, Andree
  67346 Speyer (DE)

(74) Vertreter: **BASF IP Association et al**
**BASF SE**
**G-FLP-C006**
**67056 Ludwigshafen (DE)**

(56) Entgegenhaltungen:
WO-A1-98/00499          WO-A2-02/44236
DE-A1-102006 023 001    DE-C1- 4 101 239
US-A- 4 079 028         US-A- 4 155 892
US-A- 5 594 087         US-A- 5 612 408
US-A- 5 955 063         US-B1- 6 589 517

• BARMAR M ET AL: "Investigating the effect of hydrophobic structural parameters on the thickening properties of HEUR associative copolymers", EUROPEAN POLYMER JOURNAL, PERGAMON PRESS LTD. OXFORD, GB, Bd. 41, Nr. 3, 1. März 2005 (2005-03-01), Seiten 619-626, XP004719840, ISSN: 0014-3057

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft kosmetische Zubereitungen, die neuartige Polyurethane (PU) oder Mischungen solcher Polyurethane PU insbesondere als Mittel zur Modifizierung der rheologischen Eigenschaften enthalten.

Insbesondere betrifft die Erfindung eine kosmetische Zubereitung enthaltend eine in Wasser dispergierbare Mischung von Polyurethanen (PU) mit einem im Wesentlichen linearen Rückgrat aufgebaut aus alternierenden hydrophilen und hydrophoben Abschnitten, wobei

a. die beiden endständigen Abschnitte (T) hydrophob sind,
b. sich an jeden Abschnitt T je ein hydrophiler Abschnitt (S) direkt anschließt,
c. sich an jeden Abschnitt S mindestens ein hydrophober Abschnitt (D) auf mindestens einer Seite direkt anschließt, und
d. wobei mindestens ein hydrophiler Abschnitt (P) enthalten ist, wobei mindestens ein hydrophober Abschnitt D zwei Abschnitte P trennt, falls mehr als ein Abschnitt P enthalten ist,

und die Polyurethane mindestens drei hydrophile Abschnitte umfassen, und das Verhältnis der Molekulargewichte eines jeden hydrophilen Abschnittes S zu dem Molekulargewicht eines jeden hydrophilen Abschnittes P von 1 zu 1,4 bis 1 zu 140 beträgt, die mindestens zwei hydrophoben Abschnitte D aliphatische Diisocyanatreste sind und der mindestens eine hydrophile Abschnitt P ein Polyetherrest mit einem zahlenmittleren Molekulargewicht von mindestens 1500 g/mol ist, wobei die Polyurethane eine Mischung darstellen, die Polyurethane enthält, deren Abschnitte T beide verzweigt sind, und solche, deren Abschnitte T beide linear sind, und solche, die einen linearen Abschnitt T und einen verzweigten Abschnitt T enthalten.

Die in den erfindungsgemäßen kosmetischen Zubereitungen enthaltenen Polyurethane sind Polymere, die durch Umsetzung von Alkoholalkoxylaten und/oder Polyetherpolyolen mit Isocyanaten oder Polyisocyanaten entstehen, und werden im Folgenden auch Polyetherurethane genannt. Im Folgenden wird für die in den erfindungsgemäßen kosmetischen Zubereitungen enthaltenen Polyurethane auch die Abkürzung "PU" verwendet.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet insbesondere, das Körperäußere zu pflegen, zu verbessern und zu verschönern, um auf sichtbare, fühlbare und riechbare Weise sowohl den Mitmenschen als auch sich selbst zu gefallen.

Im Rahmen dieser Erfindungen werden unter kosmetischen Zubereitungen aber auch solche Zubereitungen verstanden, die der Mundhygiene und -kosmetik dienen. Unter kosmetischen Zubereitungen werden im Rahmen dieser Erfindung auch dermatologische Zubereitungen verstanden.

Unter Modifizierung der rheologischen Eigenschaften wird ganz allgemein die Änderung des Verformungs- und Fließverhaltens von Materie verstanden. Die bedeutendsten rheologischen Eigenschaften sind Viskosität, Thixotropie, Strukturviskosität, Rheo-pexie und Dilatanz. Diese Begriffe sind dem Fachmann bekannt.

Unter Modifizierung der Rheologie wird im Besonderen die Erhöhung der Viskosität von Flüssigkeiten verstanden, üblicherweise auch als "Verdickung" bezeichnet. Diese Viskositätserhöhung kann bis zur Entstehung von Gelen oder Festkörpern reichen. Wasserdispergierbare Polyurethane, die zu einer Erhöhung der Viskosität und damit zu einer Wirkung als Verdickungsmittel führen, sind bekannt.

Üblicherweise verwendete Verdickungsmittel sind Fettsäurepolyethylenglykolmonoester, Fettsäurepolyethylenglykoldiester, Fettsäurealkanolamide, oxethylierte Fettalkohole, ethoxylierte Glycerinfettsäureester, Celluloseether, Natriumalginat, Polyacrylsäuren (INCI: Carbomer, beispielsweise Carbopol®-Marken), Tauratderivate, Polysaccharide und Neutralsalze wie beispielsweise Natriumchlorid.

Die Verwendung der vorgenannten, üblichen Verdickungsmittel ist jedoch, je nach der zu verdickenden Zubereitung, mit Nachteilen verbunden. So kann die Verdickungswirkung und die Salzstabilität der Verdickungsmittel nicht zufriedenstellend und ihre Einarbeitung in die zu verdickende Zubereitung erschwert sein. Es ist bekannt, dass Verdicker wie z.B. vernetzte (hydrophob modifizierte) Polyacrylsäuren in neutralisiertem Zustand sehr empfindlich auf Salz oder Tensid oder eine Mischung derselben reagieren. So kann Salzzugabe zu abrupter und drastischer Viskositätserniedrigung führen. Deshalb ist es beispielsweise unüblich, solche Polymere in Shampoo-Formulierungen als Verdicker einzusetzen. Aufgrund der dort vorliegenden Salzkonzentrationen (Tenside, Tensid-Gemische, NaCl als Verunreinigung in Tensiden) kann durch die Zugabe üblicher Verdickungsmittel keine signifikante Viskositätserhöhung herbeigeführt werden. Bei Anwesenheit von kationischen Hilfsstoffen kann es sogar zu Komplexbildung und Niederschlag kommen. Als äußerst schwierig gestaltet sich im Bereich der kosmetischen Zubereitungen die Suche nach salztoleranten (salzstabilen) Verdickern, die bei guter Verdickungsleistung in Gegenwart von Salz auch zu Zubereitungen mit guter Textur und angenehmen Gefühl auf der Haut und/oder den Haaren führen.

Wesentliche Anforderungen an Verdicker für kosmetische Zubereitungen sind außerdem die Verträglichkeit mit den zahlreichen weiteren Inhaltsstoffen dieser Zubereitungen, insbesondere mit Salzen und Tensiden sowie die problemlose Einarbeitbarkeit.

Die verdickten Zubereitungen dürfen auch bei Langzeitlagerung über mehrere Wochen bis Monate, Temperatur- und pH-Veränderungen keine wesentlichen Änderungen von Rheologie, physikalischer und chemischer Qualität aufweisen. Letztlich sollen diese Verdicker kostengünstig und ohne merkliche Umweltbelastung herzustellen sein.

Bereits Ende der 70er Jahre wurden in US-A-4,079,028 Verdickungsmittel vom sogenannten HEUR-Typ beschrieben (das Akronym HEUR leitet sich von "nonionic hydrophobically modified ethylene oxide urethane block copolymer" ab). Diese Verdicker sind aufgebaut aus linearen und/oder verzweigten Polyethylenglycolblöcken und Hydrophobsegmenten, die in der Regel über Urethangruppen (bei Verwendung von Aminen statt Alkoholen resultieren Harnstoffgruppen) miteinander verknüpft sind. Solche HEUR-Verdicker werden bereits seit geraumer Zeit in den unterschiedlichsten Anwendungsfeldern zur Verdickung wasserbasierter Dispersionsfarben eingesetzt. Als Wirkprinzip für die verdickende Wirkung der HEUR-Verdicker wird angenommen, dass die Polyethylenglykolsegmente die Wasserverträglichkeit sicherstellen und die Hydrophobsegmente über eine Assoziation untereinander sowie mit dispergierten Bindemittelteilchen der zu verdickenden Dispersionsfarbe in dieser einen viskositätsgebenden dreidimensionalen Molekülverbund aufbauen.

Bevorzugte hydrophobe Bausteine in marktüblichen HEUR-Verdickern sind längerkettige, in der Regel monofunktionelle Alkohole, wie beispielsweise n-Octanol, n-Dodecanol, iso-Tridecylalkohol, iso-Nonylphenol oder Ricinolsäuremethylester. Diese Alkohole kommen überwiegend als solche, aber auch in Form ihrer Additionsprodukte mit einigen wenigen Äquivalenten Ethylenoxid zum Einsatz.

Im Bereich der Kosmetik sind als HEUR-Typen besonders die Aculyn™-Marken (A-culyn™44 und Aculyn™- 46, Rohm & Haas) zu nennen.

US 4,079,028 und US 4,155,892 offenbaren lineare Polyurethan-Verdicker und deren Verwendung in der Kosmetik. Die Herstellung dieser Polyurethan-Verdicker erfolgt in Gegenwart von zinnhaltigen Polymerisationskatalysatoren.

EP 1013264-B und EP 1584331-A offenbaren kosmetische Zubereitungen, die Polyurethan-Verdicker und ein- bzw. mehrwertige niedere Alkohole enthalten. Die Polyurethan-Verdicker werden ohne Katalysator in einem Ein-Schritt-Verfahren durch Reaktion in Substanz aus Polyol, Polyisocyanat und Fettalkohol, der gewünschtenfalls ethoxyliert sein kann, hergestellt. Die Viskosität einer Zubereitung, die diese Verdicker enthalten, ändert sich angeblich nicht, wenn sich die Salzkonzentration in der Zubereitung ändert.

EP 1241198-A beschreibt wasserlösliche oder wasserdispergierbare Polyurethane, erhalten in ein- oder mehrstufiger Umsetzung, unter Einhaltung eines NCO/OH-Äquivalentverhältnisses von 0,5:1 bis 1,2:1 als Umsetzungsprodukte aus

A) einem Gemisch aus mindestens einem Polyetherpolyol a1) der mittleren Funktionalität ≥3 und mindestens einem urethangruppenhaltigen Polyetherpolyol a2) der mittleren Funktionalität ≥4,
B) mindestens einem Monoalkohol mit 6 bis 22 Kohlenstoffatomen,
C) mindestens einem (cyclo)aliphatischen und/oder aromatischen Diisocyanat,
D) gegebenenfalls mindestens einem Monoisocyanat mit 4 bis 18 Kohlenstoffatomen und
E) gegebenenfalls mindestens einem Polyisocyanat einer mittleren Funktionalität >2.

WO 02/44236 beschreibt kosmetische Zubereitungen enthaltend Polyurethan-Verdickungsmittel der Formel R1(CH2CH2O)n1CONH-X-NHCOO(CH2CH2O)mCONH-Y-NH-OC(OCH2CH2)n2OR2 in derR1 und R2 unabhängig voneinander für lineare oder verzweigte, gesättigte oder ungsättigte Alkylreste mit 6 bis 22 Kohlenstoffatomen und 0 und/oder 1 bis 3 Doppelbindungen, n1 und n2 in Summe für 0 oder für Zahlen von 1 bis 100, m für Zahlen von 4 bis 500 steht,- (CH2)z1-CR3R4]a1-[Ph]x-[CR5R6-(CH2)z2]a2, in der R3, R4, R5 und R6 unabhängig voneinander für Wasserstoff oder Alkylreste mit 1 bis 4 Kohlenstoffatomen, Ph für einen gegebenenfalls alkylsubsituierten Phenylrest und x, a1, a2, z1 und z2 unabhängig voneinander für 0 oder 1 stehen.

WO 02/83093 beschreibt kosmetische Zubereitungen, enthaltend Polyetherurethan-Verdicker gemäss Formel R1-(OCH2CH2)m-[CO-NH-CH2-CH2-CH2-CH2-CH2-CH2-NH-CO]x-(CH2CH2O)n-R2, in der R1 und R2 unabhängig voneinander für lineare oder verzweigte Alkyl und/oder Alkenylreste mit 6 bis 22 Kohlenstoffatomen, x für Zahlen von1 bis 3 und m und n unabhängig voneinander für Zahlen von 10 bis 100 stehen. Polyurethan-Verdicker für kosmetische Zubereitungen.

WO 2006/002 813 A offenbart Polyurethan-Verdicker für verschiedene Anwendungen in wässrigen Medien. Diese Verdicker werden aus hydrophilen Polyolen mit mindestens zwei Hydroxygruppen, ein oder mehr hydrophoben Verbindungen z.B. langkettigen Alkoholen und mindestens difunktionalen Isocyanaten hergestellt. Dabei wird ein Überschuß an NCO-Gruppen eingesetzt. Der bei der Herstellung eingesetzte Katalysator kann zinnhaltig, zinkhaltig oder ein Amin sein.

EP 0 725 097 B offenbart Polyurethan-Verdicker, bei deren Herstellung Polyether, erzeugt durch Alkoxylierung von Alkoholen oder Alkylphenolen, mit Polyisocyanaten umgesetzt werden, wobei das Verhältnis von NCO- zu OH-Äquivalenten im Bereich von 0,9:1 bis 1,2:1 liegt. Diese Verdicker werden für den Einsatz im Bereich niedriger Scherkräfte z.B. für den Verlauf von wässrigen Dispersionsfarben vorgeschlagen.

Eine Aufgabe der vorliegenden Erfindung war es, kosmetische Zubereitungen erhöhter Viskosität zur Verfügung zu stellen, deren rheologische Eigenschaften sich bei niedrigen und hohen Polyelektrolytkonzentrationen, pH-Wert- oder Temperatur--schwankungen über Zeiträume von mehreren Wochen nicht wesentlich verändern. Die kosmetischen Zubereitungen, insbesondere Emulsionen und Dispersionen sollten hinsichtlich ihrer chemischen und physikalischen Eigenschaften stabil sein. Die kosmetischen Zubereitungen sollten ein weiches, nicht fettiges und nicht klebriges Anfassgefühl vermitteln. Weiterhin sollten die kosmetischen Zubereitungen möglichst kosmetisch und dermatologisch akzeptabel sein, insbesondere sollten sie zinnfrei sein.

Die vorgenannten Aufgaben wurde gelöst durch die Bereitstellung einer Kosmetischen Zubereitung enthaltend enthaltend eine in Wasser dispergierbare Mischung von Polyurethanen (PU) mit einem im Wesentlichen linearen Rückgrat aufgebaut aus alternierenden hydrophilen und hydrophoben Abschnitten, wobei

a. die beiden endständigen Abschnitte (T) hydrophob sind,

b. sich an jeden Abschnitt T je ein hydrophiler Abschnitt (S) direkt anschließt,

c. sich an jeden Abschnitt S mindestens ein hydrophober Abschnitt (D) auf mindestens einer Seite direkt anschließt, und

d. wobei mindestens ein hydrophiler Abschnitt (P) enthalten ist, wobei mindestens ein hydrophober Abschnitt D zwei Abschnitte P trennt, falls mehr als ein Abschnitt P enthalten ist,

und die Polyurethane mindestens drei hydrophile Abschnitte umfassen, und das Verhältnis der Molekulargewichte eines jeden hydrophilen Abschnittes S zu dem Molekulargewicht eines jeden hydrophilen Abschnittes P von 1 zu 1,4 bis 1 zu 140 beträgt, die mindestens zwei hydrophoben Abschnitte D aliphatische Diisocyanatreste sind und der mindestens eine hydrophile Abschnitt P ein Polyetherrest mit einem zahlenmittleren Molekulargewicht von mindestens 1500 g/mol ist, wobei die Polyurethane eine Mischung darstellen, die Polyurethane enthält, deren Abschnitte T beide verzweigt sind, und solche, deren Abschnitte T beide linear sind, und solche, die einen linearen Abschnitt T und einen verzweigten Abschnitt T enthalten.

Erfindungsgemäß sind die Polyurethane in Wasser dispergierbar. Erfindungsgemäß umfasst "in Wasser dispergierbar" auch, dass die Polyurethane in Wasser emulgierbar oder vollständig oder teilweise löslich sind.

Bevorzugt weisen die in den erfindungsgemäßen Zubereitungen verwendeten Polyurethane PU die Eigenschaft auf, dass sie in einer Dispersion in Wasser bei Konzentrationen zwischen 0,1 und 10 g/L Mizellen mit einer mittleren Teilchengröße kleiner gleich 200 nm ausbilden, insbesondere kleiner gleich 100 nm (bestimmbar mittels dynamischer Lichtstreuung wie nachfolgend beschrieben). Daher kann auch von nanodispergierbaren Polyurethanen gesprochen werden. Die kritische Stoffkonzentration zur Mizellenbildung, auch kritische Mizellenkonzentration (im Englischen "Critical Micelle Concentration" CMC) ist demzufolge bevorzugt kleiner als 0,1 g/L.

Die in den erfindungsgemäßen Zubereitungen verwendeten Polyurethane weisen ein im Wesentlichen lineares Rückgrat auf, d.h. sie haben keine oder im Verhältnis zur Gesamtlänge wenige Verzweigungsstellen. Verzweigungen davon können in hydrophoben und/oder hydrophilen Abschnitten enthalten sein.

Die in den erfindungsgemäßen Zubereitungen verwendeten Polyurethane PU sind weder sternförmig noch vernetzt. Derartige Polyurethane und deren Herstellung sind aus dem Stand der Technik bekannt und nicht Teil dieser Erfindung.

Bevorzugt besitzen die in den erfindungsgemäßen Zubereitungen verwendeten Polyurethane pro Molekül kleiner gleich 4 Verzweigungen, besonders bevorzugt kleiner gleich 3 Verzweigungen pro Molekül. In einer besonders bevorzugten Ausführungsform weisen die in den erfindungsgemäßen Zubereitungen verwendeten Polyurethane außerhalb der randständigen Abschnitte T keine Verzweigungen auf. Dem Fachmann sind Methoden zur Verzweigungsbestimmung wie z.B. über NMR-Spektroskopie bekannt.

Das Rückgrat der in den erfindungsgemäßen Zubereitungen verwendeten Polyurethane ist aufgebaut aus alternierenden hydrophoben und hydrophilen Abschnitten, wobei die hydrophoben und hydrophilen Abschnitte sich zwar in der Abfolge abwechseln, aber in ihrer Größe, Länge und Natur unterschiedlich sein können. Ein hydrophiler Abschnitt schließt sich an beiden Seiten direkt an einen hydrophoben Abschnitt an. Diese hydrophoben Abschnitte können zueinander unabhängig gleich oder verschieden sein. Jeder Abschnitt kann kurzkettig oder ein Oligomerrest oder ein Polymerrest sein.

Hydrophil bezeichnet dabei solche Abschnitte, die eine ausgeprägte Wechselwirkung mit Wasser zeigen. Im Allgemeinen bestehen hydrophile Abschnitte aus Resten von Stoffen, die selbst hydrophil sind. Dem Fachmann bekannte typische hydrophile Gruppen sind nichtionische Polyether-Reste. Bevorzugte Polyether-Reste enthalten im Wesentlichen unverzweigte Alkylenoxidreste.

Polyether-Reste können homo-Alkylenoxidreste sein, oder Mischungen unterschiedlicher Alkylenoxidreste enthalten. Diese unterschiedlichen Alkylenoxidreste können statistisch verteilt in den Polyether-Resten vorhanden sein oder blockförmig vorliegen. Bevorzugte Polyether-Reste sind homo-Ethylenoxidreste oder homo-Propylenoxidreste. Nach einer anderen Ausführungsform enthalten die Polyether-Reste Mischungen aus Ethylenoxidresten und Propylenoxidresten. Diese können statistisch verteilt in den Polyether-Resten vorhanden sein oder blockförmig vorliegen.

Zu einer besonders bevorzugten Ausführungsform gehören Polyether-Reste, die mindestens 50 Gew.-% Ethylenoxidreste aufweisen, beispielsweise Polyether-Reste, die über 50 Gew.-% Ethylenoxidreste und als weitere Alkylenoxidreste Propylenoxidreste aufweisen. Ganz besonders bevorzugt bestehen die Polyether-Reste aus Ethylenoxidresten.

Die Hydrophilie eines Stoffes kann beispielsweise durch eine Trübungsmessung einer wässrigen Lösung bestimmt werden.

Die in den in den erfindungsgemäßen Zubereitungen verwendeten Polyurethanen enthaltenen hydrophoben Abschnitte verhalten sich gegenüber Wasser entgegengesetzt den hydrophilen Abschnitten. Im Allgemeinen bestehen die hydrophoben Abschnitte aus Resten von Substanzen die sich nicht oder nur sehr schlecht mit Wasser mischen und so gut wie immer lipophil sind, das heißt sie lösen sich gut in unpolaren Lösungsmitteln, Fetten und Ölen.

Typische hydrophobe Gruppen sind beispielsweise Kohlenwasserstoffreste, insbesondere langkettige Kohlenwasserstoffreste. Erfindungsgemäß sind unverzweigte oder gering verzweigte Kohlenwasserstoffreste bevorzugt. Nach einer der Ausführungsformen sind die Kohlenwasserstoffreste unverzweigt. Langkettige aliphatische Alkohole, aromatische Alkohole sowie aliphatische Diisocyanate sind Beispiele für hydrophobe Substanzen, deren Reste in den hydrophoben Abschnitten der in den erfindungsgemäßen Zubereitungen verwendeten Polyurethane enthalten sein können.

Ein Molekül, das sowohl hydrophobe wie auch hydrophile Abschnitte aufweist, wird im Allgemeinen als amphiphiles Molekül bezeichnet. Als Beispiele sind u.a. Phospholipide, Emulgatoren und Tenside zu nennen. Ein Maß für die Hydrophilie einer amphiphilen Verbindung stellt der HLB Wert dar. Der HLB-Wert (englische Abkürzung für: hydrophilic-lipophilic-balance) beschreibt den hydrophilen und lipophilen Anteil von hauptsächlich nichtionischen Tensiden und wurde im 20 Jahrhundert von W. C. Griffin vorgeschlagen (Griffin, W. C.: Classification of surface active agents by HLB, J. Soc. Cosmet. Chem. 1, 1949).

Der HLB-Wert kann folgendermaßen berechnet werden (siehe Formel I):

$$HLB = 20 * \left(1 - \frac{M_I}{M}\right) \quad \text{(Formel I)}$$

wobei $M_I$ die Molmasse des hydrophoben Anteils eines Moleküls ist und M die Molmasse des gesamten Moleküls. Der Faktor 20 ist ein von Griffin frei ausgewählter Skalierungsfaktor. Es ergibt sich damit in der Regel eine Skala von 1 bis 20. Ein HLB-Wert von 1 spricht für eine lipophile Verbindung, eine chemische Verbindung mit einem HLB-Wert von 20 hat einen hohen hydrophilen Anteil.

Die in den erfindungsgemäßen Zubereitungen verwendeten Polyurethane haben bevorzugt einen HLB Wert nach Griffin von größer gleich 7, besonders bevorzugt von größer gleich 14, auf einer Skala von 1 bis 20.

In den erfindungsgemäßen Zubereitungen verwendete Polyurethane enthalten mindestens zwei endständige hydrophobe Abschnitte (T). Die in den erfindungsgemäßen Zubereitungen verwendeten Polyurethane PU können in geringem Maße im Molekülinnern verzweigt sein (gewünschtenfalls durch Verwendung von Tri-oder Polyisocyanaten in geringen Anteilen), so dass dann mehr als zwei endständige hydrophobe Abschnitte T enthalten sein könnten. Bevorzugt sind die in den erfindungsgemäßen Zubereitungen verwendeten Polyurethane PU im Molekülinnern unverzweigt und enthalten zwei endständige hydrophobe Abschnitte T. Ihre Endständigkeit bedingt, dass sie sich nur an einen weiteren Abschnitt der in den erfindungsgemäßen Zubereitungen verwendeten Polyurethane direkt anschliessen.

Bevorzugt enthalten die endständigen hydrophoben Abschnitte T eine Kette aus Kohlenstoffatomen. Bevorzugt liegt die Kettenlänge der Abschnitte T im Bereich von 4 bis 30 Kohlenstoffatomen, besonders bevorzugt im Bereich von 6 bis 26 und ganz besonders bevorzugt im Bereich von 8 bis 20 Kohlenstoffatomen.

Derartige Abschnitte T können beispielsweise aus aromatischen Resten, aber auch aus Alkylresten bestehen. So können die Abschnitte T verzweigte oder unverzweigte Alkylreste sein, oder diese enthalten. Bevorzugt ist mindestens ein Abschnitt T ein verzweigter Alkylrest. Verzweigt bedeutet, dass an einem oder auch mehreren Kohlenstoffatomen des Alkylrests Verzweigungen ansetzen. Üblicherweise bedeutet eine Verzweigung eines Alkyls, dass neben den Mitgliedern der Hauptkette ein oder mehrere zusätzliche Kohlenstoffatome an ein oder zwei Stellen an einem Kohlenstoffatom des Kohlenstoffrückgrats kovalent gebunden sind und eine Seitenkette bilden. Die Seitenketten können identische oder unterschiedliche Größen haben. Bevorzugt sind die Seitenketten selbst Alkylreste oder Alkylenreste, besonders bevorzugt Alkylreste, insbesondere unverzweigte Alkylreste.

In einer Ausführungsform weisen die Seitenketten der Alkylreste bevorzugt eine Kettenlänge von nicht mehr als 6 Kohlenstoffatomen auf. In einer anderen Ausführungsform sind die Verzweigungen vorzugsweise deutlich kürzere Ketten als die Hauptkette. Vorzugsweise hat jede Verzweigung der Abschnitte T der in den erfindungsgemäßen Zubereitungen verwendeten Polyurethane höchstens eine Kettenlänge, die der Hälfte der Kettenlänge der Hauptkette dieses Abschnitts T entspricht. Besonders bevorzugt sind die verzweigten Alkylreste iso- und/oder neo-Alkylreste. Bevorzugt liegt die Kettenlänge der Hauptkette von Alkylresten, die in Abschnitten T enthalten sind, im Bereich von 4 bis 30 Kohlenstoffatomen, beispielsweise Alkylreste von Butan, Pentan, Hexan, Heptan, Octan, Nonan, Decan, Undecan, Dodecan, Tridecan, Tetradecan, Pentadecan, Hexadecan, Heptadecan, Octadecan, Nonadecan, Icosan, Henicosan, Docosan, Tricosan, Tetracosan, Pentacosan, Hexacosan, Heptacosan, Octacosan, Nonacosan und/oder Triacontan. Verzweigte Alkyreste dieser Alkane können verwendet werden. Ebenso können auch Reste von Cylcoalkanen oder Alkenen enthalten sein. Besonders bevorzugt enthalten die Abschnitte T Alkylreste mit einer Anzahl von Kohlenstoffatomen im Bereich von 6 bis 26, beispielsweise Reste von Hexan, Heptan, Octan, Nonan, Decan, Undecan, Dodecan, Tridecan, Tetradecan, Pentadecan, Hexadecan, Heptadecan, Octadecan, Nonadecan, Icosan, Henicosan, Docosan, Tricosan, Tetracosan, Pentacosan und/oder Hexacosan, und ganz besonders bevorzugt im Bereich von 8 bis 20 Kohlenstoffatomen, beispielsweise Reste von Octan, Nonan, Decan, Undecan, Dodecan, Tridecan, Tetradecan, Pentadecan, Hexadecan, Heptadecan, Octadecan, Nonadecan und/oder Icosan. Verzweigte Alkylreste dieser Alkane können genauso verwendet werden wie auch Reste von Cylcoalkanen oder Alkenen.

In einer bevorzugten Ausführungsform werden als verzweigte Alkylreste Reste von Iso-Alkanen verwendet. Besonders bevorzugt ist ein C13-Alkylrest, insbesondere ein iso-C13 Alkylrest.

Die Einführung der Abschnitte T in die in den erfindungsgemäßen Zubereitungen verwendeten Polyurethane kann auf verschiedene Weise erfolgen, beispielsweise als Teil von ethoxylierten Fettalkoholen.

Direkt anschliessend an jeden Abschnitt T befindet sich in den erfindungsgemäßen Zubereitungen verwendeten Polyurethanen ein hydrophiler Abschnitt (S). Der Abschnitt S wirkt abstandsgebend als sogenannter Spacer S. Eine gewisse räumliche Flexibilität der Abschnitte S ist gewünscht. Bevorzugt sind die hydrophilen Abschnitte unverzweigt. In den in den erfindungsgemäßen Zubereitungen verwendeten Polyurethanen PU können die Spacer S gleich oder unabhängig voneinander verschieden sein. In einer Ausführungsform sind die hydrophilen Abschnitte S von verschiedener Länge und linear.

In einer weiteren bevorzugten Ausführungsform haben die Abschnitte S der in den erfindungsgemäßen Zubereitungen verwendeten Polyurethane eine Kettenlänge von 5 bis 100 Atomen, bevorzugt von 6 bis 90 Atomen und besonders von 8 bis 80 Atomen, insbesondere Ketten von 15 bis 60 Atomen.

Die Abschnitte S können Reste von Alkylenoxiden enthalten. Bevorzugt liegt die Anzahl im Bereich von 2 bis 30 Alkylenoxidresten, besonders bevorzugt im Bereich von 3 bis 25 Alkylenoxidresten und ganz besonders bevorzugt im Bereich von 3 bis 20 Alkylenoxidresten.

Die mindestens zwei hydrophilen Abschnitte S der Polyurethane können jeweils Ethylenoxidreste sein. Die hydrophilen Abschnitte S enthalten in einer bevorzugten Ausführungsform Ethylenoxidreste, deren Anzahl im Bereich von 2 bis 30 Resten liegt, besonders bevorzugt im Bereich von 3 bis 25 Ethylenoxidresten und ganz besonders bevorzugt im Bereich von 3 bis 20 Resten.

Auch Mischungen aus Ethylenoxid- und Propylenoxidresten oder nur Propylenoxidresten in den Abschnitten S sind möglich.

Ebenso können die Abschnitte S längerkettige Alkylenoxide enthalten, wobei allerdings beachtet werden muss, dass die Abschnitte S insgesamt hydrophil sein müssen (z.B. durch einen entsprechend hohen Ethylenoxid-Anteil).

An jeden hydrophilen Abschnitt S schließt sich direkt auf mindestens einer Seite mindestens ein hydrophober Abschnitt (D) an. Dabei kann ein Abschnitt S auch im Molekülinnern der in den erfindungsgemäßen Zubereitungen verwendeten Polyurethane enthalten sein. In diesem Fall ist dieser Abschnitt S nicht wie ein randständiger Abschnitt S mit einem Abschnitt D und einem Abschnitt T, sondern auf mindestens zwei Seiten mit Abschnitten D verbunden. Bevorzugt ist ein Abschnitt S im Molekülinnern auf beiden Seiten mit je einem Abschnitt D verbunden. Für alle randständigen Abschnitte S gilt, dass sie mit einem endständigen Abschnitt T direkt verbunden sind.

Sollte ein Abschnitt S in geringem Maße verzweigt sein, so könnte er an zwei oder mehr Stellen mit hydrophoben Abschnitten D direkt verbunden sein. Bevorzugt schließen sich an jeden linearen hydrophilen Spacer S auf ein oder zwei Seiten je ein hydrophober Abschnitt D an.

In einer besonders bevorzugten Ausführungsform sind alle, d.h. insbesondere die zwei Abschnitte S unverzweigt, randständig, und mit einem Abschnitt T auf der einen Seite und einem Abschnitt D auf der anderen Seite verbunden.

Die in den erfindungsgemäßen Zubereitungen verwendeten Polyurethane enthalten mindestens zwei hydrophobe Abschnitte D. Die hydrophoben Abschnitte D können gleich oder unabhängig voneinander verschieden sein.

Die Abschnitte D können verzweigt mit kurzkettigen hydrophoben Verzweigungen oder unverzweigt sein. Bevorzugt sind die Abschnitte D unverzweigt.

Bevorzugt enthalten die Abschnitte D eine hydrophobe Kette von Kohlenstoffatomen, deren Länge im Bereich von 2 bis 20 Kohlenstoffatomen, bevorzugt 3 bis 16 Kohlenstoffatomen und insbesondere im Bereich von 4 bis 12 Kohlenstoffatomen liegt.

Bevorzugt enthalten die Abschnitte D Diisocyanatreste. Besonders bevorzugt enthalten die Abschnitte D Reste von aliphatischen Diisocyanaten. So kann beispielsweise ein hydrophober Abschnitt D aus einem oder mehreren aliphatischen Diisocyanatresten bestehen. Bevorzugt besteht ein Abschnitt D aus einem bis zehn aliphatischen Diisocyanatresten, besonders bevorzugt aus einem bis zu fünf aliphatischen Diisocyanatresten, ganz besonders bevorzugt enthält er ein, zwei oder drei aliphatische Diisocyanatreste.

Die hydrophoben Abschnitte D können aliphatische Diisocyanatreste mit langen, mittellangen oder kurzen aliphatischen Einheiten enthalten.

In einer der bevorzugten Ausführungsformen handelt es sich bei den Abschnitten D der in den erfindungsgemäßen Zubereitungen verwendeten Polyurethane um cycloaliphatische oder aliphatische Diisocyanatreste. Besonders bevorzugt sind die Abschnitte D aliphatische Diisocyanatreste.

Als aliphatische Diisocyanate seien beispielhaft genannt: 1,4-Butylendiisocyanat, 1,12-Dodecamethylendiisocyanat, 1,10-Decamethylendiisocyanat, 2-Butyl-2-ethylpentamethylendiisocyanat, 2,4,4- oder 2,2,4-Trimethylhexamethylendiisocyanat und insbesondere Hexamethylendiisocyanat (HDI).

Als cycloaliphatische Diisocyanate seien beispielhaft genannt: Isophorondiisocyanat (IPDI), 2-Isocyanatopropylcyclohexylisocyanat, 4-Methyl-cyclohexan-1,3-diisocyanat (H-TDI) und 1,3-Bis-(isocyanatomethyl)-cyclohexan. Auch sogenannte $H_{12}$-MDI oder im Englischen "saturated MDI" genannte Diisocyanate, wie z.B. 4,4'-Methylen-bis-(cyclohexylisocyanat) (alternativ auch Dicyclohexylmethan-4,4'-diisocyanat genannt) oder 2,4'-Methylenbis(cyclohexyl)-diisocyanat können als Reste in Abschnitten D der in den erfindungsgemäßen Zubereitungen verwendeten Polyurethane PU enthalten sein.

Selbstverständlich kann man Mischungen der vorstehend genannten Diisocyanate einsetzen, um Mischungen unterschiedlicher in den erfindungsgemäßen Zubereitungen verwendeter Polyurethane PU herzustellen.

Die in den erfindungsgemäßen Zubereitungen verwendeten Polyurethane enthalten mindestens einen hydrophilen Abschnitt (P). Dabei gilt, dass mindestens ein hydrophober Abschnitt D sich an P an mindestens einer Seite direkt anschließt. Die Abschnitte P der in den erfindungsgemäßen Zubereitungen verwendeten Polyurethane können gleich oder unabhängig voneinander verschieden sein.

Ist in einem in den erfindungsgemäßen Zubereitungen verwendeten Polyurethan mehr als ein Abschnitt P enthalten, so befindet sich zwischen den hydrophilen Abschnitten P mindestens ein hydrophober Abschnitt D. Die in den erfindungsgemäßen Zubereitungen verwendeten Polyurethane können in einer Ausführungsform zwischen zwei hydrophilen Abschnitten P eine Sequenz von Abschnitten der Reihenfolge hydrophober Abschnitt D, dann hydrophiler Abschnitt S, dann wieder hydrophober Abschnitt D enthalten. Sind in einem in den erfindungsgemäßen Zubereitungen verwendeten Polyurethan also mehr als ein Abschnitt P enthalten, so können in einem solchen Fall die Abschnitte im Molekülinnern eine Abfolge von P-D-P oder von P-D-S-D-P aufweisen. Sollten mehr als zwei Abschnitte P enthalten sein, so sind beide Abfolgen in einem Molekül möglich.

Bevorzugt sind in einem Molekül der in den erfindungsgemäßen Zubereitungen verwendeten Polyurethane nur ein oder zwei Abschnitte P enthalten.

Bevorzugt sind die hydrophilen Abschnitte P im Wesentlichen lineare Polyetherreste, z.B. Polyalkylenoxide. Besonders bevorzugt sind die hydrophilen Abschnitte P Reste von Polyetherdiolen, insbesondere von Polyethylenglykolen. Der mindestens eine hydrophile Abschnitt P der in den erfindungsgemäßen Zubereitungen verwendeten Polyurethane ist bevorzugt aus Polyethylenoxid aufgebaut.

Erfindungsgemäß haben die im Wesentlichen linearen Polyetherreste, die die Abschnitte P bilden, ein zahlenmittleres Molekulargewicht von mindestens 1500 g/mol aufzuweisen. Im Allgemeinen weisen die Abschnitte P Molekulargewichte mittlerer Größe auf, z.B. bis zu 20 000 g/mol.

In einer besonders bevorzugten Ausführungsform haben die im Wesentlichen linearen Polyetherreste zahlenmittlere Molekulargewichte im Bereich von 1500 g/mol bis 12000 g/mol. Besonders bevorzugt ist das Molekulargewicht der Abschnitte P kleiner oder gleich 10000 g/mol und insbesondere bevorzugt im Bereich von 4000 g/mol bis 9000 g/mol. Ganz besonders bevorzugt haben die linearen Polyetherreste Molekulargewichte von größer oder gleich 6000 g/mol.

Sämtliche hydrophile Abschnitte der in den erfindungsgemäßen Zubereitungen verwendeten Polyurethane, d.h. sowohl Abschnitte S wie auch Abschnitte P, können Polyetherreste sein.

In einer bevorzugten Ausführungsform bestehen die hydrophilen Abschnitte der in den erfindungsgemäßen Zubereitungen verwendeten Polyurethane aus

- Polyalkylenoxid-Einheiten (Abschnitte P) und
- Polyethylenoxid-Einheiten (Abschnitte S).

In einer besonders bevorzugten Ausführungsform der in den erfindungsgemäßen Zubereitungen verwendeten PU bestehen alle Abschnitte P und S aus Polyethylenoxid-Einheiten.

Das Rückgrat der in den erfindungsgemäßen Zubereitungen verwendeten Polyurethane enthält im Wesentlichen Reste von Polyethern und Diisocyanaten.

Die in den erfindungsgemäßen Zubereitungen verwendeten Polyurethane enthalten mindestens drei hydrophile Abschnitte. In einer der bevorzugten Ausführungsformen sind dies zwei Abschnitte S und mindestens ein Abschnitt P. In einer besonders bevorzugten Ausführungsform ist die Abfolge der Abschnitte der in den erfindungsgemäßen Zubereitungen verwendeten Polyurethane entweder T-S-D-P-D-S-T oder T-S-D-P-D-P-D-S-T.

Für jeden Abschnitt P gilt, dass seine Grösse im Vergleich zu der Grösse jedes im gleichen Molekül enthaltenen Spacers S grösser ist.

Das Verhältnis der Molekulargewichte eines jeden hydrophilen Abschnittes S der in den erfindungsgemäßen Zubereitungen verwendeten Polyurethane zu dem Molekulargewicht eines jeden hydrophilen Abschnittes P liegt im Bereich von 1 zu 1,4 bis 1 zu 140, bevorzugt im Bereich von 1 zu 1,7 bis 1 zu 120. In einer bevorzugten Ausführungsform ist das Verhältnis gleich 1 zu x, wobei x gleich oder grösser 2 ist, bevorzugt gleich oder grösser 2,3 und besonders bevorzugt ist x gleich oder grösser 2,8. Besonders bevorzugt liegt das Verhältnis im Bereich von 1 zu 2,8 bis 1 zu 115, ganz besonders bevorzugt im Bereich von 1 zu 3 bis 1 zu 95 und insbesondere bevorzugt im Bereich von 1 zu 3,4 bis 1 zu 80.

Ebenfalls erfindungsgemäß sind kosmetische Zubereitungen, die Polyurethane PU wie oben beschrieben enthalten, für die zusätzlich gilt, dass sie eine Mischung darstellen. Eine derartige Mischung kann z.B. Polyurethane enthalten, die zwar die gleiche Abfolge der Abschnitte T, S, D und / oder P aufweisen, wobei sie sich aber in mindestens einem der Abschnitte strukturell voneinander unterscheiden. Als Beispiel hierfür sei ein unterschiedlicher Abschnittsaufbau oder eine unterschiedliche Abschnittskettenlänge genannt. So können in einer Mischung von Polyurethanen PU Abschnitte T verschieden sein. Beispielsweise kann eine in den erfindungsgemäßen kosmetischen Zubereitungen enthaltene Mischung Polyurethane enthalten, deren Abschnitte T beide verzweigt sind, und solche, deren Abschnitte T beide linear sind, und solche Polyurethane, die einen linearen Abschnitt T und einen verzweigten Abschnitt T enthalten. Selbstverständlich können solche Mischungen auch andere Stoffe wie z.B. weitere, bevorzugt wasserdispergierbare Polyurethane enthalten.

Eine solche Mischung von Polyurethanen PU kann durch den Einsatz entsprechend verschiedener Einsatzstoffe bzw. deren Mischungen bei der Herstellung der in den erfindungsgemäßen Zubereitungen verwendeten Polyurethane PU, erfolgen, oder durch nachträgliche Mischung nur einheitlich hergestellter in den erfindungsgemäßen Zubereitungen verwendeter Polyurethane erzeugt werden.

In einer Ausführungsform ist die Summe der Molekulargewichte aller Abschnitte T, plus der Molekulargewichte von Abschnitten D kleiner gleich der Summe der Molekulargewichte aller Abschnitte P zu halten.

Man kann die in den erfindungsgemäßen Zubereitungen verwendeten Polyurethane PU in Abwesenheit oder vorzugsweise in Anwesenheit mindestens eines Katalysators herstellen.

Als Katalysatoren kommen beispielsweise alle in der Polyurethanchemie üblicherweise verwendeten Katalysatoren in Betracht.

Besonders bevorzugt verwendet man solche Katalysatoren, welche in organischen Lösungsmitteln wie Xylol, Toluol, Aceton, Tetrahydrofuran (THF), Butylacetat, N-Methylpyrrolidon und/oder N-Ethylpyrrolidon löslich sind.

Üblicherweise in der Polyurethanchemie verwendete Katalysatoren sind organische Amine, insbesondere tertiäre aliphatische, cycloaliphatische oder aromatische Amine, und Lewis-saure organische Metallverbindungen.

Als Lewis-saure organische Metallverbindungen kommen z.B. Metallkomplexe wie Acetylacetonate des Eisens, Titans, Zinks, Aluminiums, Cobalts, Mangans, Nickels und Zirkons in Betracht wie z.B. Zirkon-2,2,6,6-tetramethyl-3,5-heptandionat. Weitere geeignete Metallverbindungen werden von Blank et al. in Progress in Organic Coatings, 1999, 35, 19 ff. beschrieben.

Auch Wismut-, Cobalt- oder Zinkkatalysatoren sowie Cäsium- oder Titansalze können als Katalysatoren eingesetzt werden.

Bevorzugt erfolgt die Herstellung der in den erfindungsgemäßen Zubereitungen verwendeten Polyurethane PU in Gegenwart von Zink- und/oder Titan haltigen Verbindungen. Besonders bevorzugt ist die Gegenwart von mindestens einem Zink-Carboxylat oder mindestens einem Titan (IV)-alkoholat oder Mischungen derselben bei der Herstellung der in den erfindungsgemäßen Zubereitungen verwendeten Polyurethane PU.

Beispielsweise werden Titanalkoholate, bevorzugt mit einer Kettenlänge von 2 oder mehr Kohlenstoffatomen, eingesetzt. In einer bevorzugten Ausführungsform weisen die Titanalkoholate eine Kohlenstoffkette von 20 oder weniger Kohlenstoffatomen auf. Bevorzugt liegt die Kettenlänge der Titanalkoholate im Bereich von 3 bis 18 Kohlenstoffatomen. Besonders bevorzugt sind Titanalkoholate basierend auf aliphatischen Alkoholen. In einer insbesonders bevorzugten Ausführungsform erfolgt die Herstellung der in den erfindungsgemäßen Zubereitungen verwendeten Polyurethane PU in Gegenwart von Orthotitansäuretetrabutylester, auch als Titan (IV)-butylat oder Tetrabutoxytitan bekannt.

In einer bevorzugten Ausführungsform werden Zink-Carboxylate als Katalysatoren eingesetzt, die in Aceton, Toluol, Xylol und/oder aliphatischen Kohlenwasserstoffen löslich sind.

In einer weiteren bevorzugten Ausführungsform erfolgt die Herstellung der in den erfindungsgemäßen Zubereitungen verwendeten Polyurethane PU in Gegenwart von mindestens einem Zink-Carboxylat, bei denen das Anion den Formeln $(C_nH_{2n-1}O_2)$- oder $(C_{n+1}H_{2n-2}O_4)^{2-}$ mit n gleich 1 bis 20 gehorcht. Besonders bevorzugte Zinksalze weisen als Anionen Monocarboxylate der allgemeinen Formel $(C_nH_{2n-1}O_2)^-$ auf, wobei n für die Zahlen 1 bis 20 steht. Bevorzugt werden die in den erfindungsgemäßen Zubereitungen verwendeten Polyurethane PU hergestellt in Gegenwart von Zink-Carboxylaten, die aliphatische oder aromatische Carboxylate sind, und gewünschtenfalls ein oder zwei Ringstrukturen enthalten können.

In einer besonders bevorzugten Ausführungsform werden als Katalysatoren für die Herstellung der in den erfindungsgemäßen Zubereitungen verwendeten Polyurethane PU Zink-Carboxylate bevorzugt, deren Carbonsäurereste eine Kohlenstoffkette von 20 oder weniger, bevorzugt 18, besonders bevorzugt kleiner gleich 12 oder weniger Kohlenstoffatomen aufweisen, da gefunden wurde, dass bei langkettigen Carboxylatresten die Aktivität des Katalysators im erfindungsgemäßen Verfahren abnimmt.

In einer Ausführungsform können Zink-Carboxylate ohne Ringstruktur als Katalysatoren zur Herstellung der in den erfindungsgemäßen Zubereitungen verwendeten Polyurethane verwendet werden. Besonders bevorzugt werden aliphatische Zink-Carboxylate als Katalysatoren verwendet.

Ganz besonders bevorzugt werden als Katalysatoren Zink-2-Ethylhexanoat (auch Zink-Octanoat genannt), Zink-n-Octanoat, Zink-n-Decanoat, Zink- Neodecanoat, Zink-Ricinoleat und Zink-Stearat. Insbesonders bevorzugt wird Zink- Neodecanoat verwendet.

Selbstverständlich können auch Gemische aus zwei oder mehreren der vorstehend genannten Verbindungen als Katalysatoren zur Herstellung der in den erfindungsgemäßen Zubereitungen verwendeten Polyurethane PU eingesetzt werden. Bevorzugt wird nur ein Katalysator verwendet.

Die Menge des eingesetzten Katalysators spielt an sich keine Rolle. Im Allgemeinen wird eine wirtschaftliche Menge an Katalysator verwendet. Daher setzt man den Katalysator oder das Gemisch der Katalysatoren vorzugsweise in einer Menge im Bereich von 100 ppm bis 10 000 ppm bezogen auf eingesetzte Polyetherdiole auf einer Gewichtsbasis ein. Bevorzugt wird der Katalysator in einer Menge im Bereich von 500 bis 5000 ppm, besonders bevorzugt in einer Menge gleich oder kleiner 4500 ppm bezogen auf das Gewicht der Gesamtmenge aller eingesetzten Polyetherdiole eingesetzt. In einer besonders bevorzugten Ausführungsform wird der Katalysator in einer Menge im Bereich von 1000 ppm bis 3000 ppm bezogen auf bezogen auf das Gewicht der Gesamtmenge aller eingesetzten Polyetherdiole verwendet.

Man kann -je nach Beschaffenheit des Katalysators oder der Katalysatoren - den oder die Katalysatoren in fester oder flüssiger Form oder gelöst zusetzen. Geeignete Lösemittel sind mit Wasser nicht mischbare Lösungsmittel wie aromatische oder aliphatische Kohlenwasserstoffe, u.a. Toluol, Xylol, Ethylacetat, Hexan und Cyclohexan, sowie Carbonsäureester wie beispielsweise Ethylacetat. Weiterhin geeignete Lösemittel sind Aceton, THF und N-Methylpyrrolidon und N-Ethylpyrrolidon. Bevorzugt setzt man den oder die Katalysatoren in fester oder flüssiger Form zu. Bevorzugt setzt man den Katalysator gelöst in einem Lösemittel ein, ganz besonders bevorzugt gelöst in organischen Lösungsmitteln wie aliphatischen Kohlenwasserstoffen, Aceton, Toluol oder Xylol.

In einer besonders bevorzugten Ausführungsform werden der oder die Katalysatoren in gelöster Form eingesetzt.

In einer weiteren besonders bevorzugten Ausführungsform werden Zink-Carboxylate als Katalysator eingesetzt, die in aliphatischen Kohlenwasserstoffen, Aceton, Toluol, Xylol bzw. optional deren Gemischen gelöst sind.

Die in den erfindungsgemäßen Zubereitungen verwendeten Polyurethane PU werden durch ein erfindungsgemäßes Verfahren hergestellt, bei dem die Synthese in zwei Stufen erfolgt. Gewünschtenfalls folgt der zweiten Reaktionsstufe eine Aufarbeitung der Produkte.

Prinzipiell kann die Reaktion auch ohne Katalysator durchgeführt werden, allerdings sind die Produkte im Allgemeinen schlechter reproduzierbar (betrifft z.B. die zahlenmittleren und gewichtsmittleren Molekulargewichte), die Reaktionszeiten sind im Allgemeinen deutlich länger und die erzielten Viskositäten in Zubereitungen, die Wasser enthalten, sind z.T. geringer. In einigen Fällen trat durch die verstärkte Bildung von (hochmolekularen) Nebenprodukten Vernetzung auf, wenn kein Katalysator anwesend war. Bevorzugt wird daher mindestens ein, besonders bevorzugt genau ein Katalysator eingesetzt.

Ein Vorteil der Verfahren zur Herstellung von in den erfindungsgemäßen Zubereitungen verwendeten Polyurethanen PU in dieser bevorzugten Ausführungsform ist die Tatsache, dass das Produkt einheitlich strukturierte Moleküle oder eine klar definierte Mischung von Polyurethanmolekülen enthält.

Das Verfahren zur Herstellung von in den erfindungsgemäßen Zubereitungen verwendeten Polyurethanen PU kann in einer Ausführungsform die folgenden Schritte enthalten:

1. Man setzt mindestens ein Polyetherdiol mit einem Molekulargewicht von mindestens 1500 g/mol mit mindestens einem aliphatischem Diisocyanat und in Gegenwart mindestens eines Zink-Carboxylates und/oder mindestens eines Titanalkoholates um;
2. anschließend setzt man die erzeugten Zwischenprodukte mit mindestens einem ethoxylierten Fettalkohol um;
3. danach erfolgt die Aufarbeitung, d.h. in der Regel die Entfernung aller org. Lösungsmittel und die Überführung des Polymers in Wasser.

Die Umsetzung der Einsatzstoffe kann in Lösung erfolgen. Es ist auch eine Reaktion in Schmelze möglich, bei der die Einsatzstoffe nicht oder zum größten Teil nicht gelöst in Lösungsmittel vorliegen.

In einer bevorzugten Ausführungsform wird die Umsetzung in zwei Schritten in Lösung durchgeführt, insbesonders bevorzugt gelöst in organischen Lösungsmitteln wie Aceton, Toluol oder Xylol.

Bevorzugt wird möglichst wasserfreies Polyetherdiol im ersten Schritt eingesetzt. Das Entfernen des Wassers aus dem Polyether kann durch azeotrope Destillation, Trocknen unter Vakuum oder andere dem Fachmann bekannte Methoden erfolgen. Beispielsweise kann durch azeotrope Destillation Wasser entfernt werden, bis der Wassergehalt vor Zugabe der Diisocyanate bei ungefähr 300 ppm liegt. Die Vorbereitung der eigentlichen Reaktion kann beispielsweise daraus bestehen, dass

entweder das Polyetherdiol unter Vakuum gesetzt und so das Wasser ausreichend (bevorzugt bis zu einem Wassergehalt von ungefähr 300 ppm oder niedriger) entfernt wird, und danach ein Lösungsmittel beigemischt wird, oder das Polyetherdiol mit einem Lösungsmittel wie Xylol, Toluol oder Aceton vermischt wird und das Wasser durch azeotrope Destillation entfernt wird, beispielsweise bis zu einem Wassergehalt von ungefähr 300 ppm, wobei allerdings das Lösungsmittel nicht vollständig entfernt wird, sondern die Lösung von Polyether im verbliebenen Lösungsmittel zur Reaktion in Lösung verwendet wird.

Der pH Wert der Diollösung in Lösungsmittel kann vor der Umsetzung mit Diisocyanaten auf einen Wert von kleiner gleich pH 7 eingestellt und gewünschtenfalls abgepuffert werden, beispielsweise durch Entsalzen oder Zugabe von einer oder einer Mischung unterschiedlicher Säuren. Als Säuren kommen anorganische oder organische Säuren in Betracht, z.B. Salzsäure, Schwefelsäure, Schwefelige Säure, Salpetersäure, Phosphorsäure, Flussäure, Kohlensäure, organische Säuren wie Äpfelsäure, Zitronensäure, Oxalsäure, Ameisensäure, Essigsäure, Propionsäure, Buttersäure.

Die eingesetzten ethoxylierten Fettalkohole haben vorzugsweise einen Ethoxylierungsgrad, der mindestens im Bereich von 2 bis 30 Resten liegt, besonders bevorzugt im Bereich von 3 bis 25 Ethylenoxidresten und ganz besonders bevorzugt im Bereich von 3 bis 20 Resten. Am meisten bevorzugt wird als mindestens einer der eingesetzten Fettalkohole eine verzweigte, nicht-ionische Verbindung, hergestellt aus einem gesättigtem iso- C13 Alkohol der Strukturformel $RO(CH_2CH_2O)_x$ H, wobei R für einen C13 Alkylrest, bevorzugt einen iso-C13-Alkylrest steht, und mit x = 3,5,6,6.5,7,8,10,12,15 oder 20, bevorzugt x =10 (kommerziell erhältlich von der Firma BASF SE unter der Bezeichnung "Lutensol®TO" z.B. für x=10 als "Lutensol®T010"), verwendet.

Das Verhältnis (mol zu mol) der eingesetzten Polyetherdiole zu eingesetzten Diisocyanaten kann im Bereich von 1 zu 1,1 bis 1 zu 1,9 liegen. Bevorzugt liegt das Verhältnis im Bereich von 1 zu 1,1 bis 1 zu 1,8. Besonders bevorzugt liegt das Verhältnis im Bereich von 1 zu 1,1 bis 1 zu 1,75. Insbesonders bevorzugt liegt das Verhältnis im Bereich von 1 zu 1,2 bis 1 zu 1,75. Selbstverständlich kann das Verhältnis auch 1 zu x mit x größer oder gleich 1,3, bevorzugt x grösser oder gleich 1,5, sein.

In einer Ausführungsform ergibt sich daraus, dass bevorzugt in einem Molekül der in den erfindungsgemäßen Zubereitungen verwendeten Polyurethane nur ein oder zwei Abschnitte P enthalten sind.

In einer speziellen Ausführungsform des Herstellverfahrens wird zusätzlich zu den genannten Bereichen des Verhältnisses von Polyetherdiolen zu Diisocyanaten das Verhältnis von Polyetherdiolen zu ethoxylierten Fettalkoholen so gewählt, dass das Verhältnis (mol zu mol) von eingesetzten Polyetherdiolen zu eingesetzten ethoxylierten Fettalkoholen im Bereich von 5 zu 1 bis 1 zu 2 liegt. Bevorzugt liegt dieses Verhältnis (mol zu mol) im Bereich von 2 zu 1 bis 1 zu 1,8, besonders bevorzugt im Bereich von 1 zu 1 bis 1 zu 1,6 und am meisten bevorzugt bei 1 zu 1,5.

Für alle drei Einsatzstoffe gilt, dass ganz besonders bevorzugt ein Verhältnis (mol zu mol) von Polyetherdiolen zu Diisocyanaten zu ethoxylierten Fettalkoholen 1 zu 1,75 zu 1,5 eingesetzt wird.

Bevorzugte erfindungsgemäße kosmetische Zubereitungen, die die vorher beschriebenen Polyurethane PU enthalten, sind solche, die weiterhin Wasser enthalten. Bevorzugt werden dabei kosmetische Zubereitungen, die mindestens 5 Gew.-%, insbesondere mindestens 20 Gew.-%, ganz besonders bevorzugt mindestens 30 Gew.-% und am meisten bevorzugt mindestens 50 Gew.-% Wasser enthalten. Bei den Wasser enthaltenden kosmetischen Zubereitungen kann es sich beispielsweise um Lösungen, Emulsionen, Suspensionen oder Dispersionen handeln.

Eine bevorzugte Ausführungsform der Erfindung sind Zubereitungen, die neben den Polyurethanen PU Wasser und mindestens ein Salz oder mindestens ein Tensid oder Mischungen derselben enthalten.

Unter Tensiden werden im Zusammenhang mit der vorliegenden Erfindung auch Emulgatoren sowie Mischungen aus Tensiden und Emulgatoren verstanden. Unter Salz werden im Zusammenhang mit der vorliegenden Erfindung Salze und auch salzartige Strukturen auch mit niedrigem $pK_S$ Wert und Mischungen derselben verstanden.

Besonders bevorzugt sind erfindungsgemäße Zubereitungen, die neben den Polyurethanen PU mindestens 0,05 Gew.-% Salz und/oder mindestens 0,5 Gew.-% Tenside enthalten, ganz besonders bevorzugt mindestens 0,1 Gew.-% Salz und/oder mindestens 1 Gew.-% Tenside enthalten.

Einer weitere Ausführungsform sind Zubereitungen, die neben den Polyurethanen PU bis 20 Gew.-% Salz, bevorzugt bis 10 Gew.-% und besonders bevorzugt bis 5 Gew.-% Salz enthalten.

Eine weitere Ausführungsform sind Polyurethan PU enthaltende Zubereitungen, die bis 25 Gew.-% Tenside, bevorzugt bis 20 Gew.-% und besonders bevorzugt 15 Gew.-% Tenside enthalten.

Eine weitere Ausführungsform sind Polyurethan PU enthaltende Zubereitungen, die bis 10 Gew.-% Salz, bevorzugt bis 5 Gew.-% Salz und bis 20 Gew.-% Tenside, bevorzugt bis 15 Gew.-% Tenside, enthalten.

Eine weitere Ausführungsform sind salzfreie bzw. salzreduzierte Tensidsysteme.

Eine besonders bevorzugte Ausführungsform sind Polyurethan PU enthaltende Zubereitungen in Form von Öl-in-Wasser-Emulsionen (O/W-Emulsionen). Typischerweise enthalten Öl-in-Wasser-Emulsionen einen Ölanteil größer 0 Gew.-% und kleiner gleich 40 Gew.-%. Bevorzugt sind Öl-in-Wasser-Emulsionen, die einen Ölanteil im Bereich von 5 bis 40 Gew.-%, besonders im Bereich von 10 bis 35 Gew.-% und insbesondere von 15 bis 30 Gew.-% Öl enthalten. Ganz besonders bevorzugt sind Polyurethane PU enthaltende Zubereitungen, die Ölin-Wasser-Emulsionen sind und mindestens ein Salz enthalten.

Die erfindungsgemäßen kosmetischen Zubereitungen enthalten die Polyurethane PU bevorzugt in einer Menge von 0,01 bis 10 Gew.-%, bevorzugt 0,05 bis 5 Gew.-%, besonders bevorzugt 0,1 bis 1,5 Gew.-%, bezogen auf das Gewicht der Zubereitung.

Zur Herstellung der erfindungsgemäßen Zubereitungen, die beispielsweise Lösungen, Emulsionen, Suspensionen oder Dispersionen sein können, werden die Polyurethane PU bevorzugt in Form wässriger Dispersionen eingesetzt., wie sie aus dem Herstellungsprozess durch Aufarbeitung (beispielsweise durch Entfernen des Lösungsmittels, Zugabe von Wasser und gewünschtenfalls durch Zugabe eines Konservierungsmittels und/oder eines Stabilisators) erhalten werden können.

Bevorzugt werden in den kosmetischen oder dermatologischen Zubereitungen Polyurethane PU verwendet, deren 10-gewichtsprozentige wässrige Dispersionen eine dynamische Viskosität, gemessen wie nachfolgend beschrieben bei einer Schergeschwindigkeit von 100 1/s, von mindestens 100 mPa*s, besonders bevorzugt von mindestens 200 mPa*s und ganz besonders bevorzugt von mindestens 300 mPa*s aufweisen. Die wässrigen Dispersionen der Polyurethane PU können dabei entweder newtonsches oder aber strukturviskoses Verhalten zeigen. Strukturviskose Dispersionen, welche die Polyurethane PU enthalten, weisen bevorzugt dynamische Viskositäten von mindestens 1000 mPa*s, besonders bevorzugt sogar von mindestens 3000 mPa*s auf (10 Gew.-%ige wässrige Dispersionen, gemessen wie nachfolgend beschrieben bei einer Schergeschwindigkeit von 100 1/s).

Dem Fachmann ist bekannt, dass in Wasser enthaltenden Zubereitungen viele Verdickungsmittel ihre Wirkung einbüssen, d.h. die Viskosität der Zubereitung sinkt, sobald die Zubereitungen außerdem Salz und/oder Tensid enthalten. Dahingegen führen die Polyurethane PU in einer bevorzugten Ausführungsform zu einer Stabilisierung der Viskosität von Wasser enthaltenden Zubereitungen auch bei Zugabe von Salz und/oder Tensid.

In einer weiteren Ausführungsform wird die Viskosität von Wasser enthaltenden Zubereitungen, die mindestens ein Salz enthalten, durch das Vorhandensein der Polyurethane PU in der Zubereitung im Vergleich zu Zubereitungen, die nur Salz oder nur Polyurethane PU enthalten, in etwa konstant gehalten oder sogar gesteigert. Dabei ist die Reihenfolge unwesentlich, in der Polyurethane PU und Salz zugegeben werden.

Besonders bevorzugt sind Polyurethane PU, die zu einer hohen Toleranz oder gar Erhöhung der dynamischen Viskosität, gemessen wie nachfolgend beschrieben, von Wasser enthaltenden Zubereitungen führen, wenn mindestens ein Salz oder mindestens ein Tensid oder bevorzugt Mischungen derselben in den Zubereitungen enthalten sind. Besonders bevorzugt ist die Verwendung von Polyurethanen PU, die bei einer Salzkonzentration von größer oder gleich 0,5 Gew.-% nach Zugabe zu einer Stabilisierung der dynamischen Viskosität, gemessen wie nachfolgend beschrieben, von Wasser enthaltenden Zubereitungen führen. Besonders bevorzugt ist die Verwendung von Polyurethanen PU, die zu einer Stabilisierung der dynamischen Viskosität bei Zugabe von größer gleich 0,5 Gew.-% Salz und Zugabe von größer gleich 1 Gew.-% Tensid führen, wobei die Reihenfolge der Zugaben gewünschtenfalls unerheblich ist.

Besonders bevorzugt ist die Verwendung von Polyurethanen PU, die zu einer Erhöhung der dynamischen Viskosität, gemessen wie nachfolgend beschrieben, von Wasser enthaltenden Zubereitungen führen, wenn mindestens ein Salz oder mindestens ein Tensid oder Mischungen derselben in den Zubereitungen enthalten sind. Insbesondere bevorzugt sind Polyurethane PU, die bei einer Salzkonzentration von größer oder gleich 0,5 Gew.-% zu einer Erhöhung der dynamischen Viskosität, gemessen wie nachfolgend beschrieben, von Wasser enthaltenden Zubereitungen führen. Besonders bevorzugt sind solche Polyurethane, die zu einer Steigerung der dynamischen Viskosität im Vergleich zu Zubereitungen, die weniger als 0,5 Gew.-%, bevorzugt 0,1 Gew.-%, Salz, oder weniger als 1 Gew.-%, bevorzugt 0,5 Gew.-% Tensid enthalten, führen.
Ganz besonders bevorzugt sind Polyurethane PU, die bei einer Salzkonzentration von größer oder gleich 0,05 Gew.-% zu einer Erhöhung der dynamischen Viskosität, gemessen wie nachfolgend beschrieben, von Wasser enthaltenden Zubereitungen führen. Besonders bevorzugt sind solche Polyurethane, die zu einer Steigerung der dynamischen Viskosität im Vergleich zu Zubereitungen, die weniger als 0,05 Gew.-%, bevorzugt kleiner gleich 0,01 Gew.-%, Salz, oder weniger als 0,5 Gew.-%, bevorzugt kleiner gleich 0,1 Gew.-% Tensid enthalten, führen.

Eine Ausführungsform der Erfindung sind erfindungsgemäße kosmetische Zubereitungen, die wenigstens 0,5 Gew.-% wenigstens eines Salzes und wenigstens 1 Gew.-% wenigstens eines Tensids enthalten.

Die Wirkungsweise der zuvor beschriebenen Polyurethane PU ist weitgehend unabhängig von der Ladungsdichte bzw. Ionenstärke der sonstigen Inhaltsstoffe der erfindungsgemäßen Zubereitungen. Die Effektivität der Wirkung der Polyurethane PU ist bei ein- oder mehrwertigen Ionen vergleichbar.

pH-Wert-Bereich

Die zuvor beschriebenen Polyurethane PU können im Gegensatz zu vielen anderen Rheologiemodifizierern in einem großen pH-Wert-Bereich von pH=2 bis pH=12 verwendet werden.

Ein weiterer Vorteil der Polyurethane ist die Mizellenbildung in Wasser. Die kritische Stoffkonzentration zur Mizellenbildung, auch kritische Mizellenkonzentration (im Englischen "Critical Micelle Concentration" CMC) genannt, gibt die kleinstmögliche Konzentration eines Stoffes, meist eines Stoffes der hydrophobe und hydrophile Abschnitte innehat, an, bei der spontan Mizellen gebildet werden. Die CMC der Polyurethane PU in Wasser, ermittelt wie nachfolgend beschrieben, ist bevorzugt kleiner gleich 1 g/L, besonders bevorzugt kleiner gleich 0,5 g/L, insbesondere bevorzugt kleiner gleich 0,25 g/L und ganz besonders bevorzugt kleiner gleich 0,1 g/L.

Ein weiterer Vorteil der erfindungsgemäßen Zubereitungen ist die bevorzugte Verwendung von Zink- und/oder Titanhaltigen Katalysatoren bei der Herstellung der Polyurethane PU. Gerade im Bereich von kosmetischen Zubereitungen sind die aus dem Stand der Technik bekannten Verfahren mit Zinn nicht länger erwünscht, da auch in den Produkten und daraus resultierenden Zubereitungen Zinn enthalten sein kann. Zinkhaltige Zusatzstoffe von kosmetischen Zubereitungen sind akzeptiert, wobei Zink durch seine antibakteriellen und entzündungshemmenden Eigenschaften zusätzliche Vorteile einbringen kann.

Die erfindungsgemäßen kosmetischen oder dermatologischen Zubereitungen enthalten wenigstens ein Polyurethan PU und wenigstens einen kosmetisch akzeptablen Träger.

Der kosmetisch akzeptable Träger ist vorzugsweise ausgewählt aus

i) Wasser,
ii) wassermischbaren organischen Lösungsmitteln, vorzugsweise $C_2$-$C_4$-Alkanolen, insbesondere Ethanol,
iii) Ölen, Fetten, Wachsen,
iv) von iii) verschiedenen Estern von $C_6$-$C_{30}$-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
ix) Mischungen davon.

So sind beispielsweise hydrophile Träger wie Wasser oder ein-, zwei- oder mehrwertige Alkohole mit vorzugsweise 1 bis 8 Kohlenstoffatomen, wie Ethanol, n-Propanol, iso-Propanol, Propylenglykol, Glycerin und Sorbit geeignete kosmetisch akzeptable Träger.

Diole

Besonders geeignet als hydrophile Träger sind Diole. Diole können vorteilhaft in Kombination mit Polyurethan PU in kosmetische Zubereitungen eingearbeitet werden, wodurch eine Effektivitätssteigerung von Polyurethan PU beobachtet werden kann. Insbesondere der Einsatz von Propylenglykol in Gegenwart von PU ist vorteilhaft. Vorteilhafte Einsatzkonzentrationen der Diole, bevorzugt von von Propylenglykol, liegen im Bereich von 1 bis 10 Gew.-%, bezogen auf das Gesamtgewicht der Zubereitung.
Ein weiterer Gegenstand der Erfindung sind also erfindungsgemäße kosmetische Zubereitungen enthaltend Polyurethan PU und im Bereich von 1 bis 10 Gew.-% wenigstens eines Diols.

Die erfindungsgemäßen Zubereitungen können als wässrige oder wässrigalkoholische Lösungen, O/W- (bevorzugt) sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Bei den erfindungsgemäßen kosmetischen Zubereitungen kann es sich um hautkosmetische, haarkosmetische, dermatologische, hygienische oder pharmazeutische Zubereitungen handeln. Vorzugsweise liegen die erfindungsgemäßen Zubereitungen in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.
Insbesondere können unterschiedliche Darreichungsformen einer sprühbaren oder dünnflüssigen Formulierung durch Zusatz unterschiedlicher Mengen PU während oder nach der Herstellung der sprühbaren oder dünnflüssigen Formulierung erzielt werden.
In einer Ausführungsform der Erfindung wird eine niedrigviskose kosmetische Zusammensetzung, die bereits alle gewünschten sonstigen Bestandteile enthält, hergestellt. Nachdem alle gewünschten sonstigen Bestandteile zugegeben worden sind, wird zur Einstellung der gewünschten Viskosität die benötigte Menge Polyurethan PU zugegeben. So kann beispielsweise ein Teil einer dünnflüssigen Formulierung ohne Zugabe von Polyurethan PU als Spray konfektioniert werden und ein weiterer Teil kann durch Zugabe von Polyurethan PU als Lotion und durch nochmalige Zugabe von Polyurethan PU schließlich als Creme konfektioniert werden. Bei der nachträglichen Zugabe von Polyurethan PU muss die Viskositätseinstellung nicht notwendigerweise durch ein Nachhomogenisieren erfolgen; im Gegensatz zu den etablierten Verdickersystemen genügt zur Erzielung des gewünschten Effektes einfaches Einrühren. Die Möglichkeit, die gewünschte Viskosität von kosmetischen Zubereitungen nachträglich, also nachdem alle Bestandteile mit Ausnahme des Verdickers bereits enthalten sind, durch Zugabe des Verdickers einzustellen, ist einer der Vorteile der Polyurethane PU.

Die Zugabe von Polyurethan PU kann somit während oder nach der Herstellung der Zubereitung, die alle sonst gewünschten Bestandteile enthält, erfolgen.

Die erfindungsgemäßen kosmetischen Zubereitungen in Form von Öl-in-Wasser- oder Wasser-in-Öl-Emulsionen können durch Mischen der entsprechenden Öl- und Wasserphasen hergestellt werden, in dem eine der beiden Phasen heiß und die andere kalt ist, beide Phasen kalt oder beide Phasen heiß sind. "Heiß" bedeutet dabei eine Temperatur von etwa 70°C bis 80°C, "kalt" bedeutet eine Temperatur von etwa 20°C bis 30°C.

Das Polyurethan PU kann in der Öl- und/oder der Wasserphase vorliegen, wobei es bevorzugt in der Wasserphase vorliegt.

Die Erfindung betrifft bevorzugt kosmetische Zubereitungen, die ausgewählt sind aus Gelen, Gelcremes, Milche, Hydroformulierungen, Stiftformulierungen, kosmetischen Ölen und Ölgelen, Mascara, Selbstbräunern, Gesichtspflegemitteln, Körperpflegemitteln, After-Sun-Präparaten. Unter dem Begriff kosmetische Zubereitungen werden auch Zubereitungen für die Mundpflege verstanden.

Weitere erfindungsgemäße kosmetische Zubereitungen sind hautkosmetische Zubereitungen, insbesondere solche zur Pflege der Haut. Diese liegen insbesondere als W/O- oder bevorzugt O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weitere bevorzugte erfindungsgemäße Zubereitungen sind Gesichtsmasken, kosmetische Lotionen und Zubereitungen für die Verwendung in der dekorativen Kosmetik, beispielsweise für Abdeckstifte, Theaterfarben, Mascara und Lidschatten, Lippenstifte, Kajalstifte, Eyeliner, Makeups, Grundierungen, Rouges, Puder und Augenbrauenstifte.

Weitere erfindungsgemäße Zubereitungen sind Antiaknemittel, Repellentien, Rasiermittel, Haarentfernungsmittel, Intimpflegemittel, Fußpflegemittel sowie Babypflegeprodukte.

Weitere bevorzugte erfindungsgemäße Zubereitungen sind Wasch-, Dusch- und Badepräparate. Wasch-, Dusch- und Badepräparaten sind im Rahmen dieser Erfindung Seifen von flüssiger bis gelförmiger Konsistenz, Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Geeignete weitere Inhaltsstoffe für die vorgenannten erfindungsgemäßen Zubereitungen sind nachfolgend beschrieben.

Bevorzugte erfindungsgemäße Zubereitungen enthalten neben den Polyurethanen PU und dem wie vorstehend definierten Träger einen oder mehrere weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, weitere Konsistenzgeber, weitere Verdicker, Silikone, Feuchthaltemittel (z.B. 1,2-Pentandiol), Rückfetter, UV-Lichtschutzfilter, filmbildende Polymere, konditionierende Polymere, Antioxidantien, Entschäumer, Antistatika, Emollienzien, Weichmacher, Peroxide und weitere übliche Additive.

Antioxidantien

Ein Gehalt der erfindungsgemäßen Zubereitungen an Antioxidantien ist im allgemeinen bevorzugt. Erfindungsgemäß können als Antioxidantien alle für kosmetische Anwendungen geeigneten oder gebräuchlichen Antioxidantien verwendet werden. Geeignete Antioxidantien für die erfindungsgemäßen Zubereitungen sind beispielsweise auf Seite 41, Zeile 12 bis Seite 42 Zeile 33 der WO 2006/106140 beschrieben. Auf den Inhalt der genannten Textstelle wird hiermit in vollem Umfang Bezug genommen.

Öle, Fette und Wachse

Die erfindungsgemäßen Zubereitungen weisen in einer bevorzugten Ausführungsform neben den Polyurethanen PU und dem kosmetisch akzeptablen Träger eine Öl-, Fett- und/oder Wachs-Komponente auf, die ausgewählt ist unter: Kohlenwasserstoffen geringer Polarität wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten sowie Mischungen davon.

Die Öl- oder Wachskomponente kann auch ausgewählt sein aus Siliconölen und deren Derivaten wie z. B. linearen Polydimethylsiloxanen, Poly(methylphenyl)siloxanen, cyclischen Siloxanen und Mischungen davon. Das zahlenmittlere Molekulargewicht der Polydimethylsiloxane und Poly(methylphenyl)siloxane liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinus-öl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten sind auch in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Vorteilhafterweise werden diejenigen Öle, Fette und/oder Wachse gewählt, die auf Seite 28, Zeile 39 bis Seite 34, Zeile 22 der WO 2006/106140 beschrieben sind. Auf den Inhalt der genannten Textstelle wird hiermit in vollem Umfang Bezug genommen.

Der Gehalt an Ölen, Fetten und Wachsen beträgt höchstens 80, bevorzugt 50, weiter bevorzugt höchstens 30 Gewichts-%, bezogen auf das Gesamtgewicht der erfindungsgemäßen Zubereitung.

Tenside

Bevorzugte kosmetische Zubereitungen enthalten neben den Polyurethanen PU und dem wenigstens einem kosmetisch akzeptablen Träger, vorzugsweise Wasser, weiterhin wenigstens ein Tensid. Als Tenside können anionische, kationische, nichtionische und/oder amphotere Tenside eingesetzt werden.

Vorteilhafte waschaktive anionische Tenside im Sinne der vorliegenden Erfindung sind - Acylaminosäuren und deren Salze, wie Acylglutamate, insbesondere Natriumacylglutamat

- Sarcosinate, beispielsweise Myristoyl Sarcosin, TEA-Lauroyl Sarcosinat, Natriumlauroylsarcosinat und Natriumcocoylsarcosinat, Sulfonsäuren und deren Salze, wie
- Acylisethionate, beispielsweise Natrium- oder Ammoniumcocoylisethionat
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
- Alkylethersulfate, beispielsweise Natrium-, Ammonium-, Magnesium-, MIPA-, TIPA-Laurethsulfat, Natriummyrethsulfat und Natrium $C_{12-13}$ Parethsulfat,
- Alkyethersulfonate, beispielsweise Natrium C12-15 Pareth-15 Sulfonat
- Alkylsulfate, beispielsweise Natrium-, Ammonium- und TEA-Laurylsulfat.

Weitere vorteilhafte anionische Tenside sind

- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat

- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und Natrium-/Kalium Cocoyl hydrolysiertes Kollagen sowie Carbonsäuren und Derivate, wie beispielsweise Laurinsäure, Aluminiumstearat, Magnesiumalkanolat und Zinkundecylenat, Ester-Carbonsäuren, beispielsweise Calciumstearoyllactylat, Laureth-6 Citrat und Natrium PEG-4 Lauramidcarboxylat
- Alkylarylsulfonate.

Eine bevorzugte Ausführungsform der Erfindung sind kosmetische Zubereitungen, die wenigstens ein Polyurethan PU wie vorstehend definiert und wenigstens ein hydrophob modifiziertes Phosphat und/oder wenigstens ein hydrophob modifiziertes Sulfat enthalten.
Beispielhaft zu nennen als hydrophob modifizierte Phosphate sind Luviquat®Mono CP (INCI: Hydroxyethyl Cetyldimonium Phosphate), Luviquat®Mono LS (INCI: Cocotrimonium Methosulfate), Amphisol®Typen (INCI: Cetyl Phosphate, Potassium Cetyl Phosphate) und als als hydrophob modifiziertes Sulfat Lanette®E (INCI: Sodium Cetearyl Sulfate). Derartige hydrophob modifizierte Phosphate und/oder Sulfate führen in Kombination mit den Polyurethanen PU zu einer weiteren Erhöhung der Viskosität. Diese Erhöhung der Viskosität bleibt auch bei Temperaturerhöhung der Zubereitung erhalten. Bevorzugt ist es, wenn das Gewichtsverhältnis von Polyurethan PU zu hydrophob modifiziertem Phosphat und/oder hydrophob modifiziertem Sulfat im Bereich von 0,5:1 bis 2:1, bevorzugt im Bereich von 0,8:1 bis 1,2:1 liegt.
Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner

- Alkylamine,
- Alkylimidazole und
- ethoxylierte Amine

und insbesondere deren Salze.
Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.
Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.
Vorteilhafte waschaktive nicht-ionische Tenside im Sinne der vorliegenden Erfindung sind

- Alkanolamide, wie Cocamide MEA/DEA/MIPA,
- Ester, die durch Veresterung von Carbonsäuren mit Ethylenoxid, Glycerin, Sorbitan oder anderen Alkoholen entstehen,
- Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil®SPG 128V (Wacker)).

Weitere vorteilhafte nicht-ionische Tenside sind Alkohole und Aminoxide, wie Cocoamidopropylamin-Oxid.
Bevorzugte anionische, amphotere und nichtionische Tenside sind beispielsweise in "Kosmetik und Hygiene von Kopf bis Fuß", Hrsg. W. Umbach, 3. Auflage, Wiley-VCH, 2004, S.131-134 genannt, worauf an dieser Stelle in vollem Umfang Bezug genommen wird.
Unter den Alkylethersulfaten sind insbesondere Natriumalkylethersulfate auf Basis von zwei- oder dreifach ethoxyliertem Lauryl- und Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut- und Schleimhautverträglichkeit. Sie können auch als alleinige Waschrohstoffe für Shampoos eingesetzt werden können. Laurylethersulfat weist bessere Schaumeigenschaften als Myristylethersulfat auf, ist diesem aber in der Milde unterlegen. Alkylethercarboxylate mit mittlerem und besonders mit höherem gehören zu den mildesten Tensiden überhaupt, zeigen aber ein schlechtes Schaum- und Viskositätsverhalten. Sie werden oft in Kombination mit Alkylethersulfaten und amphoteren Tensiden in Haarwaschmitteln eingesetzt.

Sulfobernsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt. Amidopropylbetaine sind als alleinige Waschrohstoffe praktisch unbedeutend, da ihr Schaumverhalten sowie ihre Verdickbarkeit nur mäßig ausgeprägt sind. Demgegenüber weisen diese Tenside eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain. Amphoacetate /Amphodiacetate besitzen als amphotere Tenside eine sehr gute Haut- und Schleimhautverträglichkeit und können haarkonditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat. Alkylpolyglykoside sind nichtionische Waschrohstoffe. Sie sind mild, haben gute Universaleigenschaften, schäumen jedoch schwach. Aus diesem Grund werden sie bevorzugt in Kombinationen mit anionischen Tensiden verwendet.

Sorbitanester gehören ebenfalls zu den nichtionischen Waschrohstoffen. Wegen ihrer ausgezeichneten Milde werden sie bevorzugt zum Einsatz bei Baby-Shampoos verwendet. Als Schwachschäumer werden sie bevorzugt in Kombination mit anionischen Tensiden eingesetzt.

Es ist erfindungsgemäß von Vorteil, wenn eines oder mehrere dieser Tenside in einer Konzentration von 0,1 bis 30 Gewichts-%, bevorzugt in einer Konzentration von 1 bis 25 Gewichts-%, weiter bevorzugt in einer Konzentration 5 von 25 bis Gewichts-% und ganz besonders bevorzugt in einer Konzentration von 10 bis 20 Gewichts-%, jeweils bezogen auf das Gesamtgewicht der Zubereitung eingesetzt werden.

Lufteinschluß

Ein weiterer Vorteil der kosmetischen Zubereitungen enthaltend die Polyurethane PU ist, dass bis zu 150 Volumen-%, bezogen auf das Volumen der Zubereitung vor Lufteintrag, Luft eingetragen werden können. Ein weiterer Gegenstand der Erfindung ist also eine erfindungsgemäße kosmetische Zubereitung, dadurch erhältlich, dass in die im Übrigen bereits alle sonstigen Bestandteile enthaltende Zubereitung im Bereich von 5 bis 150 Vol.-%, bezogen auf das Volumen der Zubereitung vor der Einbringung der Luft, Luft eingebracht wird. Derartige, von 5 bis 150 Vol.-%, bevorzugt von 20 bis 100 Vol.-% Luft enthaltende Zubereitungen sind weitere Gegenstände der vorliegenden Erfindung. Die von 5 bis 150 Vol.-%, bezogen auf das Volumen der Zubereitung ohne Lufteintrag, Luft enthaltenden erfindungsgemäßen Zubereitungen sind über mehrere Monate volumenstabil. Die Einbringung von Luft führt zu einer Verbesserung von Struktur (enge Größenverteilung der Luftblasen), Sensorik und visuellem Eindruck. Beispiele solcher vorteilhafter Ausführungsformen der Erfindung sind Mousse-Zubereitungen.

Polysorbate

Auch Polysorbate können vorteilhaft in die erfindungsgemäßen Zubereitungen eingearbeitet werden. Im Sinne der Erfindung vorteilhafte Polysorbate sind

- Polyoxyethylen(20)sorbitanmonolaurat (Tween®20, CAS-Nr. 9005-64-5)
- Polyoxyethylen(4)sorbitanmonolaurat (Tween®21, CAS-Nr. 9005-64-5)
- Polyoxyethylen(4)sorbitanmonostearat (Tween®61, CAS-Nr. 9005-67-8)
- Polyoxyethylen(20)sorbitantristearat (Tween®65, CAS-Nr. 9005-71 -4)
- Polyoxyethylen(20)sorbitanmonooleat (Tween®80, CAS-Nr. 9005-65-6)
- Polyoxyethylen(5)sorbitanmonooleat (Tween®81, CAS-Nr. 9005-65-5)
- Polyoxyethylen(20)sorbitantrioleat (Tween®85, CAS-Nr. 9005-70-3).

Besonders vorteilhaft sind

- Polyoxyethylen(20)sorbitanmonopalmitat (Tween®40, CAS-Nr. 9005-66-7) und
- Polyoxyethylen(20)sorbitanmonostearat (Tween®60, CAS-Nr. 9005-67-8).

Die Polysorbate werden vorteilhaft in einer Konzentration von 0,1 bis 5 und insbesondere in einer Konzentration von 1,5 bis 2,5 Gew.-%, bezogen auf das Gesamtgewicht der Zubereitung einzeln oder als Mischung mehrerer Polysorbate, eingesetzt.

Konditionierungsmittel

Bevorzugte kosmetische Zubereitungen enthalten neben den Polyurethanen PU und wenigstens einem kosmetisch akzeptablen Träger, vorzugsweise Wasser, weiterhin wenigstens ein Konditionierungsmittel. Als Konditionierungsmittel für die erfindungsgemäßen kosmetischen Zubereitungen werden bevorzugt diejenigen Konditionierungsmittel gewählt, die auf Seite 34, Zeile 24 bis Seite 37, Zeile 10 der WO 2006/106140 beschrieben sind. Auf den Inhalt der genannten Textstelle wird hiermit in vollem Umfang Bezug genommen. Weitere bevorzugte Konditioniermittel sind hydrierte Polyisobutene, insbesondere das als Luvitol®Lite kommerziell erhältliche.

Rheologiemodifizierungsmittel

Im allgemeinen lässt sich die Rheologie der erfindungsgemäßen Zubereitungen durch die Polyurethane PU auf den jeweils gewünschten Wert einstellen. Die Verwendung weiterer Rheologiemodifizierungsmittel ist nicht zwingend notwendig. Selbstverständlich können in den erfindungsgemäßen Zubereitungen aber zusätzlich weitere Verdickungsmittel verwendet werden. Für Gele, Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird. Geeignete weitere Verdickungsmittel für die erfindungsgemäßen kosmetischen Zubereitungen sind beispielsweise auch auf Seite 37, Zeile 12 bis Seite 38, Zeile 8 der WO 2006/106140 beschrieben. Auch auf den Inhalt dieser Textstelle wird hiermit in vollem Umfang Bezug genommen. In einer Ausführungsform der Erfindung enthalten die erfindungsgemäßen Zubereitungen außer den Polyurethanen PU keine weiteren Rheologiemodifizierungsmittel. Ein weiterer Gegenstand der vorliegenden Erfindung sind kosmetische Zubereitungen, die außer den Polyurethanen PU weitere Rheologiemodifizierungsmittel enthalten. Eine Ausführung der Erfindung sind kosmetische Zubereitungen, die Polyurethane PU und Polyacrylat-Verdicker, insbesondere hydrophob modifizierte Polyacrylat-Verdicker enthalten. Eine bevorzugte Ausführungsform der Erfindung sind kosmetische Zubereitungen, enthaltend wenigstens ein Polyurethan PU und wenigstens einen Polyacrylat-Verdicker mit der INCI-Bezeichnung Acrylates/C10-30 Alkyl Acrylate Crosspolymer (z.B. Carbopol® Ultrez 21).
Kosmetische Zubereitungen, die wenigstens ein Polyurethan PU und wenigstens ein Acrylates/C10-30 Alkyl Acrylate Crosspolymer enthalten, sind beispielsweise Gele, die bei gleicher Viskosität eine verbesserte Sensorik aufweisen.
Die Kombination von Polyurethanen PU mit hydrophob modifizierten Polyacrylaten führt zu synergistischen Effekten hinsichtlich der Verdickung. Kombinationen von Polyurethanen PU mit Polyacrylaten, insbesondere hydrophob modifizierten Polyacrylaten, führen zu gleicher oder stärkerer Verdickungswirkung im Vergleich zur alleinigen Verwendung von Polyacrylatverdickern bei gleichzeitig verringerter Einsatzmenge. Die entstehenden Formulierungen weisen eine leichte Sensorik und einer hervorragende Verteilbarkeit auf beispielsweise der Haut auf.
Ein weiterer Gegenstand der Erfindung ist ein Verfahren zur Verbesserung der Verteilbarkeit von kosmetischen Zubereitungen, insbesondere in Form von Emulsionen, auf der Haut, dadurch gekennzeichnet, dass man der kosmetischen Zubereitung wenigstens ein Polyurethan PU hinzufügt, so dass der Mengenanteil Polyurethan PU, bezogen auf das Gesamtgewicht der Zubereitung, im Bereich von 0,1 bis 10 Gew.-% beträgt.

Konservierungsmittel

Insbesondere Zubereitungen mit hohen Wassergehalten müssen zuverlässig vor Verkeimung geschützt sein. Die erfindungsgemäßen kosmetischen Zubereitungen enthalten in einer bevorzugten Ausführungsform Konservierungsmittel.
Geeignete Konservierungsmittel für die erfindungsgemäßen kosmetischen Zusammensetzungen sind beispielsweise auf Seite 38, Zeile 10 bis Seite 39, Zeile 18 der WO 2006/106140 beschrieben. Auf den Inhalt der genannten Textstelle wird hiermit in vollem Umfang Bezug genommen.

Komplexbildner

Da die Rohstoffe und auch viele kosmetische Mittel selbst überwiegend in Stahlapparaturen hergestellt werden, können die Endprodukte Eisen(-Ionen) in Spurenmengen enthalten. Um zu verhindern, dass diese Verunreinigungen über Reaktionen mit Farbstoffen und Parfümölbestandteilen die Produktqualität nachteilig beeinflussen, werden den kosmetischen Zubereitungen in einer bevorzugten Ausführungsform Komplexbildner wie Salze der Ethylendiamintetraessigsäure, der Nitrilotriessigsäure, der Iminodibernsteinsäure oder Phosphate zugesetzt.

UV- Lichtschutzfilter

Um die in den erfindungsgemäßen kosmetischen Zubereitungen enthaltenen, UV-Licht-empfindlichen Inhalts-

stoffe wie beispielsweise Farbstoffe und Parfümöle gegen Veränderungen durch UV-Licht zu stabilisieren, können UV-Lichtschutzfilter, wie z.B. Benzophenon-Derivate, eingearbeitet werden. Geeignete UV- Lichtschutzfilter für die erfindungsgemäßen kosmetischen Zusammensetzungen sind beispielsweise auf Seite 39, Zeile 20 bis Seite 41 Zeile 10 der WO 2006/106140 beschrieben. Auf den Inhalt der genannten Textstelle wird hiermit in vollem Umfang Bezug genommen. Weitere geeignete UV- Lichtschutzfilter sind untenstehend in Zusammenhang mit kosmetischen UV-Lichtschutzzubereitungen genannt.

Puffer

In einer bevorzugten Ausführungsform der Erfindung enthalten die kosmetischen Zubereitungen Puffer. Puffer gewährleisten die pH-Wert-Stabilität der kosmetischen Zubereitungen. Überwiegend verwendet werden Citrat-, Lactat- und Phosphat-Puffer.

Lösungsvermittler

In einer bevorzugten Ausführungsform der Erfindung enthalten die kosmetischen Zubereitungen Lösungsvermittler. Lösungsvermittler werden eingesetzt, um pflegende Öle oder Parfümöle klar in Lösung zu bringen und auch in der Kälte klar in Lösung zu halten. Die gängigsten Lösungsvermittler sind ethoxylierte nichtionische Tenside, z. B. hydrierte und ethoxylierte Ricinusöle. Die erfindungsgemäß verwendeten Polyurethane PU können selbst als Lösungsvermittler wirken.

Keimhemmende Mittel

In einer bevorzugten Ausführungsform der Erfindung enthalten die kosmetischen Zubereitungen keimhemmende Mittel. Zu den keimhemmende Mitteln gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet und werden bevorzugt für desinfizierende Seifen und Waschlotionen verwendet. Auch zahlreiche Riechstoffe haben antimikrobielle Eigenschaften. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit. Die antibakteriell wirksamen Stoffe werden den Zubereitungen in der Regel in Konzentrationen von ca. 0,1 bis 0,3 Gew.-%, bezogen auf die Zubereitung, eingesetzt.

Dispergiermittel

Wenn in den erfindungsgemäßen Zubereitungen schwer- oder unlösliche Wirkstoffe, z.B. Antischuppenwirkstoffe oder Siliconöle, dispergiert und auf Dauer in Schwebe gehalten werden sollen, müssen Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt werden.

Erfindungsgemäß sind Dispergiermittel in einer Gesamtkonzentration von höchstens 2, bevorzugt höchstens 1,5 und besonders bevorzugt höchstens 1 Gewichts-%, bezogen auf das Gesamtgewicht der Zubereitung, enthalten.

Die erfindungsgemäßen Zubereitungen wie Gele, Shampoos und Haarpflegemittel enthalten in einer bevorzugten Ausführungsform ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glycerylolead-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.

Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.

Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

Wirkstoffe

Die erfindungsgemäßen Zubereitungen enthalten in einer bevorzugten Ausführungsform kosmetisch und/oder dermatologisch aktive Wirkstoffe. Vorteilhafte Wirkstoffe für die erfindungsgemäßen kosmetischen Zubereitungen sind beispielsweise auf Seite 44, Zeile 24 bis Seite 49 Zeile 39 der WO 2006/106140 beschrieben. Auf den Inhalt der genannten Textstelle wird hiermit in vollem Umfang Bezug genommen.

Eine weitere Ausführungsform der Erfindung sind kosmetische Zubereitungen, die wenigstens ein Polyurethan PU und von 0,1 bis 20 Gew.-%, bevorzugt von 0,5 bis 15 Gew.-%, weiter bevorzugt von 5 bis 12 Gew.-% Harnstoff enthalten. Selbst große Mengen von Harnstoff können bei gleichzeitiger Anwesenheit von Polyurethan PU stabil und unter Einstellung einer geforderten Viskosität in die kosmetischen Zubereitungen eingearbeitet werden.

Perlglanzwachse

Die erfindungsgemäßen Zubereitungen enthalten in einer bevorzugten Ausführungsform Perlglanzwachse. Geeignete Perlglanzwachse für die erfindungsgemäßen kosmetischen Zubereitungen sind beispielsweise auf Seite 50, Zeile 1 bis Zeile 16 der WO 2006/106140 beschrieben. Auf den Inhalt der genannten Textstelle wird hiermit in vollem Umfang Bezug genommen.

Die erfindungsgemäßen Zubereitungen können weiterhin Glitterstoffe und/oder andere Effektstoffe (z.B. Farbschlieren) enthalten.

Emulgatoren

Die erfindungsgemäßen kosmetischen Zubereitungen liegen in einer bevorzugten Ausführungsform der Erfindung in Form von Emulsionen, bevorzugt O/W-Emulsionen, vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben. Auf den Inhalt der genannten Textstelle wird hiermit in vollem Umfang Bezug genommen.

Parfümöle

Die erfindungsgemäßen Zubereitungen enthalten in einer bevorzugten Ausführungsform Parfümöle. Geeignete Parfümöle sind beispielsweise auf Seite 53, Zeile 10 bis Seite 54, Zeile 3 der WO 2006/106140 beschrieben. Auf den Inhalt der genannten Textstelle wird hiermit in vollem Umfang Bezug genommen.

Pigmente

Die erfindungsgemäßen Zubereitungen enthalten in einer bevorzugten Ausführungsform weiterhin Pigmente. Die Pigmente liegen im Produkt meist in ungelöster Form vor und können in einer Menge von 0,05 bis 90 Gew.%, besonders bevorzugt von 1 bis 15 Gew.% enthalten sein. In einer Ausführungsform der Erfindung, insbesondere bei Zubereitungen in Form von dekorativen Kosmetika wie beispielweise Lidschatten kann die Pigmentmenge bis zu 90 Gew.-% der Zubereitung betragen.

Die bevorzugte Teilchengröße beträgt 0,01 bis 200 $\mu$m, insbesondere 0,02 bis 150 $\mu$m, besonders bevorzugt 0,05 bis 100 $\mu$m.

Geeignete Pigmente für die erfindungsgemäßen Zusammensetzungen sind beispielsweise auf Seite 54, Zeile 5 bis Seite 55, Zeile 19 der WO 2006/106140 beschrieben. Auf den Inhalt der genannten Textstelle wird hiermit in vollem Umfang Bezug genommen.

Nanopartikel

Die erfindungsgemäßen Zubereitungen enthalten in einer bevorzugten Ausführungsform weiterhin Pigmente in Form wasserunlöslicher Nanopartikel, also Teilchen mit einer Teilchengröße im Bereich von 1 bis 200, bevorzugt von 5 bis 100 nm. Bevorzugte Nanopartikel sind Nanopartikel von Metalloxiden, insbesondere von Zinkoxid und/oder Titandioxid und/oder Siliziumdioxid.

Polymere

Die erfindungsgemäßen kosmetischen Zubereitungen enthalten in einer bevorzugten Ausführungsform außer den Polyurethanen PU weitere Polymere. Bevorzugte weitere Polymere sind wasserlösliche bzw. wasserdispergierbare Polymere, wobei wasserlösliche Polymere besonders bevorzugt sind.

Für die erfindungsgemäßen Zubereitungen geeignete weitere Polymere sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

Kosmetische und/oder dermatologische Lichtschutzmittel

Unter kosmetischen bzw. dermatologischen Lichtschutzmittel werden im Rahmen dieser Erfindung kosmetische bzw. dermatologische Zubereitungen verstanden, die wenigstens eine, bevorzugt mehrere UV-Filtersubstanzen enthalten.

Der Begriff UV-Filtersubstanz ist dem Fachmann bekannt und bezeichnet Stoffe oder Zubereitungen, die kosmetischen oder dermatologischen Mitteln zu dem Zweck hinzugefügt werden, UV-Strahlen zu filtern, um die Haut vor bestimmten schädlichen Einwirkungen dieser Strahlung zu schützen. Im engeren Sinne werden damit auch Verbindungen bezeichnet, die Licht mit Wellenlängen im UV-Bereich absorbieren. Je nach Absorptionsspektrum wird zwischen UV-A-, UV-B- und UV-C-Filtern unterschieden. In der Regel werden UV-Filter zur Erfüllung von spektralen und formulierungstechnischen Anforderungen als Kombinationen eingesetzt. Auch Pigmente und Mikropigmente, bevorzugt von Titandioxid und/oder Zinkoxid (siehe oben), die überwiegend zu vorgenanntem Zweck eingesetzt werden, sind als UV-Filter zu bezeichnen.

Erfindungsgemäße kosmetische Zubereitungen können selbstverständlich auch gleichzeitig für kosmetische und dermatologische Zwecke geeignet sein. Der Ausdruck "kosmetische oder dermatologische Lichtschutzmittel" umfasst demnach auch solche Zusammensetzungen, die gleichzeitig sowohl kosmetische als auch dermatologische Zwecke erfüllen.

Eine bevorzugte Ausführungsform der vorliegenden Erfindung betrifft Lichtschutzzusammensetzungen mit einem LSF von wenigstens 4, darunter insbesondere solche kosmetischen oder dermatologischen Lichtschutzmittel, die wenigstens eine UV-Filtersubstanz enthalten.

Die kosmetischen und/oder dermatologischen Lichtschutzzubereitungen dieser Erfindung dienen dem kosmetischen und/oder dermatologischen Lichtschutz, ferner der Behandlung und Pflege der Haut und/oder der Haare und als Schminkprodukt in der dekorativen Kosmetik.

Zu den kosmetischen und/oder dermatologischen Lichtschutzzubereitungen der vorliegenden Erfindung zählen beispielsweise Sonnencremes, -lotionen, -milche, -öle, - balsame, -gele, Lippenpflegen und Lippenstifte, Abdeckcremes und -stifte, Feuchtigkeitscremes, -lotionen, -emulsionen, Gesichts-, Körper- und Handcre-mes, Haarkuren und -spülungen, Haarfestiger, Styling-Gele, Haarsprays, Deoroller oder Augenfältchencremes, Tropicals, Sunblocker, Aftersun-Präparate. Alle diese Lichtschutzzubereitungen enthalten wenigstens eine UV-Filtersubstanz und haben bevorzugt einen LSF von wenigstens 4.

Als UV-Lichtschutzfilter dienen sowohl anorganische Pigmente, beispielsweise Titandioxid oder Zinkoxid, als auch organische Verbindungen, meist aromatische Substanzen mit ausgeprägtem pi-Elektronensystem.

Sonnenschutzmittel gibt es in verschiedenen Ausführungsformen. Besonders beliebt sind wasserfeste Produkte, bei denen die Filtersubstanzen bei Kontakt der Haut und/oder der Haare mit Wasser nicht sofort abgewaschen werden. Dieses Abwaschen wird durch die Gegenwart weiterer Substanzen in den Lichtschutzmitteln vermindert oder unterdrückt. Die vorliegende Erfindung betrifft in einer Ausführungsform kosmetische Lichtschutzzubereitungen ausgewählt aus der Gruppe der kosmetischen und dermatologischen Lichtschutzmittel enthaltend die zuvor beschriebenen Polyurethane PU. Unter kosmetischen oder dermatologischen Lichtschutzmitteln werden im Rahmen dieser Erfindung kosmetische oder dermatologische Zubereitungen verstanden, die neben den Polyurethanen PU wenigstens eine, bevorzugt mehrere UV-Filtersubstanz enthalten. Die kosmetischen oder dermatologischen Lichtschutzmittel dieser Erfindung weisen bevorzugt einen Lichtschutzfaktor (LSF) von wenigstens 4 auf (bestimmt nach der COLIPA-Methode, siehe unten).

Sonnenmilche und -Cremes werden bevorzugt als Öl-in-wasser- (O/W) Emulsionen und als Wasser-in-Öl-(W/O-)Emulsionen hergestellt. Je nach Emulsionstyp sind die Eigenschaften der Präparate sehr unterschiedlich: O/W-Emulsionen sind auf der Haut leicht verteilbar, sie ziehen meist schnell in die Haut ein und sind fast immer leicht mit Wasser abwaschbar. W/O-Emulsionen sind schwerer einzureiben, sie fetten die Haut stärker und wirken dadurch etwas klebriger, bewahren aber andererseits die Haut besser vor dem Austrocknen. W/O-Emulsionen sind meist wasserfest. Bei O/W-Emulsionen entscheiden die Emulsionsbasis, die Auswahl geeigneter Lichtschutzstoffe und ggf. der Einsatz von Hilfsstoffen (z. B. Polymere) über den Grad der Wasserfestigkeit. Die Grundlagen von flüssigen und cremeförmigen O/W-Ernulsionen ähneln in ihrer Zusammensetzung den sonstigen in der Hautpflege üblichen Emulsionen. Sonnenmilche sollen die durch Sonne, Wasser und Wind ausgetrocknete Haut ausreichend fetten. Sie dürfen nicht klebrig sein, da dies in der Hitze und bei Kontakt mit Sand als besonders unangenehm empfunden wird.

Die Lichtschutzmittel sind in der Regel auf der Basis eines Trägers, der mindestens eine Ölphase enthält. Es sind aber auch Zubereitungen allein auf wässriger Basis möglich. Demgemäss kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden oberflächenbeschichteten Titandioxidpartikeln in Frage, wobei die Emulsionen beispielsweise durch Phaseninversionstechnologie, gemäß DE-A-197 26 121 erhältlich sind (PIT-Emulsionen).

Übliche kosmetische Hilfsstoffe, die als Zusätze in Betracht kommen können, sind z.B. (Co-)Emulgatoren, Fette und Wachse, Stabilisatoren, weitere Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren.

Als Stabilisatoren können Metallsalze von Fettsäuren wie z.B. Magnesium-, Aluminium- und/oder Zinkstearat eingesetzt werden.

Unter biogenen Wirkstoffen sind beispielsweise Pflanzenextrakte, Eiweißhydrolysate und Vitaminkomplexe zu verstehen.

Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre CelluloseDerivate und ähnliche Verbindungen.

Lichtschutzfaktor (LSF) und COLIPA-Methode

Lichtschutzfaktor (LSF)

Die Bestimmung des Lichtschutzfaktors (LSF) - auch SPF (Sun Protection Factor) abgekürzt - erfolgt in Europa nach der COLIPA-Norm der European Cosmetic, Toiletry and Perfumery Association. Er bezeichnet die Schutzleistung von Sonnenschutzprodukten gegenüber UVB-Strahlen. Diese sind Hauptverursacher des Sonnenbrandes. Daneben besitzen sie immunsuppressive und zellschädigende Wirkungen, die bei chronischer Belastung zur Hautkrebsentstehung (Basaliom, Spinaliom) führen. Es werden verschiedene UV-Filter beziehungsweise UV-Filtersysteme/- kombinationen verwendet, um eine optimale Schutzleistung zu erreichen. Bestimmt man die minimale Erythem-Dosis (MED), das heißt die Menge an UVB-Bestrahlung, die eine gerade erkennbare Rötung (Erythem) induziert, ergibt sich der LSF nach folgender Formel:

$$LSF = \frac{\text{MED auf geschützter Haut}}{\text{MED auf ungeschützter Haut}} = \frac{\text{MED}_g}{\text{MED}_u}$$

Die Testdurchführung beginnt mit der Bestimmung der individuellen UV-Empfindlichkeit der Probanden durch die Bestrahlung ungeschützter Hautareale auf dem Rücken. Bestrahlungsquellen sind Solarsimulatoren, meist mit Xenonlampen ausgestattet: Sie liefern eine in ihrem Spektrum sonnenähnliche Strahlung, die jedoch von höherer Intensität ist und dadurch kürzere Bestrahlungszeiten ermöglicht. Zirka 20 Stunden nach der Bestrahlung mit unterschiedlicher Intensität wird die $MED_u$ durch visuelle Bewertung der Erytheme in sechs Hautarealen bestimmt.

Zur eigentlichen LSF-Bestimmung werden erneut Testareale auf dem Probandenrücken für die aufzutragenden Sonnenschutzpräparate markiert. Jedem dieser Produktareale wird ein benachbartes Kontrollareal mit unbehandelter Haut gegenübergestellt. Die Bestrahlungsintensität wird je nach Hautempfindlichkeit und erwartetem Lichtschutzfaktor festgelegt. Nach zirka 20 Stunden werden $MED_u$ und $MED_g$ visuell ermittelt. Die resultierenden Lichtschutzfaktoren geben die mittlere Verlängerung der individuellen Zeitspanne bis zum Eintreten eines Erythems an, die durch die Anwendung des Lichtschutzpräparates erreicht wurde.

Die COLIPA-Methode ist die dem Fachmann bekannte, seit 1997 europaweit gültige Methode zur Bestimmung des Lichtschutzfaktors (UVB-Schutz) von Sonnenschutzprodukten. Das Testverfahren ist standardisiert und genormt: Das Bestrahlungsspektrum und die Ausgangsleistung des für die Prüfung vorgesehenen Sonnensimulators sind exakt definiert. Zusätzlich sind die Auftragsmenge und die Art der Produktauftragung genau vorgegeben. Die Testmethode ist unabhängig vom Hauttyp und vom Alter der Testpersonen. Mit diesen präzisen Vorgaben der COLIPA-Methode kann nach entsprechender Statistiklage mit nur zehn Probanden die Prüfung von Sonnenschutzprodukten durchgeführt werden. Die COLIPA-Methode ist ein Verfahren, das reproduzierbare Ergebnisse mit hoher Zuverlässigkeit liefert.

UV-Filtersubstanzen

In einer Ausführungsform der Erfindung enthalten die erfindungsgemäßen Zubereitungen neben den Polyurethanen PU öllösliche und/oder wasserlösliche UVA- und/oder UVB-Filter.

Vorteilhaft enthalten diese Lichtschutzzubereitungen Substanzen, die UV-Strahlung im UVB-Bereich und Substanzen, die UV-Strahlung im UVA-Bereich absorbieren, wobei die Gesamtmenge der Filtersubstanzen z.B. 0,1 bis 50 Gew.-%, vorzugsweise 0,5 bis 30 Gew.-%, insbesondere 1 bis 15 Gew.-% beträgt, bezogen auf das Gesamtgewicht der Zubereitungen, um kosmetische Zubereitungen zur Verfügung zu stellen, welche die Haut vor dem gesamten Bereich der ultravioletten Strahlung schützen.

Der größte Teil der Lichtschutzmittel in den zum Schutz der menschlichen Epidermis dienenden kosmetischen oder dermatologischen Zubereitungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Eine weitere Ausführungsform der Erfindung sind insbesondere Sonnenschutzformulierungen enthaltend die Polyurethane PU mit einer ausgewogenen UVA-Balance, wie unter EP 1291640 A1 oder entsprechend der Deutschen Industrienorm (DIN) 67502 (Qualitätsstandard beim Schutz vor lichtbedingter Hautalterung) beschrieben, hergestellt werden. Auf diese Textstellen wird hiermit in vollem Umfang Bezug genommen.

Die UVB-Filter können öllöslich, wasserlöslich oder pigmentär sein. Vorteilhafte UVB-Filtersubstanzen sind z.B.:

- Benzimidazolsulfonsäure-Derivate wie z.B. 2-Phenylbenzimidazol-5-sulfonsäure und deren Salze
- Benzotriazol-Derivate wie z.B. 2,2'-Methylen-bis-(6-(2H-benzotriazol-2-yl)-4-(1,1,3,3-tetramethylbutyl)-phenol) (Tinosorb® M)
- 4-Aminobenzoesäure-Derivate, vorzugsweise 4-(Dimethylamino)-benzoesäure(2-ethylhexyl)ester, 4-(Dimethy1amino)benzoesäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzi mtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methyl benzophenon, 2,2'-Dihydroxy-4-methoxybenzophenon;
- Methylidencampherderivate, vorzugsweise 4-Methylbenzylidencampher, Benzyl idencampher;
- Triazinderivate, vorzugsweise 4,4',4"-(1,3,5-Triazin-2,4,6-triylimino)-tris-benzoesäure-tris-(2-ethylhexylester) [INCI: Diethylhexyl Butamido Triazine, UVA-Sorb® HEB (Sigma 3V)] und 2,4,6-Tris-[anilino-(p-carbo-2'-ethyl-1'-hexyloxy)]-1,3,5-triazin [INCI: Octyl Triazone, UVINUL®T 150 (BASF)]

Vorteilhaft zu verwendende wasserlösliche UVB-Filtersubstanzen sind z.B.:

- Sulfonsäure-Derivate des 3-Benzylidencamphers, wie z.B. 4-(2-0xo-3-bornylidenmethyl)benzolsulfonsäure,2-Methyl-5-(2-oxo-3-bornylidenmethyl)sulfonsäure und deren Salze.

Vorteilhaft zu verwendende UVA-Filter sind z.B.:

- 1,4-Phenylendimethincamphersulfonsäurederivate wie z.B. 3,3'-(1,4-phenylendimethin)-bis-(7,7-dimethyl-2-oxobicyclo[2.2.1]-heptan-l - methamsulfonsäure und ihre Salze
- 1,3,5-Triazinderivate wie 2,4-Bis{[(2-ethylhexyloxy)-2-hydroxy)-phenyl}-6-(4-methoxyphenyl)-l ,3,5)-triazin (z.B. Tinosorb®S (Ciba))
- Dibenzoylmethanderivate, vorzugsweise 4-Isopropyldibenzoylmethan, 4-(tert.-Butyl)-4'-methoxydibenzoylmethan
- Benzoxazol-Derivate, beispielsweise das 2,4-Bis-[4-[5-(1,1-dimethylpropyl)benzoxazol-2-yl]phenylimino]-6-[(2-ethylexyl)imino]-1,3,5- triazin (CAS Nr. 288254-1 6-0, Uvasorb®K2A (3V Sigma))
- Hydroxybenzophenone, beispielsweise der 2-(4'-Diethylamino-2'-hydoxybenzoyl)-benzoesäurehexylester (auch: Aminobenzophenon) (Uvinul®A Plus (BASF))

Ferner kann erfindungsgemäß gegebenenfalls von Vorteil sein, Zubereitungen mit weiteren UVA- und/oder UVB-Filtern zu versehen, beispielsweise bestimmten Salicylsäurederivaten wie 4-Isopropylbenzylsalicylat, 2-Ethylhexylsalicylat, Octylsalicylat, Homomenthylsalicylat. Die Gesamtmenge an Salicylsäurederivaten in den erfindungsgemäßen kosmetischen oder dermatologischen Zubereitungen wird vorteilhaft aus dem Bereich von 0,1-15,0, bevorzugt 0,3-10,0 Gew.-% gewählt, bezogen auf das Gesamtgewicht der Zubereitungen. Ein weiterer erfindungsgemäß vorteilhaft zu verwendender Lichtschutzfilter ist Ethylhexyl-2-cyano-3,3-diphenylacrylat (Octocrylen, im Handel beispielsweise erhältlich als Uvinul®N 539 (BASF)).

Die folgende Tabelle stellt einige für die Verwendung in den erfindungsgemäßen Zubereitungen geeignete Lichtschutzfilter zusammen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |

(fortgesetzt)

| Nr. | Stoff | CAS-Nr. (=Säure) |
|---|---|---|
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Auch polymere oder polymergebundene Filtersubstanzen wie beispielsweise Parsol®SLX können erfindungsgemäß verwendet werden.

Zur Verwendung in den erfindungsgemäßen Zubereitungen geeignete Lichtschutzmittel sind auch die in der EP-A 1 084 696 in den Absätzen [0036] bis [0053] genannten Verbindungen, worauf an dieser Stelle vollumfänglich Bezug genommen wird. Für die erfindungsgemäße Verwendung geeignet sind alle UV-Lichtschutzfilter, die in Anlage 7 (zu § 3b) der deutschen Kosmetik-Verordnung unter "Ultraviolett-Filter für kosmetische Mittel" genannt sind.

Metalloxide wie Titandioxid oder Zinkoxid finden weite Verbreitung in Sonnenschutzmitteln. Ihre Wirkung beruht im wesentlichen auf Reflexion, Streuung und Absorption der schädigenden UV-Strahlung und hängt wesentlich von der Primärpartikelgröße der Metalloxide ab. Die erfindungsgemäßen kosmetischen oder dermatologischen Zubereitungen enthalten in einer besonders bevorzugten Ausführungsform der Erfindung anorganische Pigmente auf Basis von Metalloxiden und/oder anderen in Wasser schwerlöslichen oder unlöslichen Metallverbindungen, ausgewählt aus der Gruppe der Oxide des Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle, Abmischungen aus solchen Oxiden sowie Dotierungen der Oxide mit Fremdmetallanteilen bzw. das Coaten von Metalloxiden mit anderen Metalloxiden . Besonders bevorzugt handelt es sich um Pigmente auf der Basis ZnO und/oder $TiO_2$.

Besonders bevorzugte Ausführungsformen der Erfindung sind demnach kosmetische oder dermatologische Lichtschutzzubereitungen, die Polyurethane PU und Zinkoxid und/oder Titandioxid als anorganische UV-Lichtschutzfilter enthalten.

In einer weiteren Ausführungsform wird die Viskosität von Wasser enthaltenden erfindungsgemäßen Zubereitungen, die Polyurethan PU und mindestens ein Lichtschutzmittel, insbesondere Zinkoxid oder Titandioxid, enthalten, durch das Vorhandensein der Polyurethane PU in der Zubereitung im Vergleich zu Zubereitungen, die nur Lichtschutzmittel oder nur Polyurethane PU enthalten, gesteigert. Dabei ist die Reihenfolge, in der Polyurethane PU und Lichtschutzmittel zugegeben werden, unwesentlich.

Die anorganischen Pigmente können dabei in beschichteter Form vorliegen. Diese Beschichtung kann beispielsweise darin bestehen, dass die Pigmente in an sich bekannter Weise, wie in DE-A-33 14 742 beschrieben, mit einer dünnen hydrophoben Schicht versehen sind.

Besonders bevorzugte kosmetische Lichtschutzzubereitungen enthalten neben den Polyurethanen PU und einem kosmetisch akzeptablen Träger UV-Lichtschutzmittel auf Basis von Zinkoxid, die als Z-Cote®, Z-Cote®HP1 oder Z-Cote®MAX kommerziell erhältlich sind.

Besonders bevorzugte kosmetische Lichtschutzzubereitungen enthalten neben den Polyurethanen PU UV-Lichtschutzmittel auf Basis von Titandioxid, die als T-Lite®, T-Lite™SF, T-Lite™SF-S oder T-Lite™MAX kommerziell erhältlich sind. Die Aufzählung der genannten UV-Lichtschutz-Filter, die in den erfindungsgemäßen Zubereitungen verwendet werden können, ist nicht abschließend.

Eine Ausführungsform der Erfindung sind kosmetische Zubereitungen, die Polyurethan PU und wenigstens einen wasserlöslichen UV-Filter enthalten.

Eine weitere Ausführungsform der Erfindung sind kosmetische Zubereitungen, die Polyurethan PU und wenigstens einen ionogenen UV-Filter enthalten.

Beispielsweise kann 2-Phenylbenzimidazol-5-sulfonsäure (Eusolex®232) ideal bis zur zulässigen Höchstkonzentration von 8 Gew.-% stabil formuliert werden, was bisher nur unzureichend möglich war. Ferner können auch Kombinationen von verschiedenen UV-Filtern in Gegenwart von Polyurethan PU stabil formuliert werden, wobei ein gutes Hautgefühl im Vergleich zu anderen Verdickersystemen beobachtet wird.


Zubereitungen für die dekorative Kosmetik


Die Erfindung betrifft auch kosmetische Zubereitungen, vorzugsweise in flüssiger oder pastöser Form, zur Verwendung auf der Haut, auf Semischleimhäuten, auf Schleimhäuten und insbesondere auf keratinischen Materialien wie Haaren, Wimpern und Augenbrauen, insbesondere zum Formen, Dekorieren, Färben, Verschönen derselben sowie zum Pflegen der Haut und der Hautanhangsgebilde. Derartige Zubereitungen finden beispielsweise Verwendung für das Formen und Färben, insbesondere der Wimpern und der Haare - eine solche Zubereitung bezeichnet man dann als "Mascara"

Grundsätzlich können die erfindungsgemäßen Zubereitungen bei geeigneter Einstellung und Einfärbung auch als Make-up, Concealer, Camouflage, Lidschatten, Eyeliner, Lipliner, Rouge, Lippenrouge, Lipgloss, Sonnenschutzmittel, Sunblocker, temporäres Tattoo, farbiger Effekt-Sonnenschutz für Surfer und dergleichen verwendet werden.

Eine bevorzugte Ausführungsform der vorliegenden Erfindung sind also kosmetische Zubereitungen für die dekorative Kosmetik. Dies sind bevorzugt Zubereitungen, welche zum Schminken der Haut verwendet werden und mindestens einen dekorativen Bestandteil wie Farbstoffe, Farbpigmente, Perlglanzpigmente enthalten. Derartige Zubereitungen werden auch als Foundations oder Gesichts-Make-up bezeichnet. Im Bereich der kosmetischen Zubereitungen sind zur Färbung des Produktes oder zur Färbung des zu behandelnden Objektes (Haut, Haare, Lippen) sowohl lösliche Farbmittel (im Rahmen der vorliegenden Erfindung auch als Farbstoffe bezeichnet) und unlösliche Farbmittel (im Rahmen der vorliegenden Erfindung auch als (Farb-) Pigmente bezeichnet) zugelassen. Alle dekorativen Körperpflegemittel enthalten einen mehr oder weniger großen Anteil an Farbstoffen, Farbpigmenten und/oder Perlglanzpigmenten, da die farbliche Veränderung der Gesichtshaut, der Augenregion, der Lippen und/oder der Nägel der Hauptzweck dieser Produkte ist. Daneben enthalten diese Produkte üblicherweise zusätzlich weitere Inhaltsstoffe mit hautpflegender oder hautschützender Wirkung. Die Anwendung kosmetischer Make-up-Zubereitungen soll im allgemeinen die Besonderheit und Individualität einer Person unterstreichen und dabei der Betonung der persönlichen Attraktivität und der Kaschierung eventuell vorkommender Makel dienen. Der Schminkvorgang erfolgt üblicherweise in mehreren Schritten. Zunächst wird eine flüssige Grundierung (Foundation oder Gesichts-Make-up) aufgetragen, die die Hautfarbe ausgleicht und Unregelmäßigkeiten der Haut (wie z. B. Hautunreinheiten oder Augenringe) abdeckt. Sie soll der Haut einen natürlichen und strahlenden Teint sowie ein jugendliches Aussehen verleihen. Stärkere Unebenheiten oder Rötungen kann man mit einem hautfarbenen (Abdeck-) Stift oder Flüssig-Concealer extra kaschieren. Danach wird gewöhnlich loser oder fester Puder aufgetragen, um die Gesichthaut zu mattieren. Anschließend können die Wangen mit Rouge getönt und die Augen mit Lidschatten, Kajal, Lidstrich und/oder Mascara (Wimperntusche) geschminkt werden. Gesichts-Make-up-Zubereitungen können vorteilhaft auch Siliconöle bzw. Silconderivate enthalten, weil diese Komponenten dazu beitragen, dass sich die Formulierungen sehr gut und gleichmäßig auf der Haut verteilen lassen und ferner der Haut einen fettfreien, weichen Glanz verleihen und dabei ein samtigweiches Hautgefühl hinterlassen.

In einer Ausführungsform der Erfindung sind die kosmetischen Zubereitungen von Typ W/O-Emulsion. In einer bevorzugten Ausführungsform der Erfindung sind die kosmetischen Zubereitungen von Typ O/W-Emulsion.

Die erfindungsgemäßen Zubereitungen eignen sich insbesondere zum Abdecken von Hautunreinheiten und/oder Augenringen, zur Kaschierung kleiner Fältchen, zur Erreichung eines ebenmäßigen, dabei natürlichen und strahlenden Teints und eines jugendlichen Aussehens. Dabei ist der jeweils erzielte Effekt überraschend langanhaltend. Zur Anwendung werden die erfindungsgemäßen Zubereitungen in der für Kosmetika üblichen Weise auf die Haut in ausreichender Menge aufgebracht. Sie können wie üblich zusammengesetzt sein und zur Pflege der Haut und als Schminkprodukt in der dekorativen Kosmetik dienen.

Die in den erfindungsgemäßen Zubereitungen für die dekorative Kosmetik eingesetzten Pigmente können anorganisch oder organisch sein. Bevorzugte Pigmente sind in DE 10 2006 028 549 A1, Abschnitte [0018] bis [0026] beschrieben, worauf hiermit vollumfänglich Bezug genommen wird.

Es ist ferner vorteilhaft im Sinne der vorliegenden Erfindung, wenn die erfindungsgemäße Zubereitung einen oder mehrere Farbstoffe enthält. Die Farbstoffe können sowohl synthetischen als auch natürlichen Ursprungs sein. Die erfindungsgemäßen kosmetischen Zubereitungen können ferner vorteilhaft Füllstoffe enthalten, welche z. B. die sensorischen und kosmetischen Eigenschaften der Zubereitungen weiter verbessern und beispielsweise ein samtiges oder seidiges Hautgefühl hervorrufen oder verstärken.

Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filternoch färbende Wirkung haben (wie z. B. Bornitrid etc.) und/oder Aerosile®(CAS-Nr. 7631-86-9) sowie Lauroyl Lysine, Polymethylsilesquioxane, Polymethylmethacrylate, Polymethylmethacrylate Crosspolymer, Nylon, Talkum, beschichtetes Talkum, z.B. mit Dimethicone und Trimethylsiloxysilicate, Mica, Silica.

Die erfindungsgemäßen Zubereitungen können vorteilhaft ferner einen oder mehrere weitere (Silikon-)Emulgatoren enthalten, bevorzugt Emulgatoren mit einem HLB-Wert kleiner oder gleich 8, wenn es sich bei den Zubereitungen um Wasser-in-Silikonöl-Emulsionen (W/S-Emulsionen) handelt.

Bevorzugte Silikon-Emulgatoren sind in DE 10 2006 028 549 A1, Abschnitte [0032] bis [0037] beschrieben, worauf hiermit vollumfänglich Bezug genommen wird.

Die kosmetischen Zubereitungen gemäß der Erfindung können ferner vorteilhaft einen oder mehrere grenzflächenaktive Polyether enthalten, insbesondere wenn die Zubereitungen in Form von W/S-Emulsionen vorliegen. Bevorzugte grenzflächenaktive Polyether sind in DE 10 2006 028 549 A1, Abschnitt [0038] beschrieben, worauf hiermit vollumfänglich Bezug genommen wird. Die Gesamtmenge an den grenzflächenaktiven Polyethern in den fertigen kosmetischen Zubereitungen wird vorteilhaft aus dem Bereich von 0,1 bis 30 Gew.-%, bevorzugt 0,25 bis 5,0 Gew.-% insbesondere 0,75 bis 3,5 Gew.-% gewählt, jeweils bezogen auf das Gesamtgewicht der Zubereitungen. Die erfindungsgemäße Zubereitung kann auch in Form einer Wasser-in-Öl-Emulsion (W/O-Emulsion) vorliegen. In diesem Fall sind die Emulgatoren, die in DE 10 2006 028 549 A1, Abschnitt [0041] genannt sind, erfindungsgemäß bevorzugt. In einer besonders bevorzugten Ausführungsform weist die Ölphase einen Gehalt an cyclischen und/oder linearen Siliconölen auf bzw. werden cyclische und/oder lineare Siliconöle als alleinige Ölkomponenten verwendet.

Ferner kann die Ölphase einen Gehalt an Dialkylcarbonaten aufweisen, vorteilhaft ist z. B. Dicaprylylcarbonat, beispielsweise das unter der Handelsbezeichnung Cetiol®CC (Cognis) erhältliche. Ferner kann die Ölphase Triglyceride wie Capryliclcapric Triglyceride, Dialkylether wie Dicaprylylether, natürliche Öle wie Avocado-, Sesam-, Mandel-, Soja-, Aprikosenöl und Kohlenwasserstoffe, linear oder verzweigt enthalten. Die Ölphase der erfindungsgemäßen Zubereitungen kann ferner vorteilhaft auch Wachskomponenten enthalten, insbesondere Wachse, deren Schmelzpunkt zwischen 30 und 45°C, besonders bevorzugt zwischen 30 und 40°C, liegt. Bevorzugte Wachse sind in DE 10 2006 028 549 A1, Abschnitt [0049] beschrieben, worauf hiermit vollumfänglich Bezug genommen wird.

Die erfindungsgemäßen Zubereitungen für die dekorative Kosmetik enthalten bevorzugt weiterhin Konservierungsmittel, Komplexbildner, Antioxidantien, Wirkstoffe wie beschrieben in DE 10 2006 028 549 A1, Abschnitt [0063].

Die Wasserphase der erfindungsgemäßen Zubereitungen für die dekorative Kosmetik können weitere Wirkstoffe wie beschrieben in DE 10 2006 028 549 A1, Abschnitt [0066]. Auf die dort genannten Verdickungsmittel kann aber wegen der Gegenwart der verdickend wirkenden Polyurethane PU verzichtet werden.

Es ist ferner vorteilhaft im Sinne der vorliegenden Erfindung, wenn die Zubereitung einen oder mehrere Filmbildner bzw. Polymere enthält, insbesondere um einen nachvollziehbaren Straffungseffekt auf der Haut zu erzielen, der durch deren Filmbildungseigenschaften hervorgerufen werden kann. Gleichzeitig dienen die Filmbildner zur Fixierung der Pigmente auf der Haut, insbesondere um einen langanhaltenden Effekt und eine Transfer-Resistenz zu erzielen. Erfindungsgemäß geeignete Polymere sowie deren Gehalte sind beschrieben in DE 10 2006 028 549 A1, Abschnitte [0073] bis [0076].

Die wichtigsten Bestandteile der erfindungsgemäß bevorzugten Zubereitung des O/W-Typs sind neben dem die kontinuierliche Phase der Emulsion bildenden Wasser und dem Polyurethan PU eine Wachskomponente, ein mehrwertiger Alkohol und ein Filmbildnersystem. Die Wachskomponente besteht aus wenigstens einem Wachs und gegebenenfalls zusätzlich mindestens einem Fett und/oder Öl, die jeweils pflanzlicher, tierischer, mineralischer oder synthetischer Herkunft sein können. Zusätzlich kann mindestens ein Emulgator und mindestens ein Coemulgator enthalten sein, um die Verarbeitung der Wachskomponente zu einer Emulsion zu erleichtern. Um ein ästhetisch besonders befriedigendes Ergebnis zu erhalten, kann die Wachskomponente zusätzlich ein Polyvinylpyrrolidon-Copolymer enthalten. Die Wachskomponente gibt der Masse die erwünschte Konsistenz und macht die Zusammensetzung Wasser- und tränenfest. Zu diesem Zweck kann die Wachskomponente aus fett-, öl- und wachsartigen Rohstoffen aufgebaut sein, die bei Temperaturen von 21°C bis 25°C flüssig, pastenförmig oder fest sein können. Bevorzugt wird zur Einstellung der optimalen Konsistenz eine Kombination aus mindestens einem Wachs und mindestens einem Öl eingesetzt. Bevorzugte Wachse sind in der DE 10 2005 033 520 A1, Abschnitte [0017] und [0018] beschrieben, worauf hiermit vollumfänglich Bezug genommen wird. Bevorzugte Öle und Fette sind in der DE 10 2005 033 520 A1, Abschnitt [0019] beschrieben, worauf hiermit vollumfänglich Bezug genommen wird. Bevorzugt werden Mischungen aus Wachsen, Ölen und Fetten verwendet, wobei die jeweils ausgeführten Substanzen in solchen Mengen eingesetzt werden, dass die gewünschten Eigenschaften, wie Struktur und Viskosität, erzielt werden. Die jeweils einzusetzenden Mengen und Mischungen sind dem Fachmann bekannt und bedürfen deshalb keiner näheren Erläuterung. Bevorzugte Mengen der Wachse, Fette und Öle sind außerdem in der DE 10 2005 033 520 A1, Abschnitte [0022] und [0023] beschrieben, worauf hiermit vollumfänglich Bezug genommen wird.

Erfindungsgemäß geeignete mehrwertige Alkohole sind beschrieben in der DE 10 2005 033 520 A1, Abschnitt [0026], worauf hiermit vollumfänglich Bezug genommen wird.

Die für die erfindungsgemäßen Zubereitungen für die dekorative Kosmetik geeigneten Filmbildner und deren Mengen sind in der DE 10 2005 033 520 A1, Abschnitte [0027] bis [0030] beschrieben, worauf hiermit vollumfänglich Bezug genommen wird. Die erfindungsgemäßen Zubereitungen für die dekorative Kosmetik können neben den Polyurethanen PU weitere Gelbildner enthalten. Geeignete weitere Gelbildner sind in der DE 10 2005 033 520 A1, Abschnitt [0032] beschrieben, worauf hiermit vollumfänglich Bezug genommen wird.

Shampoos, Conditioner und Cleansing-Produkte

Eine bevorzugte Ausführungsform der Erfindung sind Shampoos und kosmetische Reinigungsmittel enthaltend die Polyurethane PU. An Shampoos und kosmetische Reinigungsmittel werden je nach Haarqualität oder Kopfhautproblem gegebenenfalls zusätzliche Anforderungen gestellt. Die Wirkungsweise der bevorzugten Shampoo-Typen mit den wichtigsten zusätzlichen Wirkungen bzw. wichtigsten speziellen Zielsetzungen wird nachfolgend beschrieben.

Erfindungsgemäß bevorzugt sind beispielsweise Shampoos für normales bzw. schnell fettendes bzw. geschädigtes Haar, Antischuppenshampoos, Babyshampoos und 2-in-1-Shampoos (also Shampoo und Spülung in einem).

Erfindungsgemäße Shampoos für normales Haar: die Haarwäsche soll Haar und Kopfhaut von dem in Talgdrüsen gebildeten Hautfett, den aus Schweissdrüsen mit Wasser austretenden anorganischen Salzen, Aminosäuren, Harnstoff und Milchsäure, abgeschilferten Hautpartikeln, Umweltschmutz, Gerüchen und gegebenenfalls Rückständen haarkosmetischer Behandlungen befreien. Normales Haar bedeutet kurzes bis schulterlanges Haar, welches nur schwach geschädigt ist. Dementsprechend soll der Anteil an Konditionierhilfsstoffen auf diesen Haartyp optimiert sein.

Erfindungsgemäße Shampoos für schnell fettendes Haar: eine erhöhte Fettproduktion der Talgdrüsen der Kopfhaut führt bereits 1-2 Tage nach der Haarwäsche zu einer strähnigen, unansehnlichen Frisur. Öl- und wachsartige Hautfettbestandteile beschweren das Haar und mindern die Reibung von Haar zu Haar und verringern damit den Frisurenhalt. Das eigentliche haarkosmetische Problem bei schnell fettenden Haaren ist also das vorzeitige Zusammenfallen voluminöser Frisuren. Um dies zu vermeiden, muss man verhindern, dass die Haaroberfläche beschwert und zu glatt und geschmeidig wird. Dies wird bevorzugt durch die Tensidgrundlage aus gut reinigenden und besonders wenig substantiv ausgeprägten Waschrohstoffen erreicht. Zusätzliche Pflegestoffe, die sich zum Hautfett addieren würden, wie rückfettende Substanzen werden in Shampoos für schnell fettendes Haar nicht oder nur mit größter Vorsicht eingesetzt. Erfindungsgemäße volumengebende Shampoos für feines Haar können vergleichbar formuliert werden.

Erfindungsgemäße Shampoos für trockenes, strapaziertes (geschädigtes) Haar: Die Struktur des Haares wird im Laufe des Haarwachstums durch mechanische Einflüsse wie Kämmen, Bürsten und vor allen Dingen Toupieren (Kämmen gegen die Wuchsrichtung), durch die Einwirkung von UV-Strahlung bzw. sichtbarem Licht und durch kosmetische Behandlungen, wie Dauerwellen, Blondierung oder Färbung verändert. Die Schuppenschicht der Haare weist von der Wurzel bis zur Spitze ein zunehmend strapazierteres Aussehen auf; in Extremfällen ist sie an der Spitze völlig abgetragen, und die Haarspitzen sind gespalten (Haarspliss). Geschädigtes Haar kann grundsätzlich nicht mehr in den Zustand des gesunden Haarnachwuchses zurückgeführt werden. Es gelingt aber, diesem Idealzustand hinsichtlich Griff, Glanz und Kämmbarkeit durch Verwendung von erfindungsgemäßen Shampoos mit gegebenenfalls hohen Anteilen an pflegenden Stoffen (Konditioniermitteln) sehr nahe zu kommen. Eine noch bessere haarkonditionierende Wirkung als mit einem Shampoo wird mit einem erfindungsgemäßen Haarpflegemittel beispielsweise in Form einer Spülungs- oder Kurmittelbehandlung nach der Haarwäsche erreicht. Spülungs- oder Kurmittel für Haare, die erfindungsgemäße Polymere enthalten, sind ebenfalls erfindungsgemäß.

Erfindungsgemäße 2-in-1- Shampoos sind besonders stark pflegende Shampoos, bei denen durch die Konzipierung als "Shampoo und Spülung in einem" der Zusatznutzen Pflege gleichwertig neben den Grundnutzen Reinigung gestellt wird. Erfindungsgemäße 2-in-1-Zusammensetzungen enthalten erhöhte Mengen an Konditioniermitteln.

Antischuppenshampoos: Erfindungsgemäße Antischuppenshampoos haben im Vergleich zu Antischuppenhaarwässern den Vorteil, dass sie nicht nur durch entsprechende Wirkstoffe gegen Schuppenbefall die Bildung neuer sichtbarer Schuppen verringern und bei langfristiger Anwendung verhindern, sondern mit der Haarwäsche auch bereits abgeschilferte Schuppen entfernen. Nach dem Ausspülen der Waschflotte bleibt allerdings nur ein geringer, jedoch ausreichender Anteil der Wirkstoffe auf Kopfhaut und Haar zurück. Es gibt verschiedene Antischuppen-Wirkstoffe, die in die erfindungsgemäßen Shampoo-Zusammensetzungen eingearbeitet werden können, wie z. B. Zinkpyrithion, Ketokonazol, Elubiol, Clotrimazol, Climbazol oder Pirocton Olamin. Zusätzlich weisen diese Substanzen eine die Abschilferung normalisierende Wirkung auf.

Die Grundlage von Antischuppenshampoos entspricht überwiegend der Formulierung von Shampoos für normales Haar mit gutem Reinigungseffekt. Babyshampoos: bei einer bevorzugten Ausführungsform der Erfindung handelt es sich bei den erfindungsgemäßen Shampoo-Zubereitungen um Babyshampoos. Diese sind optimal haut- und schleimhautverträglich, zB durch Auswahl der Tensidmischung und einem reduzierten Salzgehalt. Kombinationen von Waschrohstoffen mit sehr guter Hautverträglichkeit bilden die Basis dieser Shampoos. Zusätzliche Stoffe zur weiteren Verbesserung der Haut- und Schleimhautverträglichkeit und der Pflegeeigenschaften werden vorteilhaft zugefügt, wie z. B. nichtionische Tenside, Proteinhydrolysate und Panthenol oder Bisabolol. Alle notwendigen Rohstoffe und Hilfsstoffe, wie Konservierungsmittel, Parfümöle, Farbstoffe usw., werden unter dem Aspekt einer hohen Verträglichkeit und Milde ausgewählt.

Shampoos für trockene Kopfhaut: bei einer weiteren bevorzugten Ausführungsform der Erfindung handelt es sich bei den erfindungsgemäßen Shampoo-Zubereitungen um Shampoos für trockene Kopfhaut. Das primäre Ziel dieser Shampoos ist die Verhinderung der Austrocknung der Kopfhaut, da trockene Kopfhaut zu Juckreiz, Rötung und Entzündung führen kann. Wie auch bei den Babyshampoos bilden Kombinationen von Waschrohstoffen mit sehr guter Hautverträglichkeit die Basis dieser Shampoos. Zusätzlich können gegebenenfalls Rückfetter und Feuchthaltemittel, wie z. B. Glycerin oder Harnstoff, eingesetzt werden.

Bevorzugte Shampoos und kosmetische Reinigungsmittel enthalten anionische Tenside. Weitere bevorzugte Shampoos und kosmetische Reinigungsmittel enthalten Kombinationen von anionischen und ampholytischen Tensiden. Weitere bevorzugte Shampoos und kosmetische Reinigungsmittel enthalten Kombinationen von anionischen und zwitterionischen Tensiden. Weitere bevorzugte Shampoos und kosmetische Reinigungsmittel enthalten Kombinationen von anionischen und nichtionischen Tensiden.

Geeignete Tenside aller Typen sind zuvor unter "Tenside" bereits beschrieben. Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen. Besonders bevorzugte anionische Tenside sind die Alkali- oder Ammoniumsalze des Laurylethersulfates mit einem Ethoxylierungsgrad von 2 bis 4 EO-Einheiten.

Ein bevorzugtes zwitterionisches Tensid ist das unter der INCI-Bezeichnung Cocamidopropyl Betaine bekannte Fettsäureamid-Derivat.

Besonders bevorzugte ampholytische Tenside sind das N-Kokosalkylaminopropionat, das Kokosacylaminoethylaminopropionat und das C12-C12-Acylsarcosin.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die erfindungsgemäßen Shampoo-Zusammensetzungen können auch als Shampookonzentrate mit erhöhten Tensidgehalten von 20-30 Gew.-% vorliegen. Sie basieren auf speziellen Waschrohstoffkombinationen und Konsistenzregulatoren, die die gute Verteilbarkeit und das spontane Anschäumvermögen auch einer kleinen Anwendungsmenge gewährleisten. Ein besonderer Vorteil ist beispielsweise die Möglichkeit, die Ergiebigkeit von 200 ml Shampoo mit einer 100-ml-Flasche zu erreichen.

Darreichung

Die Zubereitungen gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels. Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Die Herstellung der erfindungsgemäßen Zubereitungen kann in der üblichen Weise durch Mischen der einzelnen Bestandteile erfolgen. Die Wirkstoffe der erfindungsgemäßen Zubereitungen oder auch die vorgemischten Bestandteile der erfindungsgemäßen Zubereitungen können im Mischvorgang zugegeben werden. Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure/Phosphat-Puffergemischen. Vorzugsweise liegt der pH-Wert unter 10, z.B. im Bereich von 2-7, insbesondere im Bereich von 3-5.

Bevorzugte Shampooformulierungen enthalten

    a) 0,05 bis 10 Gew.-% wenigstens eines Polyurethans PU,
    b) 25 bis 94,95 Gew.-% Wasser,
    c) 5 bis 50 Gew.-% Tenside,
    c) 0 bis 5 Gew.-% eines Konditioniermittels,
    d) 0 bis 10 Gew.-% weitere kosmetische Bestandteile.

In einer weiteren Ausführungsform können durch den Einsatz von Polyurethanen PU auch tensidreduzierte Formulierungen mit weniger als 10 Gew.-% Tensid, bezogen auf die Zubereitung, in einer für die Zubereitung ausreichenden Viskosität hergestellt werden. Insbesondere werden Polyurethane PU zur Einstellung der gewünschten Viskosität in solchen Zubereitungen eingestellt, die mindestens 0,1 Gew.-Salz und von 0,1 bis 10 Gew.-%, bevorzugt weniger als 10 Gew.-% Tensid enthalten.

In den Shampoos und kosmetischen Reinigungsmitteln können alle in Shampoos und kosmetischen Reinigungsmitteln üblicherweise eingesetzten anionischen, neutralen, amphoteren oder kationischen Tenside verwendet werden. Geeignete Tenside wurden vorstehend genannt. Besonders bevorzugt sind Shampoos und kosmetische Reinigungsmittel mit einem Tensidgehalt von mehr als 10 Gew.-%.

In den Shampooformulierungen können zur Erzielung bestimmter Effekte übliche Konditioniermittel eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat®FC, Luviquat®HM, Luviquat®MS, Luviquat®Care), Copolymere aus N-Vinylpyrrolidon/Dimethylaminoethylmethacrylat, quaternisiert mit Diethylsulfat (Luviquat®PQ 11), Copolymere aus N-Vinylcaprolactam/N-Vinylpyrrolidon/N-Vinylimidazoliumsalzen (Luviquat®Hold); kationische Cellulosederivate (Polyquaternium-4 und -10), Acrylamidcopolymere (Polyquaternium-7). Vorteilhafte Konditionierungsmittel stellen beispielsweise die nach INCI als Polyquaternium bezeichneten Verbindungen dar (insbesondere Polyquaternium-1 bis Polyquaternium-87). Die folgende Tabelle gibt eine nicht abschließende Übersicht über Konditioniermittel, die in Kombination mit den erfindungsgemäßen Polymeren eingesetzt werden:

| Bezeichnung nach INCI | CAS-Nummer | Polymertyp | Beispiel (Handelsname) |
|---|---|---|---|
| Polyqua-ternium-2 | CAS 63451-27-4 | Urea, N, N', bis [3-(dimethylamino)propyl]-polymer mit 1, 1'-oxybis (2-chloroethan) | Mirapol® A-15 |
| Polyquaternium-5 | CAS 26006-22-4 | Acrylamid, β-Methacryloxyethyltriethylammoniummethosulfat | |
| Polyquater nium-6 | CAS 26062-79-3 | N,N-Dimethyl-N-2-propenyl-2-propen-aminiumchlorid (PolyDADMAC) | Merquat® 100 |
| Polyquaternium-7 | CAS 26590-05-6 | N,N-Dimethyl-N-2-propenyl-2-propenaminiumchlorid, 2-Propenamid | Merquat® S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SC-230M, Polymer JR 400 |
| Polyquaternium-11 | CAS 53633-54-8 | Vinylpyrrolidon/dimethylaminoethylMethacrylat-Copolymer/Diethylsulfat-Reaktionsprodukt | Gafquat® 755N |
| Polyquaternium-16 | CAS 29297-55-0 | Vinylpyrrolidon/vinylimidazolinummethochlorid-Copolymer | Luviquat® HM552 |
| Polyquaternium-17 | CAS 90624-75-2 | | Mirapol® AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinylocatedecylether | |
| Polyquaternium-21 | | Polysiloxanpolydimethyldimethylammoniumacetat-Copolymer | Abil® B 9905 |
| Polyquaternium-22 | CAS 53694-17-0 | Dimethyldiallylammoniumchlorid/Acrylsäure-Copolymer | Merquat® 280 |
| Polyquaternium-24 | CAS 107897-23-5 | Polymeres quaternäres Ammoniumsalz der Hydroxyethylcellulose | Quartisoft® LM-200 |
| Polyquaternium-28 | CAS 131954-48-8 | Vinylpyrroldon/Methacrylamidopropyltrimethylammoniumchlorid-Copolymer | Gafquat® HS-100 |
| Polyquaternium-29 | CAS 92091-36-6, 148880-30-2 | Chitosan, das mit Propylenoxid umgesetzt u. mit Epichlorhydrin quaternisiert wurde | Lexquat ® CH |
| Polyquaternium-31 | CAS 136505-02-7, 139767-67-7 | Polymeres, quaternäres Ammoniumsalz, das durch die Umsetzung von DMAPA-Acrylate/Acrylsäure/ Acrylonitrogens-Copolymeren u. Diethyl- | Hypan ® QT 100 |
| | | sulfat hergestellt wird | |
| Polyquaternium-32 | CAS 35429-19-7 | N,N,N-Trimethyl-2-([82-methyl-1-oxo-2-propenyl) oxy]-ethanaminium chlorid, polymer mit 2-Propenamid | |
| Polyquaternium-37 | CAS 26161-33-1 | | |

(fortgesetzt)

| Bezeichnung nach INCI | CAS-Nummer | Polymertyp | Beispiel (Handelsname) |
|---|---|---|---|
| Polyquaternium-44 | | Copolymeres quaternes Ammoniumsalz aus Vinylpyrrolidon und quaternisiertem Imidazolin | |
| Polyquaternium-67 | | polymeric quaternary ammonium salt of hydroxyethyl cellulose reacted with a trimethyl ammonium substituted epoxide and a lauryl dimethyl ammonium substituted epoxide | SoftCAT® |
| Polyquaternium-74 | | | Polycare® Boost |
| Polyquaternium-87 | | | Luviquat® Sensation |

Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA).

Die erfindungsgemäßen Shampoos und kosmetischen Reinigungsmittel enthalten bevorzugt Salze. Auf Aniontensiden basierende erfindungsgemäße Shampoos und kosmetische Reinigungsmittel enthalten bevorzugt Natriumchlorid. Ein besonderer Vorteil der vorliegenden Erfindung ist, dass die Viskosität und das optische Erscheinungsbild der erfindungsgemäßen Shampoos und kosmetischen Reinigungsmittel auch bei Konzentrationen von Natriumchlorid von über 0,2 Gew.-% und/oder Tensidkonzentrationen von über 10 Gew.-% über lange Zeit stabil bleiben.

In einer weiteren bevorzugten Ausführungsform der Erfindung enthalten die Shampoos und kosmetischen Reinigungsmittel außerdem mindestens einen Bestandteil aus der Gruppe der wasserunlöslichen Ölkomponenten, der Vitamine, der Provitamine, der Proteinhydrolysate, der Pflanzenextrakte, der UV-Filter, der Aminosäuren, der wasserunlöslichen Silikone, der wasserlöslichen Silikone und/oder der Amodimethicone.

Die Gesamtmenge an Öl- und Fettkomponenten in den erfindungsgemäßen Shampoo-Zubereitungen beträgt üblicherweise 6-45 Gew.-%, bezogen auf die Zubereitung. Mengen von 10-35 Gew.-% sind erfindungsgemäß bevorzugt.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden. Bevorzugt enthalten die erfindungsgemäß verwendeten Zubereitungen Vitamine, Provitamine und Vitaminvorstufen aus den Gruppen A, E, F und H. Selbstverständlich können auch mehrere Vitamine und Vitaminvorstufen gleichzeitig enthalten sein. Die Gesamt-Einsatzmenge der Vitamine, Provitamine, Vitaminvorstufen sowie deren Derivaten in den erfindungsgemäßen Zubereitungen beträgt - bezogen auf das Gesamtgewicht der Zubereitung - 0,01 bis 5 Gew.-%, vorzugsweise 0,02 bis 4 Gew.-% und insbesondere 0,05 bis 3 Gew.-%.

Bevorzugt enthalten die erfindungsgemäßen Shampoos und kosmetischen Reinigungsmittel auch Proteinhydrolysate. Als Proteinhydrolysate im Sinne der Erfindung werden Proteinhydrolysate und/oder Aminosäuren und deren Derivate verstanden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie

Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch P-Aminosäuren und deren Derivate wie P-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Bevorzugte Proteinhydrolysate sind in der DE 10 2006 032 505 A1, Absätze [0077] bis [0079], deren bevorzugte Mengen ebenda in Absatz [0080] beschrieben. Auf diese Textstellen wird hiermit in vollem Umfang Bezug genommen.

Erfindungsgemäß bevorzugt sind tensidhaltige Reinigungsmittel, insbesondere Shampoos, die Pflanzenextrakte enthalten. Bevorzugte Pflanzenextrakte sind in der DE 10 2006 032 505 A1, Absätze [0081] bis [0082], deren bevorzugte Mengen ebenda in Absatz [0086] beschrieben. Auf diese Textstellen wird hiermit in vollem Umfang Bezug genommen.

Erfindungsgemäß bevorzugt sind tensidhaltige Reinigungsmittel, insbesondere Shampoos, die UV-Lichtschutzmittel (UV-Filter) enthalten. Die Wirkung der Zubereitungen kann durch UV-Filter gesteigert werden. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA (315-400 nm)-, im UVB (280-315 nm)- oder im UVC (< 280 nm)-Bereich liegt. UV-Filter mit einem Absorptionsmaximum im Bereich von etwa 280 bis etwa 300 nm sind besonders bevorzugt. Die erfindungsgemäß geeigneten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispiele für erfindungsgemäß verwendbare UV-Filter sind in der DE 10 2006 032 505 A1, Absatz [0089] beschrieben. Auf diese Textstelle wird hiermit in vollem Umfang Bezug genommen.

Gemäß einer weiteren bevorzugten Ausführungsform der Erfindung enthalten die Shampoos und kosmetischen Reinigungsmittel außerdem mindestens ein weiteres wasserunlösliches Silikon, ein wasserlösliches Silikon und/oder ein aminofunktionalisiertes Silikon. Erfindungsgemäß geeignete Silikone bewirken die unterschiedlichsten Effekte. So beeinflussen sie beispielsweise gleichzeitig die Trocken- und Naßkämmbarkeiten, den Griff des trockenen und nassen Haares sowie den Glanz. Unter dem Begriff Silikone versteht der Fachmann mehrere Strukturen siliciumorganischer Verbindungen. Bevorzugte Silikone sind in der DE 10 2006 032 505 A1, Absatz [0113] beschrieben. Auf diese Textstelle wird hiermit in vollem Umfang Bezug genommen.

Gemäß einer weiteren bevorzugten Ausführungsform der Erfindung enthalten die Shampoos und kosmetischen Reinigungsmittel außerdem mindestens einen Emulgator. Bevorzugte Emulgatoren sind in der DE 10 2006 032 505 A1, Absatz [0147], deren bevorzugte Mengen ebenda in Absatz [0148] beschrieben. Auf diese Textstellen wird hiermit in vollem Umfang Bezug genommen.

Gemäß einer weiteren bevorzugten Ausführungsform der Erfindung enthalten die Shampoos und kosmetischen Reinigungsmittel außerdem mindestens ein weiteres Polymer. Bevorzugte Pflanzenextrakte sind in der DE 10 2006 032 505 A1, Absätze [0151] bis [0165], deren bevorzugte Mengen ebenda in Absatz [0167] beschrieben. Auf diese Textstellen wird hiermit in vollem Umfang Bezug genommen.

Die erfindungsgemäßen Shampoos und kosmetischen Reinigungsmittel können auch organische Lösungsmittel wie Ethanol, Propanol, Isopropanol, Benzylalkohol, Benzyloxyethanol, Ethoxydiglykol, Alkylencarbonate wie Ethylencarbonat und Propylencarbonat, Phenoxyethanol, Butanol, Isobutanol, Cyclohexan, Cyclohexanol, Hexylenglykol, Ethylencarbonat, Propylenglykol, Polypropylenglykole, Ethylenglykolmonoethylether, Ethylenglykolmonobutylether, Ethylenglykolmonophenylether, 1-Phenylethylalkohol, 2-Phenylethylalkohol und o-Methoxyphenol enthalten. Besonders bevorzugte organische Lösungsmittel sind Benzylalkohol, Benzyloxyethanol und Propylen-glykole. Die Menge der organischen Lösungsmittel in den erfindungsgemäßen Zubereitungen sollte 5 Gew.-% nicht überschreiten, vorzugsweise im Bereich von 0,1 bis 3%, besonders bevorzugt 0,5 bis 2,5 Gew.-%, berechnet auf die Zubereitung, liegen.

Lösungsvermittler können den Zubereitungen zugegeben werden, besonders wenn ölige Substanzen als Pflegemittel und Parfümöle mit hohen lipophilen Eigenschaften ausgewählt wurden. Typische Lösungsvermittler können hydrogenierte Talköle sein (beispielsweise Cremophor®RH). Es sollte angemerkt werden, dass auch Tensidgemische gute Lösungsvermittler für Parfümöle sein können. Übliche Mengen der Lösungsvermittler können im Bereich von 0,01 bis 2 Gew.-% besonders bei 0,1 bis 1 Gew.-%, berechnet auf die Gesamtzusammensetzung, liegen.

Weitere vorteilhafte Inhaltsstoffe der erfindungsgemäßen Shampoos und kosmetischen Reinigungsmittel und kosmetischen Reinigungsmittel sind in der DE 10 2006 032 505 A1, Absatz [0168] beschrieben. Auf diese Textstelle wird hiermit in vollem Umfang Bezug genommen.

Besonders bevorzugt sind Shampoos und kosmetische Reinigungsmittel, die wenigstens ein Polyurethan PU als Verdickungsmittel, wenigstens ein Alkylsulfat und/oder Alkylethersulfat (beispielsweise Natriumlaurylsulfat) und wenigstens ein quarternäres Ammoniumsalz (beispielsweise Cetyldimethyl-(2)-hydroxyethylammoniumdihydrogenphosphat) enthalten. Solche Zubereitungen können dank der Verwendung des Polyurethans PU als klare, transparente Zubereitungen formuliert werden, was durch herkömmliche Carbomere nicht erreicht wird.

Deo- und Antitranspirant-Zubereitungen

Die Erfindung betrifft in einer bevorzugten Ausführungsform Deodorantien oder Antitranspirantien, insbesondere Deo-Lotionen und Deodorant- oder Antitranspirant-Stifte, auf Basis einer Öl-in-Wasser-Dispersion/Emulsion zur Applikation von Wirkstoffen, insbesondere von wasserlöslichen Wirkstoffen, auf die Haut. Durch Verwendung von Polyurethan PU können mikro-, makro-, Creme- und Spray- Deo- und Antitranspirant-Zubereitungen dargestellt werden. Handelsübliche Deodorantien und Antitranspirantien werden meistens als Sprays oder als Stift konfektioniert; daneben gibt es Roll-on-Präparate und Cremes im Markt. Üblicherweise werden Antitranspirantien in mannigfaltigen Produktformen angeboten, wobei in Europa Roller, Pumpzerstäuber und Aerosole dominieren, in den USA, Mittel- und Südamerika Stifte. Es sind sowohl wasserfreie als auch wasserhaltige Produkte (hydro-alkoholische Formulierungen, Emulsionen) bekannt. Das grundsätzliche Problem bei Antitranspirantien enthaltenden Emulsionen besteht in einem destabilisierenden Effekt hoher Gehalte an Elektrolyten, insbesondere bei Zubereitungen mit niedrigen pH-Werten. Dadurch sind Emulsionen nicht lagerstabil und unterliegen oft Erscheinungen wie Aufrahmung oder Sedimentation. Dies gilt insbesondere für dünnflüssige Emulsionen mit niedriger Viskosität. Gleichzeitig müssen derartige Zubereitungen vom Anwender als angenehm anwendbar empfunden werden. Viele stiftförmige Antitranspirant-Präparate werden als wasserfreie Suspensionsstifte formuliert. Derartige Präparate hinterlassen beim Anwender ein angenehm trockenes Hautgefühl nach dem Auftragen. Eine effektive Freisetzung der wasserlöslichen Antitranspirant-Wirkstoffe aus solchen Präparaten ist jedoch limitiert (vgl. Chemistry and Technology of the Cosmetics and Toiletries Industry, Hrsg.: D. F. Williams und W. H. Schmitt, London: Blackie, 1996,2. Auflage, S. 326), und es wird meist nicht das von vielen Verbrauchern geschätzte Frischegefühl erzielt. Die wasserfreien Präparate, insbesondere solche auf Basis von flüchtigen Siliconölen, haben den Nachteil, dass die dispergierten Wirkstoffe leicht zu sichtbaren Produktrückständen auf Haut und Kleidung führen. Außerdem sind solche Zubereitungen relativ kostspielig, da die Ölkomponenten als Wirkstoffträger teurer sind als Wasser. Unter der Druckbelastung bei der Applikation kommt es häufig zu einem Ausölen, was die kosmetische Akzeptanz dieser Präparats beim Anwender herabsetzt.

Die erfindungsgemäß verwendeten Polyurethane PU sind als Verdicker insbesondere in Deo- und Antitranspirant-Zubereitungen mit hohen Gehalten an Elektrolyten und/oder sauren Deo- und Antitranspirant-Zubereitungen vorteilhaft. Insbesondere bei Salzgehalten von über 3, bevorzugt über 5 Gew.-%, bezogen auf die gesamte Zubereitung sind die Polyurethane PU vorteilhaft als Verdicker zu verwenden. Inbesondere bei pH-Werten im Bereich von 3 bis 6, bevorzugt von 4 bis 5,5 sind die Polyurethane PU vorteilhaft als Verdicker zu verwenden.

Insbesondere ist es durch die Verwendung der Polyurethane PU möglich, stabile kosmetische und/oder dermatologische Zubereitungen mit Antiperspirant-Wirkstoffen wie beispielsweise Aluminiumchlorohydrat bei einem pH-Wert im Bereich zwischen 3,5 und 6 herzustellen.

Der Antitranspirant-Wirkstoff ist in den bevorzugten O/W-Zubereitungen in der äußeren, kontinuierlichen wässrigen Phase gelöst, so dass sich eine deutlich verbesserte und effizientere Wirkstoffabgabe im Vergleich zu den bekannten wasserfreien Zubereitungen ergibt, insbesondere im Vergleich zu Suspensionsstiften und Wasser-in-Öl-Emulsionsstiften.

Die Messung des elektrischen Widerstandes derartiger Zusammensetzungen ist auch ein geeignetes Verfahren, um schnell und einfach die Unterscheidung zwischen einem Öl-in-Wasser- und einem Wasser-in-Öl-System treffen zu können. Ein Öl-in-Wasser-System weist wegen der kontinuierlichen Wasserphase eine hohe elektrische Leitfähigkeit und dementsprechend einen niedrigen elektrischen Widerstand auf. Diese Wirkstofffreisetzung lässt sich indirekt sehr gut bestimmen über die Messung des elektrischen Widerstandes des jeweiligen Produktes.

Neben der günstigen Wirkstofffreisetzung bringt eine Konfektionierung als Öl-in-Wasser-Dispersion/Emulsion weitere Vorteile mit sich. Zum einen kann die Zusammensetzung leicht von der Haut abgewaschen werden. Zum anderen bildet sich bei oder nach dem Auftragen auf die Haut zusammen mit der Hautfeuchtigkeit eine pflegende Öl-in-Wasser-Creme aus, die ein erfrischendes, kühlendes Hautgefühl erzeugt.

Die erfindungsgemäßen Deo- und Antitranspirant-Zubereitungen, insbesondere in Stiftform, umfassen in einer bevorzugten Ausführungsform Lipide und/oder Wachse. Geeignete Lipide und Wachse sind in DE 10 2006 021 780 A1, Absätze [0033] bis [0041] beschrieben, worauf an dieser Stelle in vollem Umfang Bezug genommen wird.

Bevorzugt ist die Lipid- oder Wachskomponente a) ausgewählt ist aus Estern aus einem gesättigten, einwertigen C16-C60-Alkanol und einer gesättigten C8-C36-Monocarbonsäure, insbesondere Cetylbehenat, Stearylbehenat und C20-C40-Alkylstearat, Glycerintriestern von gesättigten linearen C12-C30-Carbonsäuren, die hydroxyliert sein können, Candelillawachs, Carnaubawachs, Bienenwachs, gesättigten linearen C14-C36-Carbonsäuren sowie Mischungen der vorgenannten Substanzen. Besonders bevorzugte Lipid- oder Wachskomponenten-Mischungen a) sind ausgewählt aus Mischungen von Cetylbehenat, Stearylbehenat, gehärtetem Rizinusöl, Palmitinsäure und Stearinsäure. Weitere besonders bevorzugte Lipid- oder Wachskomponenten-Mischungen a) sind ausgewählt aus Mischungen von C20-C40-Alkylstearat, gehärtetem Rizinusöl, Palmitinsäure und Stearinsäure. Weitere bevorzugte erfindungsgemäße Deodorant- oder Antitranspirant-Stifte sind dadurch gekennzeichnet, dass die Lipid- oder Wachskomponenteln a) insgesamt in Mengen von 4-20 Gew.-%, bevorzugt 8-15 Gew.-%, bezogen auf die Gesamtzusammensetzung, enthalten ist. In einer besonders bevorzugten Ausführungsform sind die Ester aus einem gesättigten, einwertigen C50-C60-Alkohol und einer gesättigten C8-C36-Monocarbonsäure, die die Lipid- oder Wachskomponente a) darstellen, in Mengen von 2-10 Gew.-%, bevorzugt 2-6 Gew.-%, bezogen auf die Gesamtzusammensetzung, enthalten.

Bevorzugte erfindungsgemäße Deo- und Antitranspirantzubereitungen enthalten mindestens einen nichtionischen Öl-in-Wasser-Emulgator mit einem HLB-Wert von mehr als 7. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L):5 berechnet, wobei L der Gewichtsanteil der lipophilen Gruppen, das heißt der Fettalkyl-oder Fettacylgruppen, in den Ethylenoxidaddukten, ausgedrückt in Gewichtsprozent, ist.

Geeignete Öl- in Wasser-Emulgatoren sind in DE 10 2006 021 780 A1, Absätze

[0044] bis [0050], bevorzugte Mengen in Absatz [0051] beschrieben, worauf an dieser Stelle in vollem Umfang Bezug genommen wird.

Bevorzugte erfindungsgemäße Deo- und Antitranspirantzubereitungen enthalten weiterhin mindestens einen nichtionischen Wasser-in-Öl-Emulgator mit einem HLB-Wert größer 1,0 und kleiner/gleich 7,0.

Geeignete Wasser-in-Öl-Emulgatoren sind in DE 10 2006 021 780 A1, Absätze

[0053] bis [0056], bevorzugte Mengen in den Absätzen [0057] bis [0058] beschrieben, worauf an dieser Stelle in vollem Umfang Bezug genommen wird.

Bevorzugte erfindungsgemäße Deo- und Antitranspirantzubereitungen enthalten bevorzugt mindestens ein Öl. Geeignete Öle sind in DE 10 2006 021 780 A1, Absätze

[0062] bis [0066] und [0073] beschrieben, worauf an dieser Stelle in vollem Umfang Bezug genommen wird.

Bevorzugte erfindungsgemäße Deo- und Antitranspirantzubereitungen enthalten bevorzugt mindestens ein Polyol. Geeignete Polyole sind in DE 10 2006 021 780 A1, Absätze [0080] und [0081] beschrieben, worauf an dieser Stelle in vollem Umfang Bezug genommen wird.

Bevorzugte erfindungsgemäße Deo- und Antitranspirantzubereitungen enthalten weiterhin mindestens einen Deodorant- und/oder Antitranspirant-Wirkstoff.

Erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe. Geeignete Deodorant-Wirkstoffe sind in DE 10 2006 021 780 A1, Absätze [0087] und [0093] beschrieben, worauf an dieser Stelle in vollem Umfang Bezug genommen wird. Die Menge der Deodorant-Wirkstoffe (eine oder mehrere Verbindungen) in den Zubereitungen beträgt vorzugsweise 0,01 bis 10 Gew.%, besonders bevorzugt 0,05 bis 5 Gew.%, insbesondere 0,1 bis 1 Gew.% bezogen auf das Gesamtgewicht der Zubereitung.

Bevorzugte erfindungsgemäße Deodorant- oder Antitranspirant-Zubereitungen sind dadurch gekennzeichnet, dass mindestens ein Antitranspirant-Wirkstoff, ausgewählt aus den wasserlöslichen adstringierenden anorganischen und organischen Salzen des Aluminiums, Zirkoniums und Zinks bzw. beliebigen Mischungen dieser Salze, enthalten ist. Geeignete Antitranspirant -Wirkstoffe sind in DE 10 2006 021 780 A1, Absätze [0095] und [0096] beschrieben, worauf an dieser Stelle in vollem Umfang Bezug genommen wird.

Die erfindungsgemäßen Zubereitungen können in einer weiteren besonders bevorzugten Ausführungsform sowohl mindestens einen Deodorant- als auch mindestens einen Antitranspirant-Wirkstoff enthalten.

Bevorzugte erfindungsgemäße Deo- und Antitranspirantzubereitungen können auch niedrigschmelzende Lipid- oder Wachskomponenten enthalten, wie in DE 10 2006 021 780 A1, Absatz [98] beschrieben, worauf an dieser Stelle in vollem Umfang Bezug genommen wird.

Bevorzugte erfindungsgemäße Deo- und Antitranspirantzubereitungen können auch Füllstoffe enthalten, wie in DE 10 2006 021 780 A1, Absätze [100] und [101] beschrieben, worauf an dieser Stelle in vollem Umfang Bezug genommen wird.

Bevorzugte erfindungsgemäße Deo- und Antitranspirantzubereitungen enthalten Duftstoffe, wie in DE 10 2006 021 780 A1, Absätze [105] bis [108] beschrieben, worauf an dieser Stelle in vollem Umfang Bezug genommen wird.

Bevorzugte erfindungsgemäße Zubereitungen in Form von Deodorant- oder Antitranspirant-Stifte sind dadurch gekennzeichnet, dass weiterhin Pigmente, z. B. Titandioxid, enthalten sind. Der Pigmentgehalt unterstützt die kosmetische Akzeptanz des Präparates beim Anwender. Weiterhin sind besonders bevorzugte erfindungsgemäße Deodorant- oder Antitranspirant-Stifte dadurch gekennzeichnet, dass sie die üblichen Bestandteile kosmetischer Präparate enthalten, z. B. Farbstoffe, Nanosphären, Konservierungs- und Lichtschutzmittel, Antioxidantien, Enzyme sowie Pflegestoffe. Diese sind in besonders bevorzugten erfindungsgemäßen Deodorant- oder Antitranspirant-Stiften vorzugsweise in einer Menge von 0,001 bis 20 Gew.-% enthalten.

Zur Produktstabilisierung können die zuvor beschriebenen Antioxidantien, Radikalfänger, UV-Filter, Komplexbildner und Konservierungsmittel verwendet werden.

In einer Ausführungsform der Erfindung enthalten die Deodorant- oder Antitranspirant-Zubereitungen Haarwuchsinhibitoren. Geeignete Haarwuchsinhibitoren sind in DE 10 2006 021 780 A1, Absatz [0120] beschrieben, worauf an dieser Stelle in vollem Umfang Bezug genommen wird.

Haarfärbemittel

Die Polyurethane PU sind weiterhin insbesondere als Verdickungsmittel für Peroxid enthaltende Zubereitungen wie beispielsweise Haarfärbemittel geeignet. Eine weitere Ausführungsform der vorliegenden Erfindung sind also Haarfärbemittel enthaltend die Polyurethane PU sowie die Verwendung der Polyurethane PU als Verdickungsmittel in Haarfärbemitteln. Durch den Einsatz von PU können insbesondere tropfreie und/oder nicht fadenziehende Systeme aufgebaut werden.

Zusammensetzungen zum Färben von Haaren (Haarfärbemittel) werden je nach ihrer Farbbeständigkeit in drei Klassen eingeteilt: Temporäre Haarfärbemittel, die nur 1-2 Haarwäschen haltbar sind, semipermanente Haarfärbemittel, die nach 8-10 Haarwäschen erneuert werden müssen und permanente Haarfärbemittel, die sich nicht herauswaschen lassen.

Temporäre und semipermanente Haarfärbemittel werden als nicht oxidativ bezeichnet. Hier lagern sich die Farbstoffe an das Keratin des Haares an oder dringen in die Haarfaser ein. Bei permanenten Haarfärbemitteln, den mit Abstand am weitesten verbreiteten Haarfärbemitteln, werden die Farben aus farblosen Vorstufen durch chemische Reaktion in der Gegenwart von Wasserstoffperoxid, das als Oxidationsmittel dient, direkt auf und im Haar gebildet. Dabei wird das Haar vollständig durchgefärbt, die Farbe ist nicht auswaschbar. Man bezeichnet diese Haarfärbemittel als oxidative Haarfärbemittel.

Die permanente Haarfärbung ist sehr beständig gegenüber der Haarwäsche, Lichteinwirkung und anderen Haarbehandlungsmethoden. Sie ist am weitesten verbreitet und besitzt unter den Haarfärbemitteln einen Marktanteil von ca. 80%. Sie braucht nur, bedingt durch den Haarwuchs, etwa jeden Monat erneuert zu werden. Bei diesem Färbesystem werden die Farbstoffe direkt auf und im Haar gebildet, und zwar durch chemische Reaktionen, denen die ungefärbten Zwischenprodukte bzw. Vorprodukte unterworfen werden. Es laufen dabei Oxidationsreaktionen und Kupplungsvorgänge bzw. Kondensationen ab, die durch Wasserstoffperoxid in Gegenwart von Ammoniak oder Monoethanolamin hervorgerufen werden. Die Verwendung von Wasserstoffperoxid als Oxidationsmittel ist deshalb erforderlich, weil es nicht nur die Farbstoffbildung initiiert, sondern gleichzeitig auch die Melanin-Pigmente des Haares zerstört und auf diese Weise eine Blondierung hervorruft, weshalb man bei diesem Färbevorgang auch von einer aufhellenden Färbung spricht.

Zu den permanenten Haarfärbemitteln sind prinzipiell auch die sog. selbstoxidierenden Farbstoffe zu rechnen, die bereits durch Luftsauerstoff oxidiert werden.

Haarfärbemittel liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole.

Oxidations-Haarfärbemittel bestehen in der Regel aus zwei Komponenten, nämlich

    (A) der die Farbstoffe enthaltenden Farbstoffträgermasse und
    (B) der Oxidationsmittelzubereitung.

Diese Komponenten werden kurz vor der Anwendung vermischt und dann auf die zu färbende Faser aufgetragen.

Übliche Darreichungsformen für solche Permanent-oder Oxidations-Haarfärbemittel sind Cremehaarfarben, Haarfärbegele und Färbeshampoos. Um zu gewährleisten, dass die aktiven Inhaltsstoffe der Haarfärbezusammensetzungen nach der Anwendung für eine gewisse Zeit auf dem Haar verbleiben und nicht an Stellen gelangen, an denen sie unerwünscht sind, wie beispielsweise das Gesicht, müssen die Zusammensetzungen eine gewisse Mindestviskosität aufweisen. Diese Viskosität wird üblicherweise durch den Einsatz von Verdickungsmitteln erreicht, die somit ein wesentlicher Bestandteil der meisten Oxidations-Haarfärbemittel sind.

Als Verdickungsmittel werden üblicherweise vernetzte Polyacrylsäuren (z.B. Carbopol®), Hydroxyethylcellulose, Wachse und besonders Mischungen von nicht-ionischen Tensiden mit bestimmtem HLB-Wert (Hydrophobic Lipophilic Balance), anionische, kationische oder nicht-ionische Assoziationspolymere verwendet. Aufgrund der teilweise sehr hohen Salzkonzentrationen sind die weit verbreiteten Verdicker-Systeme auf Basis von Tensiden und auch Polyacrylsäuren häufig nicht mehr in der Lage, den Zubereitungen die notwendige Viskosität zu verleihen. Verdicker auf Basis von Polyacrylsäure haben außer der geringen Salztoleranz den weiteren Nachteil, dass sich das Anfassgefühl der behandelten Haare verschlechtert. Sie erlauben es häufig nicht oder in nur unzureichendem Maß, das Haar gut und gleichmäßig, d.h. mit möglichst geringer Selektivität einzufärben, und es gleichzeitig in einen guten kosmetischen Zustand zu versetzen. Häufig sind die Viskositäten der Zusammensetzungen nicht stabil sondern sinken mit der Zeit, so dass die Mittel nicht mehr am Haar haften und auf die Kopfhaut abfließen, wo sie dann eine unerwünschte Färbung hervorrufen.

Eine Aufgabe dieser Erfindung bestand nun darin, Verdickungsmittel für Wasser enthaltende kosmetische Zusammensetzungen zum Färben von Keratinfasern bereitzustellen, die für eine ausreichend hohe Viskosität sorgen, so dass das Haarfärbemittel für die Dauer der Anwendung an der gewünschten, zu färbenden Stelle verbleibt. Die derart verdickten Zusammensetzungen sollen tropffest sein, das Haar gut und gleichmäßig, d.h. mit möglichst geringer Selektivität einfärben und es weiterhin in einen guten kosmetischen Zustand versetzen. Insbesondere soll die verdickende Wirkung für die jeweils gewünschte Anwendung auch bei hohen Salz-(Elektrolyt-) konzentrationen ausreichend sein.

In bevorzugten erfindungsgemäßen Zubereitungen zum Färben von Keratinfasern liegt das Polyurethan PU bevorzugt zunächst in der Peroxid enthaltenden Komponente bei einem pH-Wert im Bereich von 1 bis 4, bevorzugt von 2 bis 3 vor. Nach dem Mischen mit der die Farbstoff-Vorstufen enthaltenden Komponente liegt das Polyurethan PU in der Mischung bei einem pH-Wert im Bereich von 7 bis 12, vorzugsweise von 8 bis 10, besonders bevorzugt von 8,5 bis 9,5 und insbesondere bei pH von etwa 9 vor.

Kosmetische Haarfärbezusammensetzungen, die Polyurethan PU enthalten, lassen sich einfach zu Gelen mit sehr guten Eigenschaften formulieren.

Erfindungsgemäße kosmetische Zubereitungen zum Färben von Keratinfasern enthalten, bezogen auf die Zusammensetzung, bevorzugt 0,05 bis 10 Gew.-%, besonders bevorzugt 1 bis 7 und insbesondere 2 bis 6 Gew.-% wenigstens eines Polyurethans PU.

Ein bevorzugter Gegenstand der Erfindung ist eine kosmetische Zusammensetzung zum Färben von Keratinfasern umfassend wenigstens 2 Komponenten (A) und (B), wobei

Komponente (A) wenigstens einen Oxidationsfarbstoff und

Komponente (B) wenigstens ein Oxidationsmittel und wenigstens ein Polyurethan PU enthalten.

Vorteilhafterweise werden (A) und (B) getrennt voneinander bereitgestellt und höchstens 30 Sekunden, bevorzugt höchstens 20 Sekunden vor dem Inkontaktbringen mit den Keratinfasern miteinander in Kontakt gebracht.

Das als Verdicker wirkende Polyurethan PU kann in Komponente A und/oder B enthalten sein. In einer bevorzugten Ausführungsform ist das Polyurethan PU in Komponente B enthalten.

Oxidationsfarbstoffe

Diese Verbindungen, die im Ausgangszustand keine Farbstoffe im eigentlichen Sinn sind, sondern Farbstoffvorprodukte darstellen, werden ihrer chemischen Beschaffenheit nach in Oxidationsbasen (Entwickler) und Kuppler (Nuancierer) eingeteilt. Oxidationsbasen sind aromatische Verbindungen, die mit mindestens zwei elektronenabgebenden Gruppen (z.B. Amino-und/oder Hydroxy-Gruppen) kernsubstituiert sind und daher leicht oxidierbar sind.

Wichtige Vertreter dieser Basen sind p-und o-Phenylendiamin, p-und o-Aminophenol sowie p-und o-Dihydroxybenzol und zahlreiche Derivate, die sich von diesen Verbindungen, z.B. durch Substitution der Amino-Gruppe durch die Methoxy-Gruppe oder durch den Ersatz des Benzol-Rings durch andere Ringsysteme wie Pyridin, Indol, Chinolin usw.,ableiten.

Eine dominierende Stellung als Farbstoffbasen nehmen bei den Oxidationsfarbstoffen jedoch p-Phenylendiamin und p-Toluylendiamin ein.

Bei den Kupplern handelt es sich ebenfalls um aromatische Verbindungen, die wie die Oxidationsbasen am Ring leicht oxidierbare Gruppen (ebenfalls Amino-und/oder Hydroxy-Gruppen) tragen, jedoch in der m-Position. Wichtige Kuppler sind m-Phenylendiamin, m-Aminophenol und m-Dihydroxybenzol.

Das erfindungsgemäße Haarfärbemittel enthält vorzugsweise Oxidationsfarbstoffvorstufen, bei denen die Färbung unter Einwirkung von Oxidationsmitteln, wie zum Beispiel Wasserstoffperoxid, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:

Geeignete Entwicklersubstanzen sind beispielsweise die in WO 02/00181, S.8, Z.34 bis S.13, Z.28 und die in DE 103 51 842 A1, Absatz [0015] beschriebenen, worauf hiermit in vollem Umfang Bezug genommen wird.

Geeignete Kupplersubstanzen sind beispielsweise die in WO 02/00181, S. 13, Z.30 bis S.14, Z.14 und die in DE 103 51 842 A1, Absatz [0016] beschriebenen, worauf hiermit in vollem Umfang Bezug genommen wird.

Die Gesamtmenge der in den erfindungsgemäßen Zusammensetzungen enthaltenen Oxidationsfarbstoffvorstufen beträgt etwa 0,01 bis 12 Gewichtsprozent, insbesondere etwa 0,2 bis 6 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo, Triphenylmethanfarbstofte, aromatische Nitrofarbstofte, Azofarbstofte, Chinonfarbstofte, kationische oder anionische Farbstoffe in den Zusammensetzungen enthalten sein.

Geeignete synthetische Farbstoffe sind beispielsweise die in DE 103 51 842 A1, Absatz [0017] bis [0019] beschriebenen, worauf hiermit in vollem Umfang Bezug genommen wird.

Wenn die erfindungsgemäßen Zubereitungen direktziehende Farbstoffe enthalten, so beträgt die Menge, bezogen auf die Zubereitung etwa 0,01 bis 7 Gewichtsprozent, vorzugsweise etwa 0,2 bis 4 Gewichtsprozent.

Weitere zur Haarfärbung bekannte und übliche Farbstoffe, die in den erfindungsgemässen Zubereitungen enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben, auf die hiermit ausdrücklich Bezug genommen wird.

Obwohl Oxidationshaarfärbemittel bevorzugt sind, ist es selbstverständlich ebenfalls möglich, dass die erfindungsgemässen Zusammensetzungen in Form eines nichtoxidativen Färbemittels auf Basis der vorstehend genannten direktziehenden Farbstoffen vorliegen.

Bevorzugte erfindungsgemäße Zubereitungen enthalten als Komponenten (A) und (B)

(A) wenigstens eine Entwicklersubstanz und/oder wenigstens eine zusätzliche Kupplersubstanz und/oder wenigstens einen direktziehenden Farbstoff und

(B) wenigstens ein Oxidationsmittel und wenigstens ein Polyurethan PU.

Ein weiterer Gegenstand der Erfindung ist eine Zubereitung enthaltend wenigstens ein Oxidationsmittel und wenigstens ein Polyurethan PU.

Darüberhinaus können die erfindungsgemässen Zubereitungen Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten.

Weiterhin können die erfindungsgemässen Zubereitungen vorzugsweise weitere, für Haarfärbemittel übliche Zusätze, wie zum Beispiel höhere Fettalkohole, Konservierungsstoffe, Komplexbildner, Lösungsmittel wie niedere aliphatische Alkohole, beispielsweise Ethanol, Propanol oder Isopropanol, oder Glykole wie Glycerin oder 1,2-Propylenglykol, Netzmittel oder Emulgatoren aus den Klassen der anionischen, kationischen, amphoteren oder nichtionogenen oberflächenaktiven Substanzen, Weichmacher, Vaseline, Silikonöle, Paraffinöl, Polysorbate und Fettsäuren sowie außerdem Pflegestoffe, wie kationische Polymere oder Harze, Lanolinderivate, Cholesterin, Vitamine, Pantothensäure und Betain, enthalten. Obwohl nicht erforderlich, können die Zubereitungen auch weitere Verdickungsmittel wie beispielsweise Homopolymere der Acrylsäure, hydrophob-modifizierte Polyacrylsäure, Pflanzen Gums, Cellulose- und Stärkederivate, Algenpolyasaccharide oder amphiphile Assoziativverdicker enthalten.

Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent, bezogen auf die Zubereitung.

Die erfindungsgemäße Zubereitung wird vorzugsweise in Form einer wässrigen oder wässrig-alkoholischen Zubereitung, beispielsweise als verdickte Lösung, als Emulsion, als Creme oder als Gel, konfektioniert.

Für die Anwendung zur oxidativen Färbung vermischt man die vorstehend beschriebene Komponente A in der Regel unmittelbar vor dem Gebrauch mit der das Oxidationsmittel enthaltenden Komponente B und trägt eine für die Färbung ausreichende Menge, in der Regel etwa 60 bis 200 Gramm, der gebrauchsfertigen Zubereitung auf die Faser auf.

Der pH-Wert der erfindungsgemäßen Zubereitung liegt bei nichtoxidativen Färbemitteln auf der Basis von direktziehenden Farbstoffen im Bereich von etwa 5 bis 10, vorzugsweise 6 bis 9.

Handelt es sich bei den Zubereitungen zum Färben von Keratinfasern um Zwei- oder Mehrkomponentensysteme, also Systeme, die vor dem Inkontaktbringen mit der Keratinfaser gemischt werden, so liegt der pH-Wert dieser Mischung im Bereich von 6 bis 12, vorzugsweise von 8 bis 11, besonders bevorzugt von 8,5 bis 9,5 und insbesondere beträgt der pH-Wert etwa 9.

Der pH-Wert der Peroxid enthaltenden Komponente liegt im Bereich von 1 bis 4, bevorzugt von 2 bis 3. Der pH-Wert der die Farbstoff-Vorstufen und Kuppler enthaltenden Komponente (Farbträgermasse) liegt im Bereich von 9 bis 11, bevorzugt bei etwa 10. Des pH-Wert des gebrauchsfertigen erfindungsgemäßen Zubereitung stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird.

Das zur erfindungsgemäßen Verwendung geeignete Polyurethan PU kann in einer oder in beiden der vorgenannten Komponenten enthalten sein. Bevorzugt ist es in der der Peroxid enthaltenden Komponente enthalten und liegt somit auch bei einem pH-Wert im Bereich von 1 bis 4, bevorzugt von 2 bis 3 vor.

Je nach Zubereitung und gewünschtem pH-Wert der Zubereitung erfolgt die Einstellung des pH-Wertes vorzugsweise mit Ammoniak oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Sofern die erfindungsgemäße Zubereitung keine Oxidationsfarbstoffvorstufen enthält beziehungsweise Oxidationsfarbstoffvorstufen enthält, welche mit Luftsauerstoff leicht oxidierbar sind, kann es ohne vorheriges Vermischen mit einem Oxidationsmittel direkt auf die Keratinfaser aufgetragen werden. Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12 Gew.-%igen, vorzugsweise 1,5 - bis 6 Gew.-%igen wässrigen Lösung in Betracht. Das Mischungsverhältnis von Färbemittel zu Oxidationsmittel ist abhängig von der Konzentration des Oxidationsmittels und beträgt in der Regel etwa 5:1 bis 1:2, vorzugsweise etwa 1:1, wobei der Gehalt an Oxidationsmittel in der gebrauchsfertigen Zubereitung vorzugsweise etwa 0,5 bis 8 Gew.-%, insbesondere 1 bis 4 Gew.-%, beträgt.

Man lässt das gebrauchsfertige Färbemittel bei 15 bis 50°C etwa 10 bis 45 Minuten, vorzugsweise etwa 15 bis 30 Minuten lang, auf die Keratinfaser (zum Beispiel menschliche Haare) einwirken, spült sodann die Faser mit Wasser aus und trocknet sie. Gegebenenfalls wird im Anschluß an diese Spülung mit einem Shampoo gewaschen und eventuell mit einer schwachen organischen Säure, wie zum Beispiel Weinsäure, nachgespült. Anschließend wird die Keratinfaser getrocknet.

Ein Gegenstand der Erfindung ist ein Verfahren zum Färben von Keratinfasern, insbesondere menschlichen Haaren, dadurch gekennzeichnet, dass die erfindungsgemäße Zubereitung mit den zu färbenden Keratinfasern in Kontakt gebracht wird und die Färbung im pH-Wert-Bereich von 8 bis 10 stattfindet, wobei Komponente (A) und Komponente (B) vor dem Inkontaktbringen mit den Keratinfasern gemischt werden und das Inkontaktbringen stattfindet, wenn die Mischung eine dynamische Viskosität von wenigstens 3000 mPa*s besitzt.

Haarpflegemittel

Ziel der Haarpflege ist es, den Naturzustand des frisch nachgewachsenen Haares über einen langen Zeitraum zu erhalten und im Falle seines Verlusts möglichst wieder herzustellen. Strahlender Glanz und ein angenehmer, glatter Griff gelten als Merkmale für natürliches, gesundes Haar.

Als Haarpflegemittel werden im Rahmen dieser Erfindung Vorbehandlungsmittel, Haarspülungen (Haarconditioner, Haarbalsame), Haarkuren, wobei man zwischen den Kurprodukten unterscheidet, die im Haar verbleiben (leaveon) und denen, die ausgespült werden (rinse-off), Haarwässer, Frisiermittel wie zum Beispiel Pomaden, Frisiercremes, Frisierlotionen, Frisiergele (Haargele, Wet-Look-Gele, Glittergele), Spitzenfluids, Hot-Oil-Treatments und Schaumkuren.

Dem Fachmann bekannte, übliche Rezepturen der genannten Haarpflegemittel werden in "Kosmetik und Hygiene von Kopf bis Fuß", Hrsg. W. Umbach, 3. Auflage, Wiley-VCH, 2004, Kap. 9.2, S.247-264 genannt, worauf an dieser Stelle vollumfänglich Bezug genommen wird. Die neben den Polyurethanen PU in den Haarpflegemitteln enthaltenen Inhaltsstoffe sind vor- und nachstehend genannt und teilweise identisch mit denen, die auch in den vorgenannten erfindungsgemäßen Shampoos enthalten sein können.

Je nach Anwendungsgebiet können die Haarpflegemittel als Spray, Schaum, Gel, Gelspray, Creme, Lotion oder Wachs appliziert werden.

Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten.

Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Konditionierungsmittel

Bevorzugte erfindungsgemäße Haarpflegemittel enthalten außer den Polyurethanen PU Konditionierungsmittel.

Erfindungsgemäß bevorzugte Konditionierungsmittel sind beispielsweise die im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humectant, Skin-Conditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen.

Vorteilhafte Konditionierungsstoffe stellen beispielsweise die nach INCI als Polyquaternium bezeichneten Verbindungen dar (insbesondere Polyquaternium-1 bis Polyquaternium-87) und sind zuvor bereits tabellarisch aufgeführt.

Zu den geeigneten Konditionierungsmitteln zählen beispielsweise auch polymere quaternäre Ammoniumverbindungen, kationische Cellulosederivate, Stärkederivate, Maltodextrinderivate und Polysaccharidderivate ebenso wie quaternäre Proteinhydrolysate und quaternäre Siliconderivate.

Weitere erfindungsgemäß vorteilhafte Konditionierer stellen Cellulosederivate, insbesondere Polyquaternium-10 und Polyquaternium-67 (z.B. Ucare®Polymer Marken, SoftCAT®Polymer Marken (Dow Chemical)) und quaternisierte Guargum Derivate, insbesondere Guar Hydroxypropylammoniumchlorid (z.B. Jaguar Excel®, Jaguar C-14S oder C-13S, Jaguar C 162® (Rhodia), CAS 65497-29-2, CAS 39421-75-5) dar. Auch nichtionische Poly-N-vinylpyrrolidon/Polyvinylacetat-Copolymere (z.B. Lu-viskol®VA 64 (BASF)), anionische Acrylat-Copolymere (z.B. Luviflex®Soft (BASF)), und/oder amphotere Amid/Acrylat/Methacrylat Copolymere (z.B. Amphomer® (National Starch)) können erfindungsgemäß vorteilhaft als Konditionierer eingesetzt werden.

Ein bevorzugter Gegenstand der vorliegenden Erfindung sind solche Haarpflegemittel, die als transparente Gele vorliegen und, bezogen auf die gesamte Zubereitung, wenigstens 0,1 Gew.-%, bevorzugt wenigstens 0,2 Gew.-% und besonders bevorzugt wenigstens 0,5 Gew.-% Elektrolyte enthalten. Die erfindungsgemäße Verwendung der Polyurethane PU als Verdickungsmittel ermöglicht die Herstellung transparenter, stabiler Gele mit einer auf die gesamte Zubereitung bezogenen Elektrolytkonzentration von wenigstens 0,1 Gew.-%, bevorzugt wenigstens 0,2 Gew.-%, besonders bevorzugt wenigstens 0,5 Gew.-% und höchstens 10 Gew.-%., bevorzugt 5 Gew.-%., besonders bevorzugt 1 Gew.-%.

Solche stabilen, transparenten Gele und Conditioner lassen sich mit herkömmlichen Verdickern nicht bereitstellen.

Bevorzugt enthalten solche Gele und Conditioner je nach Anwendungsgebiet weitere Inhaltsstoffe. Geeignete weitere Inhaltsstoffe sind dem Fachmann bekannt und zuvor ausführlich beschrieben.

Saure Zubereitungen

Zahlreiche kosmetische Zubereitungen umfassen Wirkstoffe, die ihre gewünschte Wirkung insbesondere bei sauren pH-Werten entfalten. Dazu gehören beispielsweise Zubereitungen, die alpha-Hydroxycarbonsäuren (AHA) und beta-Hydroxycarbonsäuren (BHA) umfassen, da diese in neutralisiertem Zustand wenig bis nicht wirksam sind.

Mit herkömmlichen Verdickern ist es nicht oder nur unter Schwierigkeiten möglich, solche Zubereitungen derartl zu verdicken, dass die Zubereitung über längere Zeit stabil ist.

Erfindungsgemäß sind demnach kosmetische und dermatologische Zubereitungen, die neben dem Polyurethan PU wenigstens einen Wirkstoff enthalten, der seine kosmetische und/oder dermatologische Wirksamkeit bei sauren pH-Werten, also im Bereich von 1 bis kleiner als 7 entfaltet.

Bevorzugte Wirkstoffe dieser Art sind die alpha-Hydroxycarbonsäuren und beta-Hydroxycarbonsäuren . Bevorzugt sind solche Zubereitungen, die einen pH-Wert im Bereich von 3 bis 6, besonders bevorzugt im Bereich von 4 bis 5,5 aufweisen.

Zur Behandlung von Alterserscheinungen der Haut, Verhornungsstörungen, Lichtschäden und Akne werden in der Dermatologie immer häufiger oberflächliche und mitteltiefe Peelingmethoden angewendet. Am häufigsten wird das Oberflächenpeeling mit alpha-Hydroxycarbonsäuren, auch Fruchtsäurepeeling genannt, durchgeführt. Glycolsäure hat sich als wichtigste Substanz in der dermatologischen Praxis bewährt.
alpha-Hydroxycarbonsäuren wirken auf die Haut sowohl epidermal als auch dermal. Sie beeinflussen die Korneozytenkohäsion; so wird altes oder totes Zellmaterial gelöst und die epidermale Hornschicht ausgedünnt. Die gleichzeitige Steigerung des Zellturnover führt zur Verdickung der Epidermis unter dem Stratum corneum. Ein weiterer epidermaler Effekt ist die erhöhte Glykosaminoglykansynthese und eine dadurch bedingte bessere Hydratation der Haut. Als wichtige dermale Wirkung der alpha-Hydroxycarbonsäuren wird die Neubildung von elastischen und kollagenen Fasern diskutiert, wobei der Nachweis bisher vor allem für Glykolsäure gelang. Glykolsäure und andere alpha-Hydroxycarbonsäuren sind vor allem bei Ichthyosen, Hyperkeratosen, Akne und vulgären Warzen indiziert. Für den therapeutischen Einsatz werden niedrig (5 bis 15 Prozent) und hoch konzentrierte (über 50 Prozent) Zubereitungen unterschieden. Niedrig konzentrierte Lösungen und Gele können sorgfältig eingewiesene Patienten selbst applizieren, während die Behandlung mit höher konzentrierten Zubereitungen durch den Arzt erfolgen sollte. Für dieses eigentliche Fruchtsäurepeeling werden meistens Glykolsäurelösungen verwendet, deren Konzentrationen im Laufe der Behandlungen auf bis zu 70 Prozent steigen können.

Eine Ausführungsform der Erfindung sind Peeling-Zubereitungen enthaltend wenigstens ein Polyurethan PU und wenigstens eine alpha-Hydroxycarbonsäure.

Die alpha-Hydroxysäuren sind vorzugsweise ausgewählt aus der Gruppe bestehend aus Milchsäure, Glycolsäure, Äpfelsäure, Tartronsäure, Weinsäure, Glucuronsäure, Brenztraubensäure, 2-Hydroxyisobuttersäure, 3-Hydroxyisobuttersäure, Zitronensäure, Galacturonsäure, Mandelsäure, Schleimsäure, beta-Phenylmilchsäure, beta-Phenylbrenztraubensäure, Saccharinsäure, alpha-Hydroxybuttersäure, alpha-Hydroxyisobuttersäure, alpha-Hydroxyisocapronsäure, alpha-Hydroxyisovaleriansäure, Atrolactinsäure, Galactansäure, Pantosäure, Glycerinsäure, Isocitronensäure, Dihydroxymaleinsäure, Dihydroxyweinsäure, Dihydroxyfumarsäure, Benzylameisensäure, wobei Milchsäure und Glycolsäure besonders bevorzugt sind. In einer Ausführungsform der Erfindung verwendet man eine Kombination von zwei Säuren. Geeignete beta-Hydroxycarbonsäuren sind beispielsweise Salicylsäure und D- und L-Carnitin.

Weiterhin können Ester der alpha-Hydroxysäuren verwendet werden, deren besondere Wirkung darin besteht, dass sie die alpha-Hydroxysäuren langsamer in der Haut freisetzen. Zu diesen Estern zählen insbesondere die unter den Handelsnamen Cosmacol ETL® (Di-C14-C15- alkyltartrat), Cosmacol ECL® (Tri-C14-C15-alkylcitrat), Cosmacol ELI® (C12-C13- Alkyllaktat), Cosmacol FOI® (C12-C13-Alkyloctanoat), Cosmacol EMI® (Di-C12-C13- alkylmalat), Cosmacol ECI® (Tri-C12-C13-alkylcitrat) sowie Cosmacol ETI® (Di-C12-C13-alkyltartrat) kommerziell erhältlichen Ester. Die Dosierungen dieser Ester, allein oder als Mischung, liegen im Bereich von 2 bis 15 %, vorzugsweise 4 bis 10%, des Gesamtgewichts der Zubereitung.

Zubereitungen für Mundhygiene und Zahnpflege

Eine weitere Ausführungsform der Erfindung sind Zubereitungen zur Mund- und Zahnpflege- und -reinigung, enthaltend wenigstens ein Polyurethan PU. Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.
Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern oder Mundwässern vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.
In einer bevorzugten Ausführungsform der Erfindung enthalten die Zahnpflegezubereitungen nanopartikuläre Calciumsalze, insbesondere Hydroxylapatit, Fluorapatit und/oder Calciumfluorid. Solche nanopartikulären Calciumsalze und deren Herstellung sind beispielsweise beschrieben in DE 10 2006 009 780 A1, EP 934 449, EP-B 1023035, EP 1139995 und insbesondere in WO 03/000588.
Als weiterer Inhaltsstoff der Zubereitungen sind gegebenenfalls wasserlösliche Tenside und/oder wasserlösliche polymere Schutzkolloide enthalten. Das mindestens eine wasserlösliche Tensid und/oder das mindestens eine wasserlösliche polymere Schutzkolloid ist, bezogen auf die Zubereitung, im Bereich von 0,01 bis 15 Gew.-%, vorzugsweise von 0,1 bis 10 Gew.-% und insbesondere von 0,5 bis 7,5 Gew.-% enthalten.
Geeignete anionische Tenside sind in DE 10 2006 009 780 A1, Absätze [0052] und [0053], geeignete zwitterionische Tenside ebenda in Absatz [0054], geeignete ampholytische Tenside ebenda in Absatz [0055], geeignete nichttionische Tenside ebenda in Absätzen [0056] bis [0061] und geeignete kationische Tenside ebenda in Absätzen [0062] bis [0065] beschrieben, worauf hiermit in vollem Umfang Bezug genommen wird.
Geeignete wasserlösliche polymere Schutzkolloide sind in DE 10 2006 009 780 A1, Absätze [0066] bis [0099] beschrieben, worauf hiermit in vollem Umfang Bezug genommen wird.
Die erfindungsgemäßen Zubereitungen können weiterhin Proteinkomponenten wie beispielsweise Proteinhydrolysate enthalten. Erfindungsgemäß geeignete Proteinkomponenten sind in DE 10 2006 009 780 A1, Absätze [0115] bis [0123] beschrieben, worauf hiermit in vollem Umfang Bezug genommen wird.
Die erfindungsgemäßen Mund- und Zahnpflege- und -reinigungsmittel können zusätzlich zu den vorstehend genannten Inhaltsstoffen weitere Inhaltsstoffe von Mundreinigungsmitteln, Mundpflegemitteln, Zahnreinigungsmitteln und/oder Zahnpflegemitteln enthalten. Die bevorzugten weiteren Inhaltsstoffe sind antimikrobielle Stoffe als Konservierungsmittel oder als Antiplaque-Wirkstoffe, Antikaries-Wirkstoffe, gegen Zahnstein wirksame Substanzen, Poliermittel, Putzkörper, Feuchthaltemittel, zusätzliche Konsistenzregler, die Unempfindlichkeit der Zähne steigernde Substanzen, wundheilende und entzündungshemmende Stoffe, Substanzen zur Erhöhung des mineralisierenden Potentials, Aromaöle, Süßungsmittel, Lösungsmittel und Lösungsvermittler, Pigmente, wie z.B. Titandioxid, Farbstoffe, Puffersubstanzen, Vitamine, Mineralsalze und bioaktive Gläser. Unter Wirkstoffen werden auch die Milchsäurebakterien des Lactobacillus anti-caries verstanden, die verhindern, dass sich schädliche Bakterien auf der Zahnoberfläche festsetzen und die Zähne schädigen. Bevorzugte weitere Inhaltsstoffe sind in DE 10 2006 009 780 A1, Absätze [0132] bis [0182] beschrieben, worauf hiermit in vollem Umfang Bezug genommen wird.
Die Polyurethane PU enthaltenden Zubereitungen zur Mund- und Zahnpflege- und - reinigung zeichnen sich dadurch aus, dass sich ihre Viskosität (Konsistenz) auch bei Anwesenheit von Elektrolyten und/oder Pigmenten sowie Fluorid aufbauen lässt und langzeitstabil ist.

Zubereitungen für die Haarentfernung

Eine weitere Ausführungsform der Erfindung sind Zubereitungen für die Haarentfernung enthaltend ein Polyurethan PU. Zubereitungen für die Haarentfernung werden insbesondere als Depiliercremes, Depilierlotionen oder Depilierschäume bereitgestellt. Die Wirkungsweise beruht darauf, dass mit Reduktionsmitteln im alkalischen Bereich Peptidbindungen und Dislufidbrücken des Haarkeratins gespalten werden, so dass die Haare vollständig abgetrennt werden. Die wichtigsten Haarentfernungsmittel sind Thioglykolsäure und Thiomilchsäure. Herkömmliche Verdicker erlauben es in nur unzureichendem Maße, die Thioglykolsäure, Thiomilchsäure und andere basische Haarentfernungsmittel bei den notwendigen alkalischen pH-Werten stabil zu formulieren. So ist Xanthan Gum besitzt zwar eine in diesem pH-Bereich verdickende Wirkung, jedoch weisen die derart verdickten Produkte eine unerwünschte, schleimige Konsistenz auf. Zubereitungen, die mit üblichen Acrylatverdickern verdickt wurden, zeigen keine stabile Konsistenz.

Im Gegensatz dazu lassen sich typische Zubereitungen für die Haarentfernung mit Hilfe der Polyurethane PU derart verdicken, dass die Konsistenz stabil und kosmetisch akzeptabel ist.

Zubereitungen für die dauerhafte Haarverformung

Eine weitere Ausführungsform der Erfindung sind Zubereitungen für die dauerhafte Haarverformung enthaltend ein Polyurethan PU. Die klassische Technik zur Durchführung der dauerhaften Haarverformung besteht darin, dass in einer ersten Stufe die Disulfid-Bindungen des Haarkeratins mit Hilfe eines Mittels, welches einen reduzierenden Wirkstoff enthält (Verformungsmittel), geöffnet werden, sodann das Haar in die gewünschte Form gebracht wird und anschließend die Disulfid-Bindungen unter Verwendung eines einen oxidierenden Wirkstoff enthaltenden Mittels (Fixiermittel) wieder verknüpft werden.

Typisch für Zubereitungen für die dauerhafte Haarverformung sind hohe Elektrolytkonzentrationen, oxidative- oder reduktive Milieus sowie drastische pH-Bedingungen. Übliche Verdickungsmittel führen nicht zur gewünschten stabilen verdickten Konsistenz. Durch die Verwendung der Polyurethane PU wird dies erreicht.

Als reduzierende Wirkstoffe in den Zubereitungen für die dauerhafte Haarverformung werden insbesondere Sulfite, Thioglykolsäure, Thiomilchsäure, 3-Mercaptopropionsäure, Mercaptocarbonsäureester und Cystein verwendet. Diese Mittel sind entweder sauer (Sulfit, Bisulfit und Mercaptocarbonsäureester) oder alkalisch (Alkali- und Ammoniumsalze von Mercaptocarbonsäuren) eingestellt. Im Falle von alkalisch eingestellten Verformungsmitteln wird die erforderliche Alkalität vor allem durch Zusatz von Ammoniak, organischen Aminen, Ammonium- oder Alkalicarbonat und Ammonium- oder Alkalihydrogencarbonat erreicht. Als Fixiermittel werden insbesondere wasserstoffperoxidhaltige oder bromathaltige Flüssigkeiten verwendet.

Die erfindungsgemäße Zubereitung für die dauerhafte Haarverformung enthält die keratinreduzierenden Verbindungen in den für die Haarverformung üblichen Mengen, beispielsweise die Ammoniumsalze der Thioglykolsäure oder Thiomilchsäure oder aber Cystein in einer Konzentration von 6 bis 12 Gewichtsprozent. Der pH-Wert der alkalischen Verformungsmittel liegt im allgemeinen bei 7 bis 10, wobei die Einstellung vorzugsweise mit Ammoniak, Monoethanolamin, Ammoniumcarbonat oder Ammoniumhydrogencarbonat erfolgt.

Ist die Zubereitung sauer (zum Beispiel auf pH = 6,5 bis 6,9) eingestellt, werden Ester von Mercaptocarbonsäuren, wie beispielsweise Monothioglykolsäureglykolester oder -glycerinester, vorzugsweise aber Mercaptoacetamide oder 2-Mercaptopropionsäureamide, in einer Konzentration von 2 bis 14 Gew.-%, bezogen auf die Zubereitung, oder aber die Salze der schwefeligen Säure, zum Beispiel Natrium-, Ammonium- oder Monoethanolammoniumsulfit, in einer Konzentration von 3 bis 8 Gew.-%, bezogen auf die Zubereitung (berechnet als $SO_2$), verwendet. Bevorzugt ist die verwendete haarkeratinreduzierende Verbindung das Salz oder das Derivat einer Mercaptocarbonsäure. Besonders bevorzugt ist die keratinreduzierende Verbindung ausgewählt aus Thioglykolsäure, Cystein und Thiomilchsäure oder deren Salzen.

Zur Wirkungssteigerung können der Zubereitung Quell- und Penetrationsstoffe, wie beispielsweise Harnstoff, mehrwertige Alkohole, Ether, Melamin, Alkali- oder Ammoniumthiocyanat, Isopropanol, Imidazolidin-2-on, 2-Pyrrolidon und 1-Methyl-2-pyrrolidon, in einer Konzentration von etwa 0,5 bis 50 Gewichtsprozent, vorzugsweise 2 bis 30 Gewichtsprozent (bezogen auf die Zubereitung) zugesetzt werden. Vorteilhaft enthält die Zubereitung zusätzlich das Disulfid einer haarkeratinreduzierenden Verbindung, insbesondere Dithioglykolat. Die bevorzugte Einsatzmenge ist 2 bis 20 Gew.%, vorzugsweise 3 bis 10 Gew.%, jeweils bezogen auf die Zubereitung, wobei ein Gewichts-Verhältnis zwischen haarkeratinreduzierender Verbindung und dem Disulfid von 2:1 bis 1:2, insbesondere 2:1 bis 1:1, bevorzugt ist.

Die erfindungsgemäßen Zubereitungen können in Form einer wässrigen Lösung oder einer Emulsion sowie in verdickter Form auf wässriger Basis, insbesondere als Creme, Gel oder Paste, vorliegen.

Selbstverständlich können die erfindungsgemäßen Zubereitungen alle für derartige Mittel üblichen und bekannten Zusatzstoffe, zum Beispiel weitere Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Haarkonditionierungsmittel sowie haarpflegende Bestandteile, wie zum Beispiel kationische Polymere, Lanolinderivate, Cholesterin, Pantothensäure, Kreatin oder Betain, enthalten.

Geeignete kationische Polymere sind in der DE 10 2004 054 055 A1, Absätze [0036] bis [0044] beschrieben, worauf an dieser Stelle in vollem Umfang Bezug genommen wird. Weitere geeignete kationaktive haarpflegende Verbindungen, die in den erfindungsgemäßen Zubereitungen enthalten sein können, sind kationisch modifizierte Proteinderivate oder kationisch modifizierte Proteinhydrolysate und sind in DE 10 2004 054 055 A1, Absätze [0045] bis [0046] beschrieben, worauf an dieser Stelle in vollem Umfang Bezug genommen wird.

Die Polyurethane PU zeichnen sich auch dadurch aus, dass sie in den erfindungsgemäßen Zubereitungen eine reversible Viskositätserniedrigung bei höheren Temperaturen ermöglichen. Dadurch können Zubereitungen nach ihrer Herstellung bei höheren Temperaturen einfacher und schneller konfektioniert werden.

Die Erfindung wird anhand der folgenden nicht einschränkenden Beispiele näher erläutert.

Beispiele

Soweit nichts anderes angegeben ist, beziehen sich alle Prozentangaben auf Gewichtsprozent.

Bestimmung der dynamischen Viskosität

Die dynamischen Viskositäten der erfindungsgemäß verwendeten Polyurethane PU in wässriger Dispersion wurden in Form einer 10 gewichtsprozentigen Dispersion bei 23°C gemessen. In den unten aufgeführten Beispielen wurde dazu stets die dynamische Viskosität bei Schergeschwindigkeiten von 100 1/s und 350 1/s bestimmt. Diese beiden Werte erlauben eine Aussage zu treffen, ob die erfindungsgemäß verwendeten Polyurethane PU in wässriger Dispersion strukturviskoses oder newtonsches Verdickerverhalten zeigen.

Zur Bestimmung der dynamischen Viskosität nach DIN53019 wurde verwendet:

- Verwendetes Gerät: Rotationsviskosimeter Physica Rheolab MCI Portable von der Firma Anton Paar;
- Zylinder Messsystem, Z4 DIN Zylinder (Durchmesser 14 mm)
- Verwendetes Gerät: Rotationsviskosimeter Physica Rheolab MCI Portable von der Firma Anton Paar;
- Zylinder Messsystem, Z4 DIN Zylinder (Durchmesser 14 mm)

Synthesebeispiel 1: Herstellung von Polyurethanen PU.1

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 140 ppm betrug.

Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern. Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einem Isocyanat-Gehalt von 0,27 Gew.-% reagieren gelassen.

Dann wurde ein Gemisch aus 1,42 kg eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO10 von der Firma BASF SE), und 1,64 kg eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.1 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.1 typischerweise 1 zu 12,4 bzw. 1 zu 13,6. Das letztere Verhältnis ergibt sich für Abschnitte S, die aus 10 Ethylenoxidresten bestehen, ersteres für solche, die aus 11 Ethylenoxidresten aufgebaut sind.

Das molare Verhältnis von Abschnitten P zu D beträgt 1:1,75.

Das Produkt PU.1 wurde in 86,73 kg Wasser dispergiert und auf Raumtemperatur (25 °C) abgekühlt Die Mischung aus Polymeren PU.1 (Mn = 17600 g/mol; Mw = 30500 g/mol) lag in Form einer wässrigen Dispersion vor, welche einen Feststoffgehalt von 20,5 Gew.-% aufwies. Die Viskosität einer 10 gewichtsprozentigen wässrigen Dispersion der Polyetherpolyurethane PU.1 bei 23 °C betrug 7700 mPa*s (Scherrate 100 1/s) bzw. 5900 mPa*s (Scherrate 350 1/s) und zeigte schwach strukturviskoses Verhalten.

Synthesebeispiel 2: Herstellung von Polyurethanen PU.2

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 250 ppm betrug.

Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern.

Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einen Isocyanat-Gehalt von 0,29 Gew.-% reagieren gelassen.

Dann wurde ein Gemisch aus 0,95 kg eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO10 von der Firma BASF SE), und 2,19 kg eines nicht-ionischen ethoxylierten Fettalkohols, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt und die Reaktionsmischung solange weiter auf 50 °C erhitzt, bis der Isocyanat-Gehalt 0 Gew.-% betrug.

Das Lösungsmittel Xylol wurde nachfolgend durch Vakuumdestillation bei erhöhter Temperatur bis zu einem Restgehalt von unter 500 ppm entfernt.

Das entstandene Produkt PU.2 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.2 typischerweise 1 zu 12,4 bzw. 1 zu 13,6. Das letztere Verhältnis ergibt sich für Abschnitte S, die aus 10 Ethylenoxidresten bestehen, ersteres für solche, die aus 11 Ethylenoxidresten aufgebaut sind.

Das molare Verhältnis von Abschnitten P zu D beträgt 1:1,75.

Das Produkt PU.2 wurde in 87,02 kg Wasser dispergiert und auf Raumtemperatur (25 °C) abgekühlt. Die Polymermischung PU.2 (Mn = 16700 g/mol; Mw = 29500 g/mol) lag in Form einer wässrigen Dispersion vor, welche einen Feststoffgehalt von 20,0 Gew.-% aufwies. Die Viskosität einer 10 gewichtsprozentigen wässrigen Dispersion der Polyetherpolyurethane PU.2 bei 23 °C betrug 26200 mPa*s (Scherrate 100 1/s) bzw. 12800 mPa*s (Scherrate 350 1/s) und zeigte ausgeprägt strukturviskoses Verhalten.

Synthesebeispiel 3: Herstellung von Polyurethanen PU.3

120,00 g eines linearen Polyethylenglykols mit einem zahlenmittleren Molekulargewicht von 6000 g/mol (z.B. Pluriol® E6000 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend weniger als 300 ppm betrug.

Nun wurde die Polymerlösung auf 50 °C abgekühlt. Durch Zugabe von 42 mg Zink-neodecanoat, gelöst in einem Gemisch aus aliphatischen Kohlenwasserstoffen, und 5,88 g Hexamethylendiisocyanat, gelöst in Xylol, wurde die Polymerisation gestartet und der Ansatz bei 50 °C bis zu einen Isocyanat-Gehalt von 0,25 Gew.-% reagieren gelassen.

Dann wurden 19,20 g eines nicht-ionischen ethoxylierten Fettalkohols, hergestellt aus einem gesättigten iso-C13 Alkohol und einem durchschnittlichen Ethoxylierungsgrad von 10 (z.B. Lutensol® TO10 von der Firma BASF SE), gelöst in Xylol, hinzugefügt und die Reaktionsmischung solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.3 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.3 typischerweise 1 zu 13,6. Dieses Verhältnis ergibt sich für Abschnitte S, die aus 10 Ethylenoxidresten bestehen.

Das molare Verhältnis von Abschnitten P zu D beträgt 1:1,75.

Das Produkt PU.3 wurde in 580,3 g Wasser dispergiert und auf Raumtemperatur (25 °C) abgekühlt. Die Polymermischung PU.3 (Mn = 27200 g/mol; Mw, = 51900 g/mol) lag in Form einer wässrigen Dispersion vor, welche einen Feststoffgehalt von 20,0 % aufwies. Die Viskosität einer 10 gewichtsprozentigen wässrigen Dispersion der Polyetherpolyurethane PU.3 bei 23 °C betrug 680 mPa*s (Scherrate 100 1/s) bzw. 640 mPa*s (Scherrate 350 1/s) und zeigte newtonsches Verdickerverhalten.

Synthesebeispiel 4: Herstellung von Polyurethanen PU.4

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca.140 °C wurde Xylol abdestilliert, so dass der Wassergehalt des Reaktionsansatzes anschließend ca. 120 ppm betrug.

Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern.

Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einen Isocyanat-Gehalt von 0,26 Gew.-% reagieren gelassen.

Dann wurden 2,84 kg eines nicht-ionischen ethoxylierten Fettalkohols, hergestellt aus einem gesättigten iso-C13 Alkohol und einem durchschnittlichen Ethoxylierungsgrad von 10 (z.B. Lutensol® TO10 von der Firma BASF SE), gelöst in Xylol, hinzugefügt und die Reaktionsmischung solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.4 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.4 typischerweise 1 zu 13,6. Dieses Verhältnis ergibt sich für Abschnitte S, die aus 10 Ethylenoxidresten bestehen.

Das molare Verhältnis von Abschnitten P zu D beträgt 1:1,75.

Das Produkt PU.4 wurde in 85,84 kg Wasser dispergiert und auf Raumtemperatur (25 °C) abgekühlt. Die Polymermischung PU.4 (Mn = 19200 g/mol; Mw, = 30800 g/mol) lag in Form einer wässrigen Dispersion vor, welche einen Feststoffgehalt von 18,1 % aufwies. Die Viskosität einer 10 gewichtsprozentigen wässrigen Dispersion der Polyetherpolyurethane PU.4 bei 23 °C betrug 600 mPa*s (Scherrate 100 1/s) bzw. 570 mPa*s (Scherrate 350 1/s) und zeigte newtonsches Verdickerverhalten.

Synthesebeispiel 5: Herstellung von Polyurethanen PU.5

240,00 g eines linearen Polyethylenglykols mit einem Molekulargewicht von 6000 g/mol (z.B. Pluriol® E6000 von BASF SE) wurden in 934,00 g Xylol unter Stickstoff gelöst. Nach dem Erhitzen der Lösung auf ca.140 °C wurde Xylol abdestilliert, so dass der Wassergehalt des Reaktionsansatzes anschließend weniger als 300 ppm betrug.

Nun wurde die Polymerlösung auf 50 °C abgekühlt. Durch Zugabe von 84 mg Zink-neodecanoat, gelöst in aliphatischen Kohlenwasserstoffen, und 11,76 g Hexamethylendiisocyanat, gelöst in Xylol, wurde die Polymerisation gestartet und der Ansatz bei 50 °C bis zu einem Isocyanat-Gehalt von 0,22 Gew.-% reagieren gelassen.

Dann wurden 20,70 g eines nicht-ionischen ethoxylierten Fettalkohols, hergestellt aus einem gesättigten C13 Alkohol und einem durchschnittlichen Ethoxylierungsgrad von 3 (z.B. Lutensol® AO3 von BASF SE), gelöst in Xylol, hinzugefügt und die Reaktionsmischung solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt und der Rückstand anschließend in 1089,8 g Wasser dispergiert.

Das Verhältnis der Molekulargewichte eines hydrophilen Abschnittes S zu dem Molekulargewicht eines hydrophilen Abschnittes P beträgt in den Polyurethanen PU.5 typischerweise 1 zu 45,5. Dieses Verhältnis ergibt sich für die Abschnitte S, die aus 3 Ethylenoxidresten bestehen.

Das molare Verhältnis von Abschnitten P zu D beträgt 1:1,75.

Nach dem Abkühlen auf Raumtemperatur (25 °C) lagen die Polymere PU.5 (Mn = 21300 g/mol; Mw = 36300 g/mol) in Form einer wässrigen Dispersion vor, welche einen Feststoffgehalt von 20,1 Gew.-% aufwies. Die Viskosität einer 10 gewichtsprozentigen wässrigen Dispersion der Polyetherpolyurethane PU.5 bei 23 °C betrug 10900 mPa*s (Scherrate 100 1/s) bzw. 9200 mPa*s (Scherrate 350 1/s) und zeigte schwach strukturviskoses Verhalten.

Synthesebeispiel 6: Herstellung von Polyurethanen PU.6

180,00 g eines linearen Polyethylenglykols mit einem Molekulargewicht von 6000 g/mol (z.B. Pluriol® E6000 von BASF SE) wurden in 180,00 g Aceton unter Stickstoff gelöst. Nach dem Erhitzen der Lösung auf Rückfluss (ca. 56 °C Innentemperatur) wurden kontinuierlich weitere 1362,4 g Aceton zugegeben und gleichzeitig insgesamt 1362,4 g Aceton abdestilliert. Der Wassergehalt des Reaktionsansatzes betrug anschließend nur noch ca. 240 ppm.

Nun wurde die Polymerlösung auf 50 °C abgekühlt. Durch Zugabe von 189 mg Zink-neodecanoat, gelöst in aliphatischen Kohlenwasserstoffen, und 8,82 g Hexamethylendiisocyanat, gelöst in Aceton, wurde die Polymerisation gestartet und der Ansatz bei 50 °C bis zu einem Isocyanat-Gehalt von 0,33 Gew.-% reagieren gelassen. Dann wurden 15,53 g eines nicht-ionischen ethoxylierten Fettalkohols, hergestellt aus einem gesättigten C13 Alkohol und einem durchschnittlichen Ethoxylierungsgrad von 3 (z.B. Lutensol® AO3 von BASFSE), gelöst in Aceton, hinzugefügt und die Reaktionsmischung solange weiter auf 50 °C erhitzt, bis der Isocyanat-Gehalt 0 Gew.-% betrug. Das Lösungsmittel Aceton wurde nachfolgend durch Vakuumdestillation bis zu einem Restgehalt von unter 500 ppm entfernt und der Rückstand in 817,4 g Wasser dispergiert.

Das Verhältnis der Molekulargewichte eines hydrophilen Abschnittes S zu dem Molekulargewicht eines hydrophilen Abschnittes P beträgt in den Polyurethanen PU.6 typischerweise 1 zu 45,5. Dieses Verhältnis ergibt sich für die Abschnitte S, die aus 3 Ethylenoxidresten bestehen.

Das molare Verhältnis von Abschnitten P zu D beträgt 1:1,75.

Nach dem Abkühlen auf Raumtemperatur (25 °C) lagen die Polymere PU.6 (Mn = 24900 g/mol; Mw = 40000 g/mol) in Form einer wässrigen Dispersion vor, welche einen Feststoffgehalt von 19,6 Gew.-% aufwies. Die Viskosität einer 10 gewichtsprozentigen wässrigen Dispersion der Polyetherpolyurethane PU.6 bei 23 °C betrug 8800 mPa*s (Scherrate 100 1/s) bzw. 7800 mPa*s (Scherrate 350 1/s) und zeigte schwach strukturviskoses Verhalten.

Synthesebeispiel 7: Herstellung von Polyurethanen PU.7

120,00 g eines linearen Polyethylenglykols mit einem zahlenmittleren Molekulargewicht von 6000 g/mol (z.B. Pluriol® E6000 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 120 ppm betrug.

Nun wurde die Polymerlösung auf 50 °C abgekühlt und mit 107 mg Essigsäure, gelöst in 5 ml Xylol, versetzt, um die zuvor quantitativ bestimmte Menge an Kaliumacetat im Polyethylenglykol abzupuffern. Durch Zugabe von 252 mg Zink-neodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,25 Gew.-% reagieren gelassen.

Dann wurden 22,20 g eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.7 ist eine Mischung, welche lineare Polyurethane mit randständigen, unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.7 typischerweise 1 zu 12,4. Dieses Verhältnis ergibt sich für Abschnitte S, die aus 11 Ethylenoxidresten bestehen. Das molare Verhältnis von Abschnitten P zu D beträgt 1:1,75.

Das Produkt PU.7 wurde in 592,3 g Wasser dispergiert und auf Raumtemperatur (25 °C) abgekühlt. Die Mischung aus Polymeren PU.7 (Mn = 18700 g/mol; Mw = 30900 g/mol) lag in Form einer wässrigen Dispersion vor, welche einen Feststoffgehalt von 20,4 Gew.-% aufwies. Die Viskosität einer 10 gewichtsprozentigen wässrigen Dispersion der Polyetherpolyurethane PU.7 bei 23 °C betrug 35500 mPa*s (Scherrate 100 1/s) bzw. 14500 mPa*s (Scherrate 350 1/s) und zeigte stark strukturviskoses Verhalten.

Synthesebeispiel 8: Herstellung von Polyurethanen PU.8

180,00 g eines linearen Polyethylenglykols mit einem zahlenmittleren Molekulargewicht von 9000 g/mol (z.B. Pluriol® E9000 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 70 ppm betrug.

Nun wurde die Polymerlösung auf 50 °C abgekühlt und mit 208 mg Essigsäure, gelöst in 5 ml Xylol, versetzt, um die zuvor quantitativ bestimmte Menge an Kaliumacetat im Polyethylenglykol abzupuffern. Durch Zugabe von 378 mg Zink-neodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,27 Gew.-% reagieren gelassen.

Dann wurden 10,20 g eines nicht-ionischen ethoxylierten Fettalkohols, hergestellt aus einem gesättigten iso-C13 Alkohol und einem durchschnittlichen Ethoxylierungsgrad von 3 (z.B. Lutensol® TO3 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.8 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.8 typischerweise 1 zu 68,2. Dieses Verhältnis ergibt sich für Abschnitte S, die aus 3 Ethylenoxidresten bestehen.

Das molare Verhältnis von Abschnitten P zu D beträgt 1:1,75.

Das Produkt PU.8 wurde in 784,3 g Wasser dispergiert und auf Raumtemperatur (25 °C) abgekühlt. Die Mischung aus Polymeren PU.8 (Mn = 27300 g/mol; Mw = 46500 g/mol) lag in Form einer wässrigen Dispersion vor, welche einen Feststoffgehalt von 20,2 Gew.-% aufwies. Die Viskosität einer 10 gewichtsprozentigen wässrigen Dispersion der Polyetherpolyurethane PU.8 bei 23 °C betrug 1060 mPa*s (Scherrate 100 1/s & Scherrate 350 1/s) und zeigte ausgeprägtes, newtonsches Verhalten.

Synthesebeispiel 9: Herstellung von Polyurethanen PU.9

180,00 g eines linearen Polyethylenglykols mit einem zahlenmittleren Molekulargewicht von 9000 g/mol (z.B. Pluriol® E9000 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 70 ppm betrug.

Nun wurde die Polymerlösung auf 50 °C abgekühlt und mit 208 mg Essigsäure, gelöst in 5 ml Xylol, versetzt, um die zuvor quantitativ bestimmte Menge an Kaliumacetat im Polyethylenglykol abzupuffern. Durch Zugabe von 378 mg Zink-neodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,28 Gew.-% reagieren gelassen.

Dann wurde ein Gemisch aus 5,10 g eines nicht-ionischen ethoxylierten Fettalkohols, hergestellt aus einem gesättigten iso- C13 Alkohol und einem durchschnittlichen Ethoxylierungsgrad von 3 (z.B. Lutensol® TO3 von der Firma BASF SE), und 11,10 g eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.9 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.9 typischerweise 1 zu 12,4 oder 1 zu 68,2. Das letztgenannte Verhältnis ergibt sich für Abschnitte S, die aus 3 Ethylenoxidresten bestehen, ersteres für solche, die aus 11 Ethylenoxidresten aufgebaut sind.

Das molare Verhältnis von Abschnitten P zu D beträgt 1:1,75.

Das Produkt PU.9 wurde in 764,0 g Wasser dispergiert und auf Raumtemperatur (25 °C) abgekühlt Die Mischung aus Polymeren PU.9 (Mn = 25000 g/mol; Mw = 45500 g/mol) lag in Form einer wässrigen Dispersion vor, welche einen Feststoffgehalt von 20,8 Gew.-% aufwies. Die Viskosität einer 10 gewichtsprozentigen wässrigen Dispersion der Polyetherpolyurethane PU.9 bei 23 °C betrug 7500 mPa*s (Scherrate 100 1/s) bzw. 4500 mPa*s (Scherrate 350 1/s) und zeigte stark strukturviskoses Verhalten.

Synthesebeispiel 10: Herstellung von Polyurethanen PU.10

120,00 g eines linearen Polyethylenglykols mit einem zahlenmittleren Molekulargewicht von 1500 g/mol (z.B. Pluriol® E1500 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 110 ppm betrug.

Nun wurde die Polymerlösung auf 50 °C abgekühlt und mit 90 mg Essigsäure, gelöst in 5 ml Xylol, versetzt, um die zuvor quantitativ bestimmte Menge an Kaliumacetat im Polyethylenglykol abzupuffern. Durch Zugabe von 252 mg Zink-neodecanoat, gelöst in einem Gemisch aus aliphatischen Kohlenwasserstoffen und Xylol, und 15,72 g Hexamethylendiisocyanat, gelöst in Xylol, wurde die Polymerisation gestartet und der Ansatz bei 50 °C bis zu einem Isocyanat-Gehalt von 0,29 Gew.-% reagieren gelassen.

Dann wurden 17,41 g eines nicht-ionischen ethoxylierten Fettalkohols, hergestellt aus einem gesättigten iso-C13 Alkohol und einem durchschnittlichen Ethoxylierungsgrad von 10 (z.B. Lutensol® TO10 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.10 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.10 typischerweise 1 zu 13,6. Dieses Verhältnis ergibt sich für Abschnitte S, die aus 10 Ethylenoxidresten bestehen.

Das molare Verhältnis von Abschnitten P zu D beträgt 1:1,17.

Das Produkt PU.10 wurde in 612,5 g Wasser dispergiert und auf Raumtemperatur (25 °C) abgekühlt Die Mischung aus Polymeren PU.10 (Mn = 18600 g/mol; Mw = 34900 g/mol) lag in Form einer wässrigen Dispersion vor, welche einen Feststoffgehalt von 20,1 Gew.-% aufwies. Die Viskosität einer 10 gewichtsprozentigen wässrigen Dispersion der Polyetherpolyurethane PU.10 bei 23 °C betrug 165 mPa*s (Scherrate 100 1/s & Scherrate 350 1/s) und zeigte ausgeprägtes, newtonsches Verhalten.

Synthesebeispiel 11: Herstellung von Polyurethanen PU.11

90,00 g eines linearen Polyethylenglykols mit einem zahlenmittleren Molekulargewicht von 1500 g/mol (z.B. Pluriol® E1500 von BASF SE) wurden in 467,00 g Xylol unter Stickstoff gelöst. Nach dem Erhitzen der Lösung auf ca.140 °C wurde Xylol abdestilliert, so dass der Wassergehalt des Reaktionsansatzes anschließend nur noch ca. 80 ppm betrug.

Nun wurde die Polymerlösung auf 50 °C abgekühlt und mit 68 mg Essigsäure, gelöst in 5 ml Xylol, versetzt, um die zuvor quantitativ bestimmte Menge an Kaliumacetat im Polyethylenglykol abzupuffern. Durch Zugabe von 189 mg Zink-neodecanoat, gelöst in einem Gemisch aus aliphatischen Kohlenwasserstoffen und Xylol, und 17,64 g Hexamethylendiisocyanat, gelöst in Xylol, wurde die Polymerisation gestartet und der Ansatz bei 50 °C bis zu einem Isocyanat-Gehalt von 0,97 Gew.-% reagieren gelassen.

Dann wurden 99,00 g eines nicht-ionischen ethoxylierten Fettalkohols, hergestellt aus einem gesättigten iso-C13 Alkohol und einem durchschnittlichen Ethoxylierungsgrad von 20 (z.B. Lutensol® TO20 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.11 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.11 typischerweise 1 zu 1,7. Dieses Verhältnis ergibt sich für Abschnitte S, die aus 20 Ethylenoxidresten bestehen.

Das molare Verhältnis von Abschnitten P zu D beträgt 1:1,75.

Das Produkt PU.11 wurde in 826,6 g Wasser dispergiert und auf Raumtemperatur (25 °C) abgekühlt Die Mischung aus Polymeren PU.11 (Mn = 4000 g/mol; Mw = 9000 g/mol) lag in Form einer wässrigen Dispersion vor, welche einen Feststoffgehalt von 20,0 Gew.-% aufwies. Die Viskosität einer 10 gewichtsprozentigen wässrigen Dispersion der Polyetherpolyurethane PU.11 bei 23 °C betrug 150 mPa*s (Scherrate 100 1/s & Scherrate 350 1/s) und zeigte ausgeprägtes, strukturviskoses Verhalten.

Bestimmung der kritischen Mizellenkonzentration

Die CMC der erfindungsgemäß verwendeten Polyurethane in Wasser wurde mit der Methode der dynamischen Lichtstreuung ermittelt.

Hierzu wurde ein Goniometer SP‑86 (ALV-Laser Vertriebsgesellschaft mbH, Langen, Germany) als kombinierte DLS/SLS Anlage verwendet. Die Anlage umfasste auch ein ALV 5000 Korrelator und einen He-Ne Laser der Wellenlänge 633 nm (beide ebenfalls ALV, Langen). Die verwendeten Bedingungen für die Messreihen umfassend Konzentrationen von 0,0001 g/L bis 10 g/L waren ein Messwinkel von 90° bei einer Temperatur von 23°C. Die Auswertung erfolgte mithilfe des im Stand der Technik bekannten Programms CONTIN (Constrained Inversion) mit Intensitätsverteilung (CONTIN ebenfalls von ALV, Langen).

Vergleichsbeispiel:

Ein nicht-ionisches, hydrophob modifiziertes, ethoxyliertes Polyurethan des Standes der Technik, das aus Stearylalkohol, einem Diisocyanat und einem Polyethylenglykol hergestellt wird (vertrieben von der Firma Rohm & Haas als Aculyn® 46) wurde zur Bestimmung der CMC im Vergleich eingesetzt. Aculyn® 46 wies keine messbare CMC auf. Bei Konzentrationen von 0,001 bis 10 g/L lagen als Hauptkomponente stets größere undefinierte Aggregate im Bereich 100 bis 500 nm vor.

CMC der Polyurethane der vorliegenden Erfindung:

Für die in Synthesebeispiel 1 und 2 hergestellten Mischungen von Polyurethanen PU.1 sowie PU.2 wurde gefunden, dass bei 0,1 g/L definierte Mizellen mit mittleren Teilchendurchmessern von 30 nm vorlagen. Die CMC betrug daher für beide kleiner 0,1 g/L. Für die in Synthesebeispiel 4 hergestellten erfindungsgemäß verwendeten Polyurethane PU.4 wurde gefunden, dass bei einer Konzentration von PU.4 von 1 g/L Mizellen mit Durchmesser 17 nm vorlagen und bei einer Konzentration von 0,1 g/L sowohl Mizellen einer mittleren Größe von 15 nm als auch ein geringer Anteil undefinierte Aggregate einer Größe von ungefähr. 200 nm nebeneinander existierten. Daher lag auch in diesem Fall eine CMC von <0,1 g/L vor.

Zubereitungsbeispiel 1: Herstellung kosmetischer Zubereitungen unter Verwendung der Polyurethane PU.1. bis PU.5 mit einer nicht-ionischen Grundlage (Z.1.1 bis Z.1.5)

Die kosmetischen Zubereitungen wurden durch Zugabe der Wasserphase B zur Ölphase A und anschließendem Versetzen der erhaltenen O/W-Emulsion mit dem Konservierungsmittel (Phase C) hergestellt. Man erhielt die auf einer nicht-ionischen Grundlage basierenden Zubereitungen Z.1.1 bis Z.1.5. (Tab. 1).

Tabelle 1. Zusammensetzung der auf einer nicht-ionischen Grundlage basierenden kosmetischen Zubereitungen Z. 1.1 bis Z.1.5.

| Phase | Inhaltsstoffe | Z.1.1 | Z.1.2 | Z.1.3 | Z.1.4 | Z.1.5 |
|---|---|---|---|---|---|---|
| Phase A | Ceteareth-6, Stearyl Alkohol | 2,0 g | 2,0 g | 2,0 g | 2,0 g | 2,0 g |
| | Ceteareth-25 | 2,0 g | 2,0 g | 2,0 g | 2,0 g | 2,0 g |
| | Cetearyl Alkohol | 2,5 g | 2,5 g | 2,5 g | 2,5 g | 2,5 g |
| | Paraffinöl | 5,0 g | 5,0 g | 5,0 g | 5,0 g | 5,0 g |
| | Cetearyl Ethylhexanoat | 5,0 g | 5,0 g | 5,0 g | 5,0 g | 5,0 g |
| Phase B | PU | PU.1 0,5 g | PU.2 0,5 g | PU.3 2,0 g | PU.4 2,0 g | PU.5 0,5 g |
| | 1,2-Propylenglykol | 5,0 g | 5,0 g | 5,0 g | 5,0 g | 5,0 g |
| | Wasser | 77,5 g | 77,5 g | 76,0 g | 76,0 g | 77,5 g |
| Phase C | Konservierungsmittel Euxyl® K300 (Phenoxyethanol, Methylparaben, Ethylparaben, Butylparaben, Propylparaben, Isobutylparaben), kommerziell erhältlich von Schülke&Mayr | 0,5 g | 0,5 g | 0,5 g | 0,5 g | 0,5 g |

Zubereitungsbeispiel 2: Herstellung kosmetischer Zubereitungen unter Verwendung der Polyurethane PU.1. bis PU.5; nicht-ionische Grundlage (Z.2.1 bis Z.2.5)

Die kosmetischen Zubereitungen wurden durch Zugabe der Wasserphase B zur Ölphase A und anschließendem Versetzen der erhaltenen O/W-Emulsion mit dem Konservierungsmittel (Phase C) hergestellt. Man erhielt die auf einer nicht-ionischen Grundlage basierenden Zubereitungen Z.2.1 - Z.2.5. (Tab. 2).

Tabelle 2. Zusammensetzung der auf einer nicht-ionischen Grundlage basierenden kosmetischen Zubereitungen Z. 2.1 - Z.2.5.

| Phase | Inhaltsstoffe | Z.2.1 | Z.2.2 | Z.2.3 | Z.2.4 | Z.2.5 |
|---|---|---|---|---|---|---|
| Phase A | Glyceryl Stearat | 2,0 g | 2,0 g | 2,0 g | 2,0 g | 2,0 g |
| | Stearyl Alkohol | 2,0 g | 2,0 g | 2,0 g | 2,0 g | 2,0 g |
| | Cyclopentasiloxan, Cyclohexasiloxan | 3,0 g | 3,0 g | 3,0 g | 3,0 g | 3,0 g |
| | Dicaprylyl Ether | 3,0 g | 3,0 g | 3,0 g | 3,0 g | 3,0 g |
| | Dimethicon | 2,0 g | 2,0 g | 2,0 g | 2,0 g | 2,0 g |
| | Aluminum Stärke Octenylsuccinat | 1,0 g | 1,0 g | 1,0 g | 1,0 g | 1,0 g |
| | PEG-40 Stearat | 2,0 g | 2,0 g | 2,0 g | 2,0 g | 2,0 g |

(fortgesetzt)

| Phase | Inhaltsstoffe | Z.2.1 | Z.2.2 | Z.2.3 | Z.2.4 | Z.2.5 |
|---|---|---|---|---|---|---|
| Phase B | PU | PU.1 0,5 g | PU.2 0,5 g | PU.3 2,0 g | PU.4 2,0 g | PU.5 0,5 g |
| | Glycerin | 5,0 g | 5,0 g | 5,0 g | 5,0 g | 5,0 g |
| | Wasser | 79,0 g | 79,0 g | 77,5 g | 77,5 g | 79,0 g |
| Phase C | Konservierungsmittel Euxyl® K300 (Phenoxyethanol, Methylparaben, Ethylparaben, Butylparaben, Propylparaben, Isobutylparaben), kommerziell erhältlich von Schülke&Mayr | 0,5 g | 0,5 g | 0,5 g | 0,5 g | 0,5 g |

Bestimmung der dynamischen Viskosität von Zubereitungen mit Hilfsstoffen Die dynamische Viskosität von Wasser enthaltenden Zubereitungen, die weitere Hilfsstoffe enthalten, z.B. jener kosmetischer Zubereitungen, die nicht einschränkend in den Zubereitungssbeispielen offenbart sind, wurde mit Hilfe eines Brookfield-Viskosimeters (Firma Brookfield), Typ DV-II+Pro Viskosimeter (Model: RVDVII+Pro) bestimmt. Als Messsystem wurde ein RV Spindelset bei einer Temperatur von 20 °C und 20 U/Min. Schergeschwindigkeit eingesetzt.

Viskositäten der kosmetischen Zubereitungen Z.1.1 bis Z.1.5 (basierend auf einer nicht-ionischen Grundlage) in Abhängigkeit von der Salzkonzentration

Tabelle 3. Viskositäten der kosmetischen Zubereitungen Z.1.1 bis Z.1.5 in Abhängigkeit von der Salzkonzentration.

| Zubereitung | Salz nachträglich portionsweise zugegeben Dynamische Viskosität [Pa*s] | | | | |
|---|---|---|---|---|---|
| | 0 Gew.-% NaCl | 0,5 Gew.-% NaCl | 2,0 Gew.-% NaCl | 5,0 Gew.-% NaCl | 10,0 Gew.-% NaCl |
| Z.1.1 | 33,2 | 24,0 | 13,2 | 7,9 | 7,0 |
| Z.1.2 | 39,5 | 29,8 | 14,8 | 11,0 | 11,3 |
| Z.1.3 | 4,1 | 6,1 | 6,3 | 7,7 | 8,6 |
| Z.1.4 | 3,0 | 4,3 | 3,9 | 4,3 | 2,4 |
| Z.1.5 | 11,3 | 9,7 | 6,9 | 5,1 | 3,8 |
| Gesamtmenge Salz in Phase B eingearbeitet Dynamische Viskosität [Pa*s] | | | | | |
| Z.1.1 | 33,6 | --- | 39,3 | 39,6 | --- |

Die Zubereitungen Z.1.3 und Z.1.4 zeigen bei Salzzugabe steigende bzw. weitgehend stabile Viskositäten. Z.1.1, Z.1.2 und Z.1.5 zeigen auch bei moderater Salzzugabe noch gute Verdickerleistung.

Viskositäten der kosmetischen Zubereitungen Z.2.1 bis Z.2.5 (basierend auf einer nicht-ionischen Grundlage) in Abhängigkeit von der Salzkonzentration

Tabelle 4. Viskositäten der kosmetischen Zubereitungen Z.2.1 bis Z.2.5 in Abhängigkeit von der Salzkonzentration, die nachträglich portionsweise zugegeben wurde

| Zubereitung | Dynamische Viskosität [Pa*s] | | | | |
|---|---|---|---|---|---|
| | 0 Gew.-% NaCl | 0,5 Gew.-% NaCl | 2,0 Gew.-% NaCl | 5,0 Gew.-% NaCl | 10,0 Gew.-% NaCl |
| Z.2.1 | 23,3 | 18,0 | 15,0 | 10,6 | 5,3 |
| Z.2.2 | 16,4 | 11,2 | 9,5 | 7,6 | 4,6 |
| Z.2.3 | 13,1 | 14,4 | 15,6 | 18,0 | 20,3 |
| Z.2.4 | 5,4 | 13,0 | 13,3 | 15,2 | 13,7 |
| Z.2.5 | 27,0 | 30,6 | 23,5 | 23,8 | 16,1 |

Zubereitung Z.2.5 zeigt bei Salzzugabe stabile und teilweise sogar steigende Viskositäten. Dies ist für Z.2.3 und Z.2.4 noch ausgeprägter, diese zeigen eine starke Erhöhung der dynamischen Viskositäten bei Salzzugabe bis 10 Gew.-%. Z.2.1 und Z.2.2 weisen auch bei moderater Salzzugabe noch gute Verdickerleistung auf.

Im Folgenden sind typische erfindungsgemäße Zubereitungen beschrieben, ohne jedoch die Erfindung auf diese Beispiele zu beschränken.

Die Prozentangaben sind Gew.-%, sofern nicht ausdrücklich anderweitig beschrieben.

### Sonnenschutzcreme 1

| | % | Inhaltsstoff | INCI |
|---|---|---|---|
| A | 58,7 | Water dem. | Aqua |
| | 0,1 | Edeta®BD | Disodium EDTA |
| | 1,0 | Butylenglycol | Butylene Glycol |
| | 2,0 | Uvinul®MS 40 | Benzophenone-4 |
| | 1,0 | TEA | Triethanolamine |
| | 0,5 | Panthenol®75 W | Panthenol |
| | 2,4 | Polyurethan PU.1 | |
| | | | |
| B | 5,0 | Neo Heliopan®OS | Octyl Salicylate |
| | 3,0 | Eusolex®9020 | Avobenzone |
| | 5,0 | Neo Heliopan®HMS | Homosalate |
| | 8,0 | Uvinul®N 539 T | Octocrylene |
| | 1,0 | Cremophor®GS 32 | Polyglyceryl-3 Distearate |
| | 1,0 | Cremophor®A 6 | Ceteareth-6, Stearyl Alcohol |
| | 1,0 | Cremophor®A 25 | Ceteareth-25 |
| | 2,0 | Lanette®E | Sodium Cetearyl Sulfate |
| | 0,5 | Span®60 | Sorbitan Stearate |
| | 3,0 | Luvitol® Lite | Hydrogenated Polyisobutene |
| | 2,0 | Lanette® O | Cetearyl Alcohol |
| | 1,5 | Lanette® 16 | Cetyl Alcohol |
| | 1,0 | Cetiol® SB 45 | Butyrospermum Parkii (Shea Butter) |
| | 0,1 | Vitamin E Acetat | Tocopheryl Acetate |
| | 0,2 | Bisabolol rac. | Bisabolol |
| | | | |
| C | 0,5 | Glydant® LTD | DMDM Hydantoin |

Herstellung

Die Phasen A und B getrennt auf ca. 80°C erwärmen.

Phase B in Phase A einrühren und kurz homogenisieren.

Unter Rühren auf ca. 40°C abkühlen, Phase C hinzugeben, unter Rühren auf Raumtemperatur abkühlen und nochmals kurz homogenisieren.

Viskositäten

a) ohne Polyurethan PU.1: 7,2 Pa s (Brookfield RVD VII+/Spindle No. 6)

b) mit Polyurethan PU.1: 56,2 Pa s (Brookfield RVD VII+/Spindle No. 7)

Anstelle der Sonnenschutzcreme enthaltend das Polyurethan PU.1 werden auch Sonnenschutzcremes enthaltend eines oder mehrere der Polyurethane PU.2 bis PU.11 hergestellt.

### Sonnenschutzcreme 2

| | % | Inhaltsstoff | INCI |
|---|---|---|---|
| A | 2,0 | Cremophor® A 6 | Ceteareth-6, Stearyl Alcohol |

(fortgesetzt)

| | % | Inhaltsstoff | INCI |
|---|---|---|---|
| | 2,0 | Cremophor® A 25 | Ceteareth-25 |
| | 5,0 | Luvitol® EHO | Cetearyl Ethylhexanoate |
| | 5,0 | Paraffinöl, dickflüssig | Mineral Oil |
| | 2,5 | Lanette® O | Cetearyl Alcohol |
| B | 5,0 | Z-Cote® MAX | Zinc Oxide, Dimethoxydiphenylsilane/ Triethoxycaprylylsilane Crosspolymer |
| C | 2,4 | Polyurethan PU.1 | |
| | 5,0 | 1,2-Propylenglycol | Propylene Glycol |
| | 70,5 | Wasser, demin. | Water |
| D | 0,5 | Euxyl® K 300 | Phenoxyethanol, Methylparaben, Ethylparaben, Butylparaben, Propylparaben |

Herstellung

Phase A auf 80°C erwärmen. Phase B in Phase A geben.
Phase A+B 3 min homogenisieren.
Phase C auf 80°C erwärmen, in Phase A+B einrühren und homogenisieren.
Emulsion unter Rühren auf 40°C abkühlen.
Phase D zugeben, unter Rühren auf RT abkühlen und homogenisieren.

Viskositäten

a) ohne Polyurethan PU.1: 1,5 Pa s (Brookfield RVD VII+/Spindle No. 6)
b) mit Polyurethan PU.1: 24,3 Pa s (Brookfield RVD VII+/Spindle No. 6)

Anstelle der Sonnenschutzcreme enthaltend das Polyurethan PU.1 werden auch Sonnenschutzcremes enthaltend eines oder mehrere der Polyurethane PU.2 bis PU.11 hergestellt.

Tagescreme mit UV-Schutz

| % | Inhaltsstoff | INCI |
|---|---|---|
| A | | |
| 3,00 | Tego Care® 450 | Polyglyceryl-3 Methyl Glucose Distearate |
| 3,00 | Lanette® 18 | Stearyl Alcohol |
| 2,00 | Cutina® GMS | Glyceryl Stearate |
| 4,00 | Estol® 1540 | Ethylhexyl Cocoate |
| 5,00 | Luvito®l EHO | Cetearyl Ethylhexanoate |
| 8,00 | Uvinul® A Plus B | Ethylhexyl Methoxycinnamate, Diethylamino Hydroxybenzoyl Hexyl Benzoate |
| B | | |
| 5,00 | D-Panthenol 50 P | Panthenol, Propylene Glycol |
| 0,10 | Edeta BD | Disodium EDTA |
| 1,0-5,0 | Polyurethan PU.1 | |
| ad 100 | Wasser dem. | Aqua dem. |
| C | | |
| 0,20 | Bisabolol nat. | Bisabolol |
| q.s. | Parfümöl | |
| 0,50 | Aloe Vera Gel Konzentrat 10/1 | Water, Aloe Barbadensis Leaf Juice |
| 0,50 | Euxyl® K 300 | Phenoxyethanol, Methylparaben, Butylparaben, Ethylparaben, Propylparaben, Isobutylparaben |

Herstellung

Die Phasen A und B getrennt auf ca. 80°C erwärmen.
Phase B in Phase A einrühren und kurz homogenisieren.
Unter Rühren auf ca. 40°C abkühlen, Phase C hinzugeben, unter Rühren auf Raumtemperatur abkühlen und nochmals kurz homogenisieren.
Anstelle der Tagescreme enthaltend das Polyurethan PU.1 werden auch Tagescremes enthaltend eines oder mehrere der Polyurethane PU.2 bis PU.11 hergestellt.

Make up

| % | Inhaltsstoff | INCI |
|---|---|---|
| A | | |
| 4,00 | Dracorin® 100 SE | Glyceryl Stearate, PEG-100 Stearate |
| 1,00 | Uvinul® A Plus | Diethylamino Hydroxybenzoyl Hexyl Benzoate |
| 3,00 | Uvinul® MC 80 | Ethylhexyl Methoxycinnamate |
| 0,50 | Emulmetik® 100 | Lecithin |
| 0,50 | Rylo® PG 11 | Polyglyceryl Dimer Soyate |
| B | | |
| 0,35 | Sicovit® Braun 75 E 172 | Iron Oxides |
| 2,00 | Sicovit® Rot 30 E 172 | Iron Oxides |
| 1,00 | Sicovit® Gelb 10 E 172 | Iron Oxides |
| 2,25 | Prisorine® 3630 | Trimethylolpropane Triisostearate |
| C | | |
| 5,50 | Dow Corning® 345 Fluid | Cyclopentasiloxane, Cyclohexasiloxane |
| 4,00 | Tegosoft® OP | Ethylhexyl Palmitate |
| 1,50 | Jojobaöl | Simmondsia Chinensis (Jojoba) Seed Oil |
| 2,00 | Miglyol® 840 | Propylene Glycol Dicaprylate/Dicaprate |
| 1,50 | Mandelöl, süß | Sweet Almond (Prunus Amygdalus Dulcis) Oil |
| 0,50 | Vitamin E-Acetat | Tocopheryl Acetate |
| 1,00 | Cetiol® SB 45 | Butyrospermum Parkii (Shea Butter) |
| 5,00 | Uvinul® TiO2 | Titanium Dioxide, Trimethoxycaprylylsilane |
| 0,50 | Dehymuls® PGPH | Polyglyceryl-2 Dipolyhydroxystearate |
| D | | |
| 5,00 | 1,2-Propylenglykol Care | Propylene Glycol |
| 0,50 | Lutrol® F 68 | Poloxamer 188 |
| 0,10 | Edeta BD | Disodium EDTA |
| 1,0 - 5,0 | Polyurethan PU.1 | |
| ad 100 | Wasser dem. | Aqua dem. |
| E | | |
| 1,00 | Euxyl® K 300 | Phenonip |
| | | Phenoxyethanol, Methylparaben, Ethylparaben, Butylparaben, Propylparaben, Isobutylparaben |
| 0,20 | Bisabolol rac. | Bisabolol |
| q.s. | Parfümöl | |

Herstellung

Die Phasen A, B, C und D getrennt voneinander auf 70°C erwärmen.
Phase B über Dreiwalzenstuhl homogenisiert. Phase B in Phase A einrühren. Alles noch einmal kurz homogenisieren.
Phase C lösen und in Phase A+B einrühren.

Phase D lösen, in die kombinierten Phasen A+B+C einrühren und homogenisieren.
Unter Rühren auf ca. 40°C abkühlen, Phase E zugeben und auf Raumtemperatur abkühlen. Kurz homogenisieren.
Anstelle des Make ups enthaltend das Polyurethan PU.1 werden auch Make ups enthaltend eines oder mehrere der Polyurethane PU.2 bis PU.11 hergestellt.

Getönte Tagescreme

| % | Inhaltsstoff | INCI |
|---|---|---|
| A | | |
| ad 100 | Wasser dem. | Aqua dem. |
| 5,00 | Glycerin 87% | Glycerin |
| 4,00 | D-Panthenol 50 P | Panthenol, Propylene Glycol |
| 0,75 | Cloisonné® Gold | Mica, Titanium Dioxide, Iron Oxides |
| 0,25 | Cloisonné® Super Rouge | Mica, Iron Oxides |
| 1,0-5,0 | Polyurethan PU.1 | |
| B | | |
| 3,00 | Uvinul® A Plus | Diethylamino Hydroxybenzoyl Hexyl Benzoate |
| 7,00 | Luvitol® Lite | Hydrogenated Polyisobutene |
| 1,50 | Lanette® O | Cetearyl Alcohol |
| 1,50 | Cutina® GMS | Glyceryl Stearate |
| 3,50 | Cetiol® SB 45 | Butyrospermum Parkii (Shea Butter) |
| 3,50 | Olivenöl | Olive (Olea Europaea) Oil |
| 1,00 | Eumulgin® B 2 | Ceteareth-20 |
| 1,00 | Cremophor® A6 | Ceteareth-6, Stearyl Alcohol |
| 1,00 | Cetiol® OE | Dicaprylyl Ether |
| 0,05 | BHT | BHT |
| C | | |
| 0,20 | Sodium Ascorbyl Phosphate | Sodium Ascorbyl Phosphate |
| 5,00 | Wasser dem. | Aqua dem. |
| D | | |
| 1,00 | Euxyl® PE 9010 | Phenoxyethanol, Ethylhexylglycerin |
| 0,25 | Bisabolol rac. | Bisabolol |
| 1,00 | Vitamin E-Acetat | Tocopheryl Acetate |
| q.s. | Parfümöl | |

Herstellung

Phase A auf 80°C erwärmen.
Phase B auf ca. 80°C erwärmen und unter Rühren in Phase A einrühren. Homogenisieren.
Unter Rühren auf ca. 40°C abkühlen, Phase C + D hinzugeben und unter Rühren auf Raumtemperatur abkühlen.
Kurz homogenisieren.
Anstelle der getönten Tagescreme enthaltend das Polyurethan PU.1 werden auch getönte Tagescremes enthaltend eines oder mehrere der Polyurethane PU.2 bis PU.11 hergestellt.

Deolotion

| % | Inhaltsstoff | INCI |
|---|---|---|
| A | | |
| 1,50 | Cremophor® A 6 | Ceteareth-6, Stearyl Alcohol |
| 1,50 | Cremophor® A 25 | Ceteareth-25 |
| 2,00 | Cremophor® CO 40 | PEG-40 Hydrogenated Castor Oil |
| 2,00 | Cutina® GMS | Glyceryl Stearate |
| 2,00 | Lanette® O | Cetyl Alcohol |
| 2,00 | Softisan® 100 | Hydrogenated Coco-Glycerides |
| 8,00 | Cetiol® V | Decyl Oleate |

(fortgesetzt)

| % | Inhaltsstoff | INCI |
|---|---|---|
| 0,50 | Abil® B 8843 | PEG-14 Dimethicone |
| 0,30 | Farnesol | Farnesol |
| B | | |
| q.s. | Konservierungsmittel | Preservative |
| 1,0-5,0 | Polyurethan PU.1 | |
| ad 100 | Wasser dem. | Aqua dem. |
| C q.s. | Parfümöl | Fragrance |
| D 5-20 | Locron® L | Aluminum Chlorohydrate |

Herstellung

Die Phasen A und B getrennt auf ca. 80°C erwärmen.

Phase B unter Homogenisieren in Phase A einrühren, kurz nachhomogenisieren.

Abkühlen auf ca. 40°C, die Phasen C und D unter Homogenisieren zugeben und unter Rühren auf Raumtemperatur abkühlen lassen.

Anstelle der Deolotion enthaltend das Polyurethan PU.1 werden auch Deolotionen enthaltend eines oder mehrere der Polyurethane PU.2 bis PU.11 hergestellt.

Haarwachs mit Pigmenten

| % | Phase | Inhaltsstoff | INCI |
|---|---|---|---|
| 5 | A | Cremophor®CO 40PEG-40 | Hydrogenated Castor Oil |
| 15 | | Cremophor®A 25 | Ceteareth-25 |
| 0,5-10 | | Polyurethan PU.1 | |
| 15 | | Luvitol®Lite | Hydrogenated Polyisobutene |
| 3 | | Marlipal®MG | Laureth-7 |
| 2 | | Brij®98 | Oleth-20 |
| 1 | | Euxyl®PE 9010 | Phenoxyethanol and Ethylhexylglycerin |
| | | | |
| 5 | B | Abil®B 88183 | PEG/PPG-20/6 Dimethicone |
| ad 100 | | Wasser dem. | Water dem |
| 1 | | Gemtone®Emerald | Mica and Titanium Dioxide and Chromium Oxide Greens and Ferric Ferrocyanide |

Herstellung:

I: Getrennte Einwaage der Phasen A und B und Erwärmen unter Rühren auf 80°C

II: Phase A und B bei 80°C unter Rühren vereinigen

III: Kühlen auf RT unter Rühren

Anstelle des Haarwachses enthaltend das Polyurethan PU.1 werden auch Haarwachse enthaltend eines oder mehrere der Polyurethane PU.2 bis PU.11 hergestellt.

Haargel mit UV-Schutz

| Phase | % | INCI | Inhaltsstoff |
|---|---|---|---|
| A | 44,55 | Aqua dem. | |
| | 0,45 | Acrylates/C10-30 Alkyl Acrylate Crosspolymer | |
| | | | |
| B | 0,36 | Aminomethyl Propanol | |
| C | 0,66 | Panthenol | D-Panthenol 75W® |

(fortgesetzt)

| Phase % | | INCI | Inhaltsstoff |
|---|---|---|---|
| | 10,00 | PVP/VA Copolymer | Luviskol® VA 64 W |
| | 2,50 | Polyquaternium-46 | Luviquat® Hold |
| | 5,00 | Sorbitol | |
| | 0,10 | Disodium EDTA | |
| | 0,5 | Benzophenone-4 | Uvinul® MS 40 |
| | q.s. | Perfume | |
| q.s. | | PEG-40 Hydrogenated Castor Oil Cremophor® CO 40 | |
| q.s. | | Preservative | |
| 5,00 | | Alcohol | |
| ad 55 | | Aqua dem. | |
| 0,5-4 | | Polyurethan PU.1 | |

Herstellung:

I: Separate Einwaage der Phasen A,B und C und ggf. Rühren bis zur Homogenität bei RT
II: Phase B und A bei RT unter Rühren vereinigen und homogen Rühren, dann Phase C unter Rühren hinzufügen und glatt Rühren

Anstelle des Haargels enthaltend das Polyurethan PU.1 werden auch Haargele enthaltend eines oder mehrere der Polyurethane PU.2 bis PU.11 hergestellt.

| | | Haarschaum |
|---|---|---|
| % | Inhaltsstoff | INCI |
| qs Phase A | Deionized Water | (Aqua dem.) |
| 11,00 | Luviquat® Hold | (Polyquaternium 46) |
| 1,50 | Uvinul® MS 40 | (Benzophenone-4) 20% sol.,neutr.m. Triethanolamin |
| 0,1-3 | Polyurethan PU.1 | |
| 0,40 | D,L Panthenol 50W | (Panthenol) |
| 0,20 | Masil®SF 19 CG | (PEG-8 Methicone) |
| 0,40 | Glydant®Plus liquid | (DMDM Hydantoin (and) Iodopropynyl Butylcarbamate) |
| 0,20 Phase B | Cremophor® CO 40 | (PEG-40 Hydrogenated Castor Oil) |
| 0,40 | Vitamin E Acetate | (Tocopheryl Acetate) |
| 0,20 | Bell®6101232 | (Fragrance |
| 0,70 | Rhodasurf®L-4 | (Laureth-4) |
| 6,00 Phase C | Treibgas A46 | (Propan/ Isobutan) |

Herstellung:

1. Einwaage der Stoffe Phase A unter Rühren bis vollst. Lösung in aufgelisteter Reihenfolge
2. Einwaage der Stoffe Phase B unter Rühren und Erwärmen bei 40-45°C.
3. Vereinigen der Phasen A und B und Umfüllen in geeigneteTreibgasbehälter für Haarschäume
4. Verschließen und befüllen mit Treibgas Phase C.

Anstelle des Haarschaums enthaltend das Polyurethan PU.1 werden auch Haarschäume enthaltend eines oder mehrere der Polyurethane PU.2 bis PU.11 hergestellt.

Gesichtscreme mit 3% Sodium Ascorbyl Phosphate

| % | Inhaltsstoff | INCI |
|---|---|---|
| A | | |
| 2,00 | Cremophor® A 6 | Ceteareth-6, Stearyl Alcohol |
| 2,00 | Cremophor® A 25 | Ceteareth-25 |
| 3,00 | Jojobaöl | Simmondsia Chinensis (Jojoba) Seed Oil |
| 3,00 | Lanette® O | Cetearyl Alcohol |
| 10,00 | Paraffinöl, dickflüssig | Mineral Oil |
| 5,00 | Vaseline | Petrolatum |
| 4,00 | Miglyol® 812 | Caprylic/Capric Triglyceride |
| B | | |
| 5,00 | 1,2-Propylenglykol Care | Propylene Glycol |
| 0,10 | Edeta BD | Disodium EDTA |
| 1,0-5,0 | Polyurethan PU.1 | |
| 0,30 | Abiol® | Imidazolidinyl Urea |
| ad 100 | Wasser dem. | Aqua dem. |
| C | | |
| 0,08 | Natriumhydroxid | Sodium Hydroxide |
| D | | |
| 0,50 | Vitamin E-Acetat | Tocopheryl Acetate |
| 0,20 | Phenoxyethanol | Phenoxyethanol |
| 3,00 | Sodium Ascorbyl Phosphate | Sodium Ascorbyl Phosphate |

Herstellung

Die Phasen A und B getrennt auf ca. 80°C erwärmen.
Phase B in Phase A einrühren und homogenisieren.
Phase C in Phase A+B einrühren und homogenisieren.
Unter Rühren auf ca. 40°C abkühlen.
Phase C einrühren und kurz nachhomogenisieren.
Unter Rühren auf Raumtemperatur abkühlen.

Anstelle der Gesichtscreme enthaltend das Polyurethan PU.1 werden auch Gesichtscremes enthaltend eines oder mehrere der Polyurethane PU.2 bis PU.11 hergestellt.

Verringerung der durchschnittlichen Tröpfchengrößenverteilung / O/W-Emulsion

| % | Inhaltsstoff | INCI |
|---|---|---|
| Phase A | | |
| 2,0 | Lanette® O | Cetearyl Alcohol |
| 5,0 | Finsolv® TN | C12-15 Alkyl Benzoate |
| 10,0 | Miglyol® 812 | Caprylic/Capric Triglyceride |
| 5,0 | Cetiol® B | Dibutyl Adipate |
| 2,0 | Amphisol® K | Potassium Cetyl Phosphate |
| 0,5 | Elfacos® ST-9 | PEG-45/Dodecyl Glycol Copolymer |
| Phase B | | |
| 5,0 | 1,2 Propylenglykol Care | Propylene Glycol |
| ad 100 | Wasser, demin. | Water |
| 1,0-5,0 | Polyurethan PU.1 | |
| Phase C | | |

(fortgesetzt)

| % | Inhaltsstoff | INCI |
|---|---|---|
| 0,5 | Euxyl® K 300 | Phenoxyethanol, Methylparaben, Ethylparaben, Butylparaben, Propylparaben |

Phasen A und B auf ca. 80°C erwärmen. Phase B in Phase A einrühren, homogenisieren. Kaltrühren, Phase C einrühren, kurz nachhomogenisieren.

Anstelle der O/W-Emulsion enthaltend das Polyurethan PU.1 werden auch O/W-Emulsionen enthaltend eines oder mehrere der Polyurethane PU.2 bis PU.11 hergestellt.

AHA-Creme

| % | Inhaltsstoff | INCI |
|---|---|---|
| **A** | | |
| 2,00 | Cremophor® A 6 | Ceteareth-6, Stearyl Alcohol |
| 2,00 | Cremophor® A 25 | Ceteareth-25 |
| 8,00 | Paraffinöl, dickflüssig | Mineral Oil |
| 7,00 | Luvitol® EHO | Cetearyl Ethylhexanoate |
| 6,00 | Cutina® GMS | Glyceryl Stearate |
| 1,00 | Lanette® 16 | Cetyl Alcohol |
| 0,20 | Abil® 350 | Dimethicone |
| 0,20 | Bisabolol nat. | Bisabolol |
| **B** | | |
| 1,00 | D-Panthenol USP | Panthenol |
| 3,00 | 1,2-Propylenglykol Care | Propylene Glycol |
| 1,0-5,0 | Polyurethan PU.1 | |
| 5,00 | Hydroxysäure | |
| q.s | Natriumhydroxid | Sodium Hydroxide |
| q.s. | Konservierungsmittel | Preservative |
| ad 100 | Wasser dem. | Aqua dem. |
| **C** | | |
| q.s. | Parfümöl | Fragrance |

Hinweis

Alpha-Hydroxysäuren: Milchsäure, Citronensäure, Äpfelsäure, Glycolsäure Di-Hydroxysäure: Weinsäure Beta-Hydroxysäure: Salicylsäure

Herstellung

Phase A und B getrennt auf ca. 80°C erwärmen. pH-Wert der Phase B gegebenenfalls mit NaOH auf 3 einstellen. Phase B in Phase A unter Homogenisieren einrühren, kurz nachhomogenisieren.

Abkühlen auf ca. 40°C, Phase C zugeben, nochmals nachhomogenisieren.

Anstelle der AHA-Creme enthaltend das Polyurethan PU.1 werden auch AHA-Cremes enthaltend eines oder mehrere der Polyurethane PU.2 bis PU.11 hergestellt.

Creme mit Vitamin-A-Säure

| % | Inhaltsstoff | INCI |
|---|---|---|
| **A** | | |
| 1,50 | Cremophor® A 25 | Ceteareth-25 |
| 1,50 | Cremophor® A 6 | Ceteareth-6, Stearyl Alcohol |
| 3,00 | Tegin® | Glyceryl Stearate SE |

(fortgesetzt)

| % | Inhaltsstoff | INCI |
|---|---|---|
| 2,00 | Lanette® O | Cetearyl Alcohol |
| 10,00 | Luvitol® EHO | Cetearyl Ethylhexanoate |
| 5,00 | Paraffinöl, dickflüssig | Mineral Oil |
| 0,10 | D, L-Alpha-Tocopherol | Tocopherol |
| 0,10 | Vitamin A-Säure | Tretionin |
| B | | |
| 1,0-5,0 | Polyurethan PU.1 | |
| 4,00 | 1,2-Propylenglykol Care | Propylene Glycol |
| 0,10 | Edeta BD | Disodium EDTA |
| q.s. | Konservierungsmittel | Preservative |
| ad 100 | Wasser dem. | Aqua dem. |
| C | | |
| 0,40 | Triethanolamin Care | Triethanolamine |
| 3,00 | Vitamin E-Acetat | Tocopheryl Acetate |
| 0,10 | Vitamin A-Säure | Tretionin |
| q.s. | Parfümöl | Fragrance |

Herstellung

Phase A und Phase B getrennt auf ca. 75°C erwärmen. Phase B in Phase A einrühren und homogenisieren. Kaltrühren. Phase C bei ca. 30°C zugeben.
Hinweis: Die Formulierung wird ohne Schutzgas hergestellt. Die Abfüllung muß in sauerstoffundurchlässige Verpackungen, z.B.
Aluminiumtuben, erfolgen.

Anstelle der Creme mit Vitamin-A-Säure enthaltend das Polyurethan PU.1 werden auch Cremes mit Vitamin-A-Säure enthaltend eines oder mehrere der Polyurethane PU.2 bis PU.11 hergestellt.

Enthaarungscreme 1

| % | Inhaltsstoff | INCI |
|---|---|---|
| A | | |
| 4,20 | Lanette® 16 | Cetyl Alcohol |
| 1,26 | Brij® 35 | Laureth-23 |
| B | | |
| 15,00 | Luviquat® Care | Polyquaternium-44 |
| 0,90 | D-Panthenol USP | Panthenol |
| 0,35 | Allantoin | Allantoin |
| q.s. | Konservierungsmittel | Preservative |
| 22,40 | Calciumcarbonat | Calcium Carbonate |
| 10,00 | Calciumhydroxid | Calcium Hydroxide |
| 5,40 | Calciumthioglykolat | Calcium Thioglycolate |
| ad 100 | Wasser dem. | Aqua dem. |
| 1,0-5,0 | Polyurethan PU.1 | |
| C | | |
| q.s. | Parfümöl | Fragrance |

Herstellung

Phase A und B getrennt auf ca. 80°C erwärmen.
Phase B in Phase A unter Homogenisieren einrühren, kurz nachhomogenisieren.
Abkühlen auf ca. 40°C, Phase C zugeben, nochmals homogenisieren.
Anstelle der Enthaarungscreme enthaltend das Polyurethan PU.1 werden auch Enthaarungscremes enthaltend eines oder mehrere der Polyurethane PU.2 bis PU.11 hergestellt.

### Enthaarungscreme 2

| % | Inhaltsstoff | INCI |
|---|---|---|
| A | | |
| 1,00 | Cremophor® A 6 | Ceteareth-6, Stearyl Alcohol |
| 1,00 | Cremophor® A 25 | Ceteareth-25 |
| 4,00 | Lanette® O | Cetearyl Alcohol |
| 6,00 | Paraffinöl, dickflüssig | Mineral Oil |
| q.s. | Konservierungsmittel | Preservative |
| B | | |
| 8,00 | Calciumthioglykolat | Calcium Thioglycolate |
| 2,00 | 1,2-Propylenglykol Care | Propylene Glycol |
| 1,0-5,0 | Polyurethan PU.1 | |
| 1,00 | Natriumhydroxid | Sodium Hydroxide |
| ad100 | Wasser dem. | Aqua dem. |
| C | | |
| q.s. | Parfümöl | Fragrance |

Herstellung

Phase A und B getrennt auf ca. 80°C erwärmen.
Phase B in Phase A unter Homogenisieren einrühren, kurz nachhomogenisieren.
Abkühlen auf ca. 40°C, Phase C zugeben, nochmals homogenisieren.
Anstelle der Enthaarungscreme enthaltend das Polyurethan PU.1 werden auch Enthaarungscremes enthaltend eines oder mehrere der Polyurethane PU.2 bis PU.11 hergestellt.

### Conditioner Shampoo 1

| | |
|---|---|
| 35,70 g | Sodium Laureth Sulfate |
| 6,50 g | Cocamidopropyl Betaine |
| 0,20 g | Polyurethan PU.1 |
| 0,50 g | Polyquaternium-7, PQ-10, PQ-39, PQ-44, PQ-67, Guarhydroxypropyltrimonium Chloride und/oder PQ-87 |
| 0,10 g | Konservierungsmittel |
| 0,10 g | Parfumöl / etherisches Öl |
| ad 100 g | Aqua dem. |

Anstelle des Conditioner Shampoos 1 enthaltend das Polyurethan PU.1 werden auch Conditioner Shampoos enthaltend eines oder mehrere der Polyurethane PU.2 bis PU.11 hergestellt.

### Conditioner Shampoo 2

| | |
|---|---|
| 35,70 g | Sodium Laureth Sulfate |
| 6,50 g | Cocamidopropyl Betaine |
| 0,50 g | Polyurethan PU.1 |
| 0,20 g | Guarhydroxypropyltrimonium Chloride |
| 0,10 g | Konservierungsmittel |
| 0,10 g | Parfumöl / etherisches Öl |
| ad 100 g | Aqua dem. |

Anstelle des Conditioner Shampoos 2 enthaltend das Polyurethan PU.1 werden auch Conditioner Shampoos enthaltend eines oder mehrere der Polyurethane PU.2 bis PU.11 hergestellt.

Beispiel 3: Conditioner Shampoo 3

| | |
|---|---|
| 35,70 g | Sodium Laureth Sulfate |
| 6,50 g | Cocamidopropyl Betaine |
| 0,20 g | Polyurethan PU.1 |
| 0,50 g | Polyquaternium-7, PQ-10, PQ-39, PQ-44, PQ-67, Guarhydroxypropyltrimonium Chloride und/oder PQ-87 |
| 0,10 g | Konservierungsmittel |
| 0,10 g | Parfumöl / etherisches Öl |
| ad 100 g | Aqua dem. |

Anstelle des Conditioner Shampoos 4 enthaltend das Polyurethan PU.1 werden auch Conditioner Shampoos enthaltend eines oder mehrere der Polyurethane PU.2 bis PU.11 hergestellt.

Beispiel 4: Shampoo

Phase A

| | |
|---|---|
| 15,00 g | Cocamidopropyl Betaine |
| 10,00 | Disodium Cocoamphodiacetate |
| 5,00 g | Polysorbate 20 |
| 5,00 g | Decyl Glucoside |
| 0,50 g | Polyquaternium-7, PQ-10, PQ-39, PQ-44, PQ-67, Guarhydroxypropyltrimonium Chloride und/oder PQ-87 |
| 0,20 g | Polyurethan PU.1 |
| 0,10 g | Parfumöl / etherisches Öl |
| q.s. | Konservierungsmittel |
| 2,00 g | Laureth-3 |
| add 100 | Aqua dem. |
| q.s. | Citric Acid |

Phase B

| | |
|---|---|
| 3,00 g | PEG-150 Distearate |

Herstellung

Komponenten der Phase A einwiegen und lösen; pH-Wert auf 6-7 einstellen. Phase B zugeben und auf 50°C erwärmen. Auf Raumtemperatur abkühlen lassen unter Rühren.

Anstelle des Shampoos enthaltend das Polyurethan PU.1 werden auch Shampoos enthaltend eines oder mehrere der Polyurethane PU.2 bis PU.11 hergestellt.

Shampoo

| | |
|---|---|
| 30,00 g | Sodium Laureth Sulfate |
| 6,00 g | Sodium Cocoamphoacetate |
| 0,50 g | Polyquaternium-7, PQ-10, PQ-39, PQ-44, PQ-67, Guarhydroxypropyltrimonium Chloride und/oder PQ-87 |
| 0,50 g | Polyurethan PU.1 |
| 3,00 g | Sodium Laureth Sulfate, Glycol Distearate, Cocamide MEA, Laureth-10 |
| 2,00 g | Dimethicone |
| q.s. | Parfum |

(fortgesetzt)

| q.s. | Konservierungsmittel |
|---|---|
| q.s. | Citric Acid |
| 1,00 g | Sodium Chloride |
| add 100 | Aqua dem. |

Anstelle des Shampoos enthaltend das Polyurethan PU.1 werden auch Shampoos enthaltend eines oder mehrere der Polyurethane PU.2 bis PU.11 hergestellt.

Duschgel 1

| 20,00 g | Ammonium Laureth Sulfate |
|---|---|
| 15,00 g | Ammonium Lauryl Sulfate |
| 0,50 g | Polyurethan PU.1 |
| 0,50 g | Polyquaternium-10, PQ-22, PQ-44, PQ-67, Guarhydroxypropyltrimonium Chloride und/oder PQ-87 |
| 2,50 g | Sodium Laureth Sulfate, Glycol Distearate, Cocamide MEA, Laureth-10 |
| 0,10 g | Parfumöl / etherisches Öl |
| q.s. | Konservierungsmittel |
| 0,50 g | Sodium Chloride |
| add 100 | Aqua dem. |

Anstelle des Duschgels 1 enthaltend das Polyurethan PU.1 werden auch Duschgele enthaltend eines oder mehrere der Polyurethane PU.2 bis PU.11 hergestellt.

Duschgel 2

| 40,00 g | Sodium Laureth Sulfate |
|---|---|
| 5,00 g | Decyl Glucoside |
| 5,00 g | Polyurethan PU.1 |
| 1,00 g | Panthenol |
| 0,50 g | Polyquaternium-10, PQ-44, PQ-67, Guarhydroxypropyltrimonium Chloride und/oder PQ-87 |
| 0,10 g | Parfumöl / etherisches Öl |
| q.s. | Konservierungsmittel |
| q.s. | Citric Acid |
| 2,00 g | Sodium Chloride |
| add 100 | Aqua dem. |

Anstelle des Duschgels 2 enthaltend das Polyurethan PU.1 werden auch Duschgele enthaltend eines oder mehrere der Polyurethane PU.2 bis PU.11 hergestellt.

Shampoo

| 12,00 g | Sodium Laureth Sulfate |
|---|---|
| 1,50 g | Decyl Clucoside |
| 0,50 g | Polyquaternium-10, PQ-44, PQ-67, Guarhydroxypropyltrimonium Chloride und/oder PQ-87 |
| 0,50 g | Polyurethan PU.1 |
| 5,00 g | Coco-Glucoside Glyceryl Oleate |
| 2,00 g | Sodium Laureth Sulfate, Glycol Distearate, Cocomide MEA, Laureth-10 |
| q.s. | Konservierungsmittel |
| q.s. | Sunset Yelow C. I. 15 985 |
| 0,10 g | Parfumöl / etherisches Öl |
| 1,00 g | Sodium Chloride |
| add 100 | Aqua dem. |

Anstelle dieses Shampoos enthaltend das Polyurethan PU.1 werden auch Shampoos enthaltend eines oder mehrere der Polyurethane PU.2 bis PU.11 hergestellt.

Die Polyurethane PU können auch in Haarstyling-Zubereitungen, insbesondere Haarschäumen (Aerosolschäume mit Treibgas und Pumpschäume ohne Treibgas), Haarsprays (Pumpssprays ohne Treibgas) und Haargelen verwendet werden. Treibmittel sind die üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

### Aerosolhaarschaum

| | |
|---|---|
| 2.00 g | Cocotrimonium Methosulfate |
| 0,10 g | Parfumöl / etherisches Öl |
| 3,50 g | Festigerpolymer bzw. Kombinationen aus z.B. PVP, PVP/VA Copolymer, Polyquaternium-4, PQ-11, PQ-16, PQ-46, PQ-44, PQ-68, VP/Methacrylamide/Vinyl Imidazole Copolymer, etc. |
| 0,80 g | Polyurethan PU.1 |
| q.s. | Preservative |
| 75,00 g | Wasser dem. |
| 10,00 g | Propane/Butane (3.5 bar) |

Anstelle dieses Aerosolhaarschaums enthaltend das Polyurethan PU.1 werden auch Aerosolhaarschäume enthaltend eines oder mehrere der Polyurethane PU.2 bis PU.11 hergestellt.

Haarstylinggel 1

### Phase A

| | |
|---|---|
| 0,50 g | Carbomer oder Acrylates/C10-30 Alkyl Acrylate Crosspolymer |
| 86,40 g | Wasser dem. |

### Phase B

| | |
|---|---|
| 0,70 g | Triethanolamine |

### Phase C

| | |
|---|---|
| 11,00 g | Festigerpolymer bzw. Kombinationen aus z.B. PVP, PVP/VA Copolymer, Polyquaternium-4, PQ-11, PQ-16, PQ-46, PQ-44, PQ-68, VP/Methacrylamide/Vinyl Imidazole Copolymer, etc. |
| 0,20 g | PEG-25 PABA |
| 2,00 g | Polyurethan PU.1 |
| 0,10 g | Parfumöl / etherisches Öl |
| q.s. | PEG-14 Dimethicone |
| q.s. | Konservierungsmittel |
| 0,10 g | Tocopheryl Acetate |

Anstelle dieses Haarstylinggels 1 enthaltend das Polyurethan PU.1 werden auch Haarstylinggele enthaltend eines oder mehrere der Polyurethane PU.2 bis PU.11 hergestellt.

Haarstylinggel 2

### Phase A

| | |
|---|---|
| 0,50 g | Carbomer oder Acrylates/C10-30 Alkyl Acrylate Crosspolymer |
| 91,20 g | Wasser dem. |

Phase B

0,90 g    Tetrahydroxypropyl Ethylenediamine

Phase C

| | |
|---|---|
| 7,00 g | VP/VA Copolymer |
| 0,70 g | Polyurethan PU.1 |
| 0,20 g | Parfumöl / etherisches Öl |
| q.s. | Konservierungsmittel |
| 0,10 g | Propylene Glycol |

Anstelle dieses Haarstylinggels 2 enthaltend das Polyurethan PU.1 werden auch Haarstylinggele enthaltend eines oder mehrere der Polyurethane PU.2 bis PU.11 hergestellt.

Hair Wax Cream

| | |
|---|---|
| 6,00 g | Caprylic/Capric Triglyceride |
| 3,00 g | Glyceryl Stearate |
| 2,00 g | Cetyl Alcohol |
| 3,50 g | Polyurethan PU.1 |
| 0,50 g | Cremophoer A6 |
| 0,70 g | Cremophor A25 |
| 0,50 g | Dimethicone |
| 0,50 g | Vitamin E Acetate |
| 2,00 g | Caprylic/Capric Triglyceride and Sodiumacrylates Copolymer |
| 1,00 g | D-Panthenol USP |
| 0,10 g | EDTA |
| 10,00 g | Festigerpolymer |
| q.s. | Konservierungsmittel |
| ad 100 g | Wasser dem. |

Anstelle dieser Hair Wax Cream enthaltend das Polyurethan PU.1 werden auch Hair Wax Creams enthaltend eines oder mehrere der Polyurethane PU.2 bis PU.11 hergestellt.

Haarpudding

| | |
|---|---|
| 3,00 g | Kollicoat IR (BASF) |
| q.s. | Konservierungsmittel |
| 2,00 g | Festigerpolymer |
| 4,00 g | Acrylates/beheneth-25 Methacrylate Copolymer |
| 0,70 g | Polyurethan PU.1 |
| 0,50 g | Dimethicone Copolyol |
| 0,10 g | EDTA |
| 0,20 g | Benzophenone-4 |
| ad 100 g | Wasser dem. |

Anstelle dieses Haarpuddings enthaltend das Polyurethan PU.1 werden auch Haarpuddings enthaltend eines oder mehrere der Polyurethane PU.2 bis PU.11 hergestellt.

Sprühgel

Phase A

| | |
|---|---|
| 1,25 g | Festigerpolymer |
| 96,15 g | Aqua dem. |

Phase B

| | |
|---|---|
| 0,70 g | Acrylates/Steareth-20 Itaconate Copolymer |
| 0,10 g | Propylene Glycol |
| 0,50 g | Polyurethan PU.1 |
| 0,10 g | Glycerin |
| 0,10 g | Parfumöl / etherisches Öl |
| q.s. | Konservierungsmittel |

Phase C

| | |
|---|---|
| 0,70 g | Triethanolamine |

Anstelle dieses Sprühgels enthaltend das Polyurethan PU.1 werden auch Sprühgele enthaltend eines oder mehrere der Polyurethane PU.2 bis PU.11 hergestellt.

Eine erfindungsgemäß für Styling-Sprays geeignete Zubereitung kann beispielsweise wie folgt zusammengesetzt sein:

Pumphaarspray

| | |
|---|---|
| 11,20 g | PEG/PPG-25/25 Dimethicone/Acrylates Copolymer oder Acrylates Copolymer |
| 2,80 g | VP/VA Copolymer |
| 1,34 g | Aminomethyl Propanol |
| 0,30 g | Polyurethan PU.1 |
| 0,10 g | Parfumöl / etherisches Öl |
| 11,26 g | Aqua dem. |
| 73,00 g | Alcohol |

Anstelle dieses Pumphaarsprays enthaltend das Polyurethan PU.1 werden auch Sprühgele enthaltend eines oder mehrere der Polyurethane PU.2 bis PU.11 hergestellt.

Pumphaarspray VOC55

| | |
|---|---|
| 2,00 g | VP/Methacrylamide/Vinyl Imidazole Copolymer |
| 1,90 g | Polyquaternium-46 |
| 2,00 g | Polyurethan PU.1 |
| 0,10 g | Parfumöl / etherisches Öl |
| 55,00 g | Alcohol |
| 39,00 g | Aqua dem. |

Dekorativkosmetische Zusammensetzungen

Anstelle dieses Pumphaarsprays VOC55 enthaltend das Polyurethan PU.1 werden auch Sprühgele enthaltend eines oder mehrere der Polyurethane PU.2 bis PU.11 hergestellt.

Flüssiges Makeup

Phase A

| | |
|---|---|
| 1,70 g | Glyceryl Stearate |
| 1,70 g | Cetyl Alcohol |
| 1,70 g | Ceteareth-6 |
| 1,70 g | Ceteareth-25 |
| 5,20 g | Caprylic/Capric Triglyceride |
| 5,20 g | Mineral Oil oder Luvitol® Lite (INCI Hydrogenated Polyisobuten) |

Phase B

| | |
|---|---|
| q.s. | Konservierungsmittel |
| 4,30 g | Propylene Glycol |
| 2,50 g | Polyurethan PU.1 |
| 59,50 g | Aqua dem. |

Phase C

| | |
|---|---|
| 0,10 g | Parfumöl / etherisches Öl |

Phase D

| | |
|---|---|
| 2,00 g | Iron Oxides |
| 12,00 g | Titanium Dioxide |

Anstelle dieses flüssigen Makeups enthaltend das Polyurethan PU.1 werden auch flüssige Makeups enthaltend eines oder mehrere der Polyurethane PU.2 bis PU.11 hergestellt.

Eyeliner

Phase A

| | |
|---|---|
| 40,60 g | dest. Wasser |
| 0,20 | Disodium EDTA |
| q.s. | Konservierungsmittel |

Phase B

| | |
|---|---|
| 0,60 g | Xanthan Gum |
| 0,40 g | Veegum |
| 3,00 g | Butylene Glycol |
| 0,20 g | Polysorbate-20 |

Phase C

| | |
|---|---|
| 15,00 g | Iron oxide / Al Powder / Silica (z.B. Sicopearl® Fantastico Gold von BASF oder andere Effektpigmente) |

Phase D

| 10,00 g | Aqua dem. |
|---|---|
| 25,00 g | Festigerpolymer bzw. Kombinationen aus z.B. PVP, PVP/VA Copolymer, Polyquaternium-4, PQ-11, PQ-16, PQ-46, PQ-44, PQ-68, Polyurethane-1 oder VP/Methacrylamide/ Vinyl Imidazole Copolymer, etc. |
| 5,00 g | Polyurethan PU.1 |

Anstelle dieses Eyeliners enthaltend das Polyurethan PU.1 werden auch Eyeliner enthaltend eines oder mehrere der Polyurethane PU.2 bis PU.11 hergestellt.

Gesichtswasser

Phase A

| 3,00 g | Polyurethan PU.1 |
|---|---|
| 0,10 g | Parfumöl / etherisches Öl |
| 0,30 g | Bisabolol |

Phase B

| 3,00 g | Glycerin |
|---|---|
| 1,00 g | Hydroxyethyl Cetyldimonium Phosphate |
| 5,00 g | Whitch Hazel (Hamamelis Virginiana) Distillate |
| 0,50 g | Panthenol |
| q.s. | Konservierungsmittel |
| 87,60 g | Aqua dem. |

Anstelle dieses Gesichtswassers enthaltend das Polyurethan PU.1 werden auch Gesichtswasser enthaltend eines oder mehrere der Polyurethane PU.2 bis PU.11 hergestellt.

Gesichtswaschpaste mit Peelingeffekt

Phase A

| 73,00 g | Aqua dem. |
|---|---|
| 1,50 g | Polyurethan PU.1 |
| q.s. | Konservierungsmittel |

Phase B

| q.s. | Parfümöl |
|---|---|
| 7,00 g | Potassium Cocoyl Hydrolyzed Protein |
| 4,00 g | Conditioningpolymer bzw. Kombinationen aus Polyquaternium-7, PQ-10, PQ-39, PQ-44, PQ-67, Guarhydroxypropyltrimonium Chloride, PQ-87 |

Phase C

| 1,50 g | Triethanolamine |
|---|---|

Phase D

13,00 g    Polyethylene (Luwax A™ von BASF)

Anstelle dieser Gesichtswaschpaste enthaltend das Polyurethan PU.1 werden auch Gesichtswaschpasten enthaltend eines oder mehrere der Polyurethane PU.2 bis PU.11 hergestellt.

Seife

Phase A

| | |
|---|---|
| 25,00 g | Potassium Cocoate |
| 20,00 | Disodium Cocoamphodiacetate |
| 2,00 g | Lauramide DEA |
| 1,0 g | Glycol Stearate |
| 2,00 g | Polyurethan PU.1 |
| 0,50 g | Conditioningpolymer bzw. Kombinationen aus Polyquaternium-7, PQ-10, PQ-39, PQ-44, PQ-67, Guarhydroxypropyltrimonium Chloride, PQ-87 |
| 50,00 g | Aqua dem. |
| q.s. | Citric Acid |

Phase B

| | |
|---|---|
| q.s. | Konservierungsmittel |
| 0,10 g | Parfumöl / etherisches Öl |

Anstelle dieser Seife enthaltend das Polyurethan PU.1 werden auch Seifen enthaltend eines oder mehrere der Polyurethane PU.2 bis PU.11 hergestellt.

Gesichtsreinigungsmilch Typ O/W

Phase A

| | |
|---|---|
| 1,50 g | Ceteareth-6 |
| 1,50 g | Ceteareth-25 |
| 2,00 g | Glyceryl Stearate |
| 2,00 g | Cetyl Alcohol |
| 10,00 g | Mineral Oil |

Phase B

| | |
|---|---|
| 5,00 g | Propylene Glycol |
| q.s. | Konservierungsmittel |
| 1,00 g | Conditioningpolymer bzw. Kombinationen aus Polyquaternium-7, PQ-10, PQ-39, PQ-44, PQ-67, Guarhydroxypropyltrimonium Chloride, PQ-87 |
| 66,30 g | Aqua dem. |

Phase C

| | |
|---|---|
| 0,20 g | Polyurethan PU.1 |
| 10,00 g | Cetearyl Octanoate |

Phase D

0,40 g    Tetrahydroxypropyl Ethylenediamine


Phase E

0,10 g    Parfumöl / etherisches Öl
0,10 g    Bisabolol

Anstelle dieser Gesichtsreinigungsmilch enthaltend das Polyurethan PU.1 werden auch Gesichtsreinigungsmilche enthaltend eines oder mehrere der Polyurethane PU.2 bis PU.11 hergestellt.

Transparente Seife

4,20 g    Sodium Hydroxide
3,60 g    dest. Wasser
5,00 g    Conditioningpolymer bzw. Kombinationen aus Polyquaternium-7, PQ-10, PQ-39, PQ-44, PQ-67, Guarhydroxypropyltrimonium Chloride, PQ-87
5,00 g    Polyurethan PU.1
22,60 g   Propylene Glycol
18,70 g   Glycerin
5,20 g    Cocoamide DEA
2,40 g    Cocamine Oxide
4,20 g    Sodium Lauryl Sulfate
7,30 g    Myristic Acid
16,60 g   Stearic Acid
5,20 g    Tocopherol

Anstelle dieser transparenten Seife enthaltend das Polyurethan PU.1 werden auch transparente Seifen enthaltend eines oder mehrere der Polyurethane PU.2 bis PU.11 hergestellt.

Rasierschaum

6,00 g    Ceteareth-25
5,00 g    Poloxamer 407
52,00 g   Aqua dem.
1,00 g    Triethanolamine
5,00 g    Propylene Glycol
1,00 g    PEG-75 Lanolin Oil
2,00 g    Conditioningpolymer bzw. Kombinationen aus Polyquaternium-7, PQ-10, PQ-39, PQ-44, PQ-67, Guarhydroxypropyltrimonium Chloride, PQ-87
3,00 g    Polyurethan PU.1
q.s.      Konservierungsmittel
0,10 g    Parfumöl / etherisches Öl
25,00 g   Sodium Laureth Sulfate

Abfüllung: 90 Teile Wirksubstanz und 10 Teile Propan/Butan-Mischung 25:75.
Anstelle dieses Rasierschaumes enthaltend das Polyurethan PU.1 werden auch Rasierschäume enthaltend eines oder mehrere der Polyurethane PU.2 bis PU.11 hergestellt.

After Shave Balsam

Phase A

| | |
|---|---|
| 0,25 g | Polyurethan PU.1 |
| 1,50 g | Tocopheryl Acetate |
| 0,20 g | Bisabolol |
| 10,00 g | Caprylic/Capric Triglyceride |
| q.s. | Parfum |
| 1,00 g | Conditioningpolymer bzw. Kombinationen aus Polyquaternium-7, PQ-10, PQ-39, PQ-44, PQ-67, Guarhydroxypropyltrimonium Chloride, PQ-87 |

Phase B

| | |
|---|---|
| 1,00 g | Panthenol |
| 15,00 g | Alcohol |
| 5,00 g | Glycerin |
| 0,05 g | Hydroxyethyl Cellulose |
| 1,90 g | Polyurethan PU.1 |
| 64,02 g | dest. Wasser |

Phase C

| | |
|---|---|
| 0,08 g | Sodium Hydroxide |

Anstelle dieses After Shave Balsams enthaltend das Polyurethan PU.1 werden auch After Shave Balsame enthaltend eines oder mehrere der Polyurethane PU.2 bis PU.11 hergestellt.

Zahnpaste

Phase A

| | |
|---|---|
| 34,79 g | Aqua dem. |
| 3,00 g | Polyurethan PU.1 |
| 20,00 g | Glycerin |
| 0,76 g | Sodium Monofluorophosphate |

Phase B

| | |
|---|---|
| 1,20 g | Sodium Carboxymethylcellulose |

Phase C

| | |
|---|---|
| 0,80 g | Aromaöl |
| 0,06 g | Saccharin |
| q.s. | Konservierungsmittel |
| 0,05 g | Bisabolol |
| 1,00 g | Panthenol |
| 0,50 g | Tocopheryl Acetate |
| 2,80 g | Silica |
| 1,00 g | Sodium Lauryl Sulfate |
| 7,90 g | Dicalciumphosphate Anhydrate |
| 25,29 g | Dicalciumphosphate Dihydrate |
| 0,45 g | Titanium Dioxide |

Anstelle dieser Zahnpaste enthaltend das Polyurethan PU.1 werden auch Zahnpasten enthaltend eines oder mehrere der Polyurethane PU.2 bis PU.11 hergestellt.

Mundwasser

|  |  |
|---|---|
|  | Phase A |
| 2,00 g | Aromaöl |
| 4,50 g | Polyurethan PU.1 |
| 1,00 g | Bisabolol |
| 30,00 g | Alcohol |

|  |  |
|---|---|
|  | Phase B |
| 0,20 g | Saccharin |
| 5,00 g | Glycerin |
| q.s. | Konservierungsmittel |
| 5,00 g | Poloxamer 407 |
| 52,30 g | Aqua dem |

Anstelle dieses Mundwassers enthaltend das Polyurethan PU.1 werden auch Mundwasser enthaltend eines oder mehrere der Polyurethane PU.2 bis PU.11 hergestellt.

Prothesenhaftmittel

|  |  |
|---|---|
|  | Phase A |
| 0,20 g | Bisabolol |
| 1,00 g | Beta-Carotene |
| q.s. | Aromaöl |
| 20,00 g | Cetearyl Octanoate |
| 5,00 g | Silica |
| 33,80 g | Mineral Oil |

|  |  |
|---|---|
|  | Phase B |
| 5,00 g | Polyurethan PU.1 |
| 35,00 g | PVP (20%ige Lösung in Wasser) |

Anstelle dieses Prothesenhaftmittels enthaltend das Polyurethan PU.1 werden auch Prothesenhaftmittel enthaltend eines oder mehrere der Polyurethane PU.2 bis PU.11 hergestellt.

**Patentansprüche**

1. Kosmetische Zubereitung enthaltend eine in Wasser dispergierbare Mischung von Polyurethanen (PU) mit einem im Wesentlichen linearen Rückgrat aufgebaut aus alternierenden hydrophilen und hydrophoben Abschnitten, wobei

   a. die beiden endständigen Abschnitte (T) hydrophob sind,
   b. sich an jeden Abschnitt T je ein hydrophiler Abschnitt (S) direkt anschließt,
   c. sich an jeden Abschnitt S mindestens ein hydrophober Abschnitt (D) auf mindestens einer Seite direkt anschließt, und

d. wobei mindestens ein hydrophiler Abschnitt (P) enthalten ist, wobei mindestens ein hydrophober Abschnitt D zwei Abschnitte P trennt, falls mehr als ein Abschnitt P enthalten ist,

und die Polyurethane mindestens drei hydrophile Abschnitte umfassen, und das Verhältnis der Molekulargewichte eines jeden hydrophilen Abschnittes S zu dem Molekulargewicht eines jeden hydrophilen Abschnittes P von 1 zu 1,4 bis 1 zu 140 beträgt, die mindestens zwei hydrophoben Abschnitte D aliphatische Diisocyanatreste sind und der mindestens eine hydrophile Abschnitt P ein Polyetherrest mit einem zahlenmittleren Molekulargewicht von mindestens 1500 g/mol ist, wobei die Polyurethane eine Mischung darstellen, die Polyurethane enthält, deren Abschnitte T beide verzweigt sind, und solche, deren Abschnitte T beide linear sind, und solche, die einen linearen Abschnitt T und einen verzweigten Abschnitt T enthalten.

2. Kosmetische Zubereitung nach Anspruch 1, wobei alle hydrophilen Abschnitte des Polyurethans Polyetherreste sind.

3. Kosmetische Zubereitung nach mindestens einem der Ansprüche 1 bis 2, wobei die mindestens zwei hydrophilen Abschnitte S des Polyurethans Ethylenoxidreste sind.

4. Kosmetische Zubereitung nach mindestens einem der Ansprüche 1 bis 3, worin der mindestens eine hydrophile Abschnitt P des Polyurethans ein zahlenmittleres Molekulargewicht von 1500 bis 10000 g/mol aufweist.

5. Kosmetische Zubereitung nach mindestens einem der Ansprüche 1 bis 4, wobei die kosmetische Zubereitung wenigstens 0,5 Gew.-% wenigstens eines Salzes und wenigstens 0,1 Gew.-% wenigstens eines Tensids enthält.

6. Kosmetische Zubereitung nach mindestens einem der Ansprüche 1 bis 5, wobei die kosmetische Zubereitung vom Typ Öl-in-Wasser-Emulsion ist.

7. Kosmetische Zubereitung nach mindestens einem der Ansprüche 1 bis 6, wobei die Zubereitung eine Lichtschutzzubereitung ist und Zinkoxid und/oder Titandioxid als anorganische UV-Lichtschutzfilter enthält.

8. Kosmetische Zubereitung nach mindestens einem der Ansprüche 1 bis 7, wobei die kosmetische Zubereitung Wasserstoffperoxid enthält.

9. Kosmetische Zubereitung nach mindestens einem der Ansprüche 1 bis 8, wobei die kosmetische Zubereitung im Bereich von 0,5 bis 15 Gew.-% Harnstoff enthält.

**Claims**

1. A cosmetic preparation comprising a water-dispersible mixture of polyurethanes (PU) having an essentially linear backbone composed of alternating hydrophilic and hydrophobic segments, wherein

  a. both terminal segments (T) are hydrophobic,
  b. a hydrophilic segment (S) is joined to each segment T,
  c. at least one hydrophobic segment (D) is joined directly on at least one side to each segment S, and
  d. wherein at least one hydrophilic segment (P) is present, wherein at least one hydrophobic segment D separates two P segments if more than one segment P is present,

and the polyurethanes comprise at least three hydrophilic segments, and the ratio of the molecular weights of each hydrophilic segment S to the molecular weight of each hydrophilic segment P is from 1:1.4 to 1:140, the at least two hydrophobic segments D are aliphatic diisocyanate residues and the at least one hydrophilic segment P is a polyether residue having a number-average molecular weight of at least 1500 g/mol, wherein the polyurethanes are a mixture comprising polyurethanes of which segments T are both branched and those which segments T are both linear and those which comprise one linear segment T and one branched segment T.

2. The cosmetic preparation according to claim 1, wherein all hydrophilic segments of the polyurethane are polyether residues.

3. The cosmetic preparation according to at least one of claims 1 to 2, wherein the at least two hydrophilic segments S of the polyurethane are ethylene oxide residues.

4. The cosmetic preparation according to at least one of claims 1 to 3, wherein the at least one hydrophilic segment P of the polyurethane has a number-average molecular weight of 1500 to 10 000 g/mol.

5. The cosmetic preparation according to at least one of claims 1 to 4, wherein the cosmetic preparation comprises at least 0.5% by weight of at least one salt and at least 0.1% by weight of a surfactant.

6. The cosmetic preparation according to at least one of claims 1 to 5, wherein the cosmetic preparation is of the oil-in-water emulsion type.

7. The cosmetic preparation according to at least one of claims 1 to 6, wherein the preparation is a light protection preparation and comprises zinc oxide and/or titanium dioxide as inorganic UV light protection filter.

8. The cosmetic preparation according to at least one of claims 1 to 7, wherein the cosmetic preparation comprises hydrogen peroxide.

9. The cosmetic preparation according to at least one of claims 1 to 8, wherein the cosmetic preparation comprises urea in the range of 0.5 to 15% by weight.


**Revendications**

1. Préparation cosmétique contenant un mélange dispersible dans l'eau de polyuréthanes (PU) ayant un squelette essentiellement linéaire formé par des sections hydrophiles et hydrophobes alternées,

   a. les deux sections terminales (T) étant hydrophobes,
   b. chaque section T étant directement suivie par une section hydrophile (S),
   c. chaque section S étant directement suivie par au moins une section hydrophobe (D) sur au moins un côté, et
   d. au moins une section hydrophile (P) étant contenue, au moins une section hydrophobe D séparant deux sections P si plus d'une section P est contenue,

   et les polyuréthanes comprenant au moins trois sections hydrophiles, et le rapport entre le poids moléculaire d'une section hydrophile S et le poids moléculaire d'une section hydrophile P étant de 1 sur 1,4 à 1 sur 140, lesdites au moins deux sections hydrophobes D étant des radicaux diisocyanate aliphatiques et ladite au moins une section hydrophile P étant un radical polyéther ayant un poids moléculaire moyen en nombre d'au moins 1 500 g/mol, les polyuréthanes étant un mélange qui contient des polyuréthanes dont les sections T sont toutes les deux ramifiées et des polyuréthanes dont les sections T sont toutes les deux linéaires et des polyuréthanes qui contiennent une section T linéaire et une section T ramifiée.

2. Préparation cosmétique selon la revendication 1, dans laquelle toutes les sections hydrophiles du polyuréthane sont des radicaux polyéther.

3. Préparation cosmétique selon au moins l'une quelconque des revendications 1 à 2, dans laquelle lesdites au moins deux sections hydrophiles S du polyuréthane sont des radicaux oxyde d'éthylène.

4. Préparation cosmétique selon au moins l'une quelconque des revendications 1 à 3, dans laquelle ladite au moins une section hydrophile P du polyuréthane présente un poids moléculaire moyen en nombre de 1 500 à 10 000 g/mol.

5. Préparation cosmétique selon au moins l'une quelconque des revendications 1 à 4, dans laquelle la préparation cosmétique contient au moins 0,5 % en poids d'au moins un sel et au moins 0,1 % en poids d'au moins un tensioactif.

6. Préparation cosmétique selon au moins l'une quelconque des revendications 1 à 5, dans laquelle la préparation cosmétique est une émulsion de type huile dans eau.

7. Préparation cosmétique selon au moins l'une quelconque des revendications 1 à 6, dans laquelle la préparation est une préparation photoprotectrice, et contient de l'oxyde de zinc et/ou du dioxyde de titane en tant que filtre inorganique de protection contre la lumière UV.

8. Préparation cosmétique selon au moins l'une quelconque des revendications 1 à 7, dans laquelle la préparation cosmétique contient du peroxyde d'hydrogène.

9. Préparation cosmétique selon au moins l'une quelconque des revendications 1 à 8, dans laquelle la préparation cosmétique contient dans la plage allant de 0,5 à 15 % en poids d'urée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4079028 A
- US 4155892 A
- EP 1013264 A
- EP 1584331 A
- EP 1241198 A
- WO 0244236 A
- WO 0283093 A
- WO 2006002813 A
- EP 0725097 B
- WO 2006106140 A
- DE 19726121 A
- EP 1291640 A1
- EP 1084696 A
- DE 3314742 A
- DE 102006028549 A1
- DE 102005033520 A1
- DE 102006032505 A1
- DE 102006021780 A1
- WO 0200181 A
- DE 10351842 A1
- EP 934956 A
- DE 102006009780 A1
- EP 934449 A
- EP 1023035 B
- EP 1139995 A
- WO 03000588 A
- DE 102004054055 A1

**In der Beschreibung aufgeführte Nicht-Patentliteratur**

- **GRIFFIN, W. C.** Classification of surface active agents by HLB. *J. Soc. Cosmet. Chem.,* 1949, vol. 1
- **BLANK et al.** *Progress in Organic Coatings,* 1999, vol. 35, 19 ff
- **KARL-HEINZ SCHRADER.** Grundlagen und Rezepturen der Kosmetika. Verlag Hüthig, 319-355
- Kosmetik und Hygiene von Kopf bis Fuß. Wiley-VCH, 2004, 131-134
- *CHEMICAL ABSTRACTS,* 9005-64-5
- *CHEMICAL ABSTRACTS,* 9005-67-8
- *CHEMICAL ABSTRACTS,* 9005-71 -4
- *CHEMICAL ABSTRACTS,* 9005-65-6
- *CHEMICAL ABSTRACTS,* 9005-65-5
- *CHEMICAL ABSTRACTS,* 9005-70-3
- *CHEMICAL ABSTRACTS,* 9005-66-7
- Kosmetik und Hygiene von Kopf bis Fuß. Wiley-VCH, 2004, 235-236
- *CHEMICAL ABSTRACTS,* 288254-1 6-0
- *CHEMICAL ABSTRACTS,* 7631-86-9
- *CHEMICAL ABSTRACTS,* 63451-27-4
- *CHEMICAL ABSTRACTS,* 26006-22-4
- *CHEMICAL ABSTRACTS,* 26062-79-3
- *CHEMICAL ABSTRACTS,* 26590-05-6
- *CHEMICAL ABSTRACTS,* 53568-66-4
- *CHEMICAL ABSTRACTS,* 53633-54-8
- *CHEMICAL ABSTRACTS,* 29297-55-0
- *CHEMICAL ABSTRACTS,* 90624-75-2
- *CHEMICAL ABSTRACTS,* 110736-85-1
- *CHEMICAL ABSTRACTS,* 110736-86-2
- *CHEMICAL ABSTRACTS,* 53694-17-0
- *CHEMICAL ABSTRACTS,* 107897-23-5
- *CHEMICAL ABSTRACTS,* 131954-48-8
- *CHEMICAL ABSTRACTS,* 92091-36-6
- *CHEMICAL ABSTRACTS,* 136505-02-7
- *CHEMICAL ABSTRACTS,* 35429-19-7
- *CHEMICAL ABSTRACTS,* 26161-33-1
- Chemistry and Technology of the Cosmetics and Toiletries Industry. Blackie, 1996, 326
- **KIRK-OTHMER.** Encyclopedia of Chemical Technology. 1979, vol. 8, 913-916
- **E. SAGARIN.** Cosmetics, Science and Technology. Interscience Publishers Inc, 1957, 503 ff
- **H. JANISTYN.** Handbuch der Kosmetika und Riechstoffe. 1973, vol. 3, 388 ff
- **K. SCHRADER.** *Grundlagen und Rezepturen der Kosmetika,* 1989, 782-815
- Kosmetik und Hygiene von Kopf bis Fuß. Wiley-VCH, 2004, 247-264
- International Cosmetic Ingredient Dictionary and Handbook. The Cosmetic, Toiletry, and Fragrance Association, 2002, vol. 4
- *CHEMICAL ABSTRACTS,* 65497-29-2
- *CHEMICAL ABSTRACTS,* 39421-75-5